# EUROPEAN PATENT APPLICATION

(11) **EP 3 858 834 A1**
(43) Date of publication of application: **04.08.2021**
(21) Application number: 19867599.3
(22) Date of filing: 29.09.2019
(51) Int. Cl.: C07D 487/04, A61K 31/519, A61P 35/00, A61P 29/00

(54) **PYRAZOLOPYRIMIDINE DERIVATIVE AS SELECTIVE TRK INHIBITOR**

(30) Priority: 29.09.2018 CN 201811153373; 22.05.2019 CN 201910432821
(71) Applicant: Shandong Luye Pharmaceutical Co., Ltd., Yantai, Shandong 264670 (CN)
(72) Inventor: WANG, Jianfei, Shanghai 200131 (CN); ZHANG, Yang, Shanghai 200131 (CN); SUN, Jikui, Shanghai 200131 (CN); CHEN, Shuhui, Shanghai 200131 (CN)
(74) Representative: karo IP
(86) International application number: PCT/CN2019/109043
(87) International publication number: WO 2020/063965

(57) **Abstract**

The present invention discloses a compound of formula (II), a tautomer thereof or a pharmaceutically acceptable salt thereof, and relates to use thereof in preparing a medicament for treating solid tumor-related diseases.

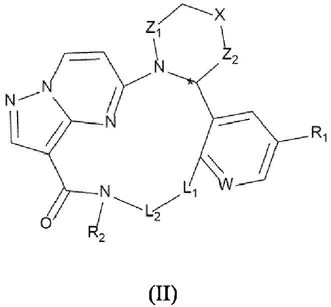

## Description

### CROSS-REFERENCE TO RELATED APPLICATIONS

The present application claims priority to Chinese Patent Application No. CN201811153373.7 filed on Sep. 29, 2018 and Chinese Patent Application No. CN201910432821.5 filed on May 22, 2019.

### TECHNICAL FIELD

The present invention relates to various oncogenic fusion kinase inhibitors, use thereof and a synthetic method thereof, and in particular to use of a compound of formula (II), a tautomer thereof or a pharmaceutically acceptable salt thereof in preparing a medicament for treating solid tumor-related diseases.

### BACKGROUND

Protein kinase plays an important role in human body and is widely involved in such processes as proliferation, differentiation, metabolism and apoptosis of various cells in the human body. Oncogenic forms of the protein kinase are abundantly expressed in a variety of different human tumor types and are highly responsive to specific kinase inhibitors. Tropomyosin-related kinase (Trk) is a type of Nerve Growth Factor Receptor (NGFR) that is highly expressed in nerve cells. The Trk family consists of highly homologous tropomyosin-related kinase A (TrkA), tropomyosin-related kinase B (TrkB) and tropomyosin-related kinase C (TrkC), which encode NTRK1, NTRK2 and NTRK3 respectively, involve 4 ligands of NGF, BDNF, NT-4 and NT-3 in total, and are widely involved in important physiological activities such as cell proliferation, differentiation, survival and neuronal growth by regulating main signal pathways such as PI3K-AKT, RAS-RAF-ERK and PLCγ-PKC. The continuously activated oncogenic form of Trk was first discovered from colorectal cancer as an oncogenic fusion gene (TPM3-NTRK1). Oncogenic Trk gene fusion, without ligand activation, can promote cancer cell proliferation and affect cancer-related downstream signaling pathway such as ERK and AKT. Various Trk gene fusions have been discovered so far, such as LMNA-NTRK1 and ETV6-NTRK3. Medicament targeting the TRK gene fusion, e.g., larotrectinib (LOXO-101), also proved effective in the initial clinic use. However, acquired resistance mutations also develop in treated patients under sustained action. The new medicament targeting the TRK gene fusion such as LOXO-195 incompletely solves the problem of resistance mutation. Accordingly, for the clinical treatment of some cancers related to the TRK fusion gene, a type of compounds with inhibitory effect on various oncogenic fusion kinases and mutations thereof are urgently needed.

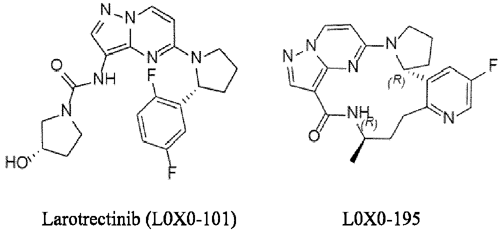

### SUMMARY

The present invention provides a compound of formula (II), an isomer thereof or a pharmaceutically acceptable salt thereof, 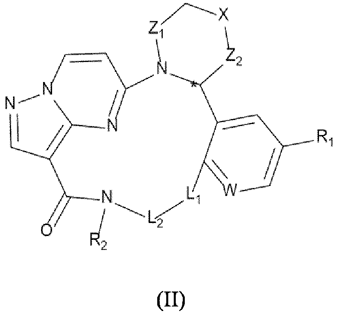 wherein,
W is selected from -C(R₃)- and N;
X is selected from -C(R₄)(R₅)-, -O-, and -N(R₆)-;
Z₁ is selected from -CH(R₇)-;
Z₂ is selected from -CH₂-, -CH₂CH₂-, and -CH₂CH₂CH₂-;
R₁ is selected from H, F, Cl, Br, I, OH, NH₂, CN, and C₁₋₆ alkyl optionally substituted with 1, 2, or 3 Rₐ;
R₂ is selected from H and C₁₋₆ alkyl optionally substituted with 1, 2, or 3 R_{b};
R₃ is selected from H, F, Cl, Br, I, OH, and NH₂;
R₄ and R₅ are each independently selected from H, F, Cl, Br, I, OH, NH₂, and C₁₋₆ alkyl optionally substituted with 1, 2, or 3 R_{c};
R₆ is selected from H and C₁₋₆ alkyl optionally substituted with 1, 2, or 3 R_{d};
R₇ is selected from H, F, Cl, Br, I, OH, NH₂, CN and C₁₋₆ alkyl optionally substituted with 1, 2, or 3 R_{g};
L₁ is selected from -O-, -N(R)-, and -C₁₋₃ alkyl- optionally substituted with 1, 2, or 3 Rₑ;
L₂ is selected from -C₁₋₃ alkyl-, -C₃₋₆ cycloalkyl-, -3-6 membered heterocycloalkyl-, and -C₃₋₆ cycloalkyl-C₁₋₃ alkyl-, the -C₁₋₃ alkyl-, -C₃₋₆ cycloalkyl-, -3-6 membered heterocycloalkyl-, and -C₃₋₆ cycloalkyl-C₁₋₃ alkyl-being optionally substituted with 1, 2, or 3 R_{f};
Rₐ, R_{b}, R_{c}, R_{d}, Rₑ, R_{f}, and R_{g} are each independently selected from H, F, Cl, Br, I, OH, and NH₂;
R is selected from H and C₁₋₃ alkyl; and
the carbon atom with "*" is a chiral carbon atom present in a form of a single (R)- or (*S*)- enantiomer or in a form enriched with one enantiomer;
wherein the 3-6 membered heterocycloalkyl contains 1, 2, 3, or 4 heteroatoms or heteroatom groups independently selected from O, NH, S, and N.

The present invention provides a compound of formula (I), an isomer thereof or a pharmaceutically acceptable salt thereof, 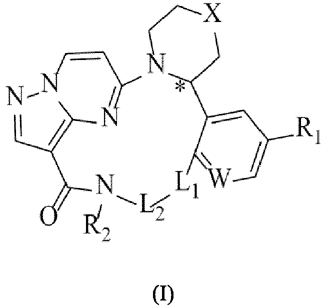 wherein,
W is selected from C(R₃) and N;
X is selected from -C(R₄)(R₅)-, -O-, and -N(R₆)-;
R₁ is selected from H, F, Cl, Br, I, OH, NH₂, CN, and C₁₋₆ alkyl optionally substituted with 1, 2, or 3 Rₐ;
R₂ is selected from H and C₁₋₆ alkyl optionally substituted with 1, 2, or 3 R_{b};
R₃ is selected from H, F, Cl, Br, I, OH, and NH₂;
R₄ and R₅ are each independently selected from H, F, Cl, Br, I, OH, NH₂, and C₁₋₆ alkyl optionally substituted with 1, 2, or 3 R_{c};
R₆ is selected from H and C₁₋₆ alkyl optionally substituted with 1, 2, or 3 R_{d};
L₁ is selected from -O-, -N(R)-, and -C₁₋₃ alkyl- optionally substituted with 1, 2, or 3 Rₑ;
L₂ is selected from -C₁₋₃ alkyl-, -C₃₋₆ cycloalkyl-, and -C₃₋₆ cycloalkyl-C₁₋₃ alkyl-, the -C₁₋₃ alkyl-, -C₃₋₆ cycloalkyl-, and -C₃₋₆ cycloalkyl-C₁₋₃ alkyl- being optionally substituted with 1, 2, or 3 R_{f};
Rₐ, R_{b}, R_{c}, R_{d}, Rₑ, and R_{f} are each independently selected from H, F, Cl, Br, I, OH, and NH₂; and
R is selected from H and C₁₋₃ alkyl; and
the carbon atom with "*" is a chiral carbon atom present in a form of a single (*R*)- or (*S*)- enantiomer or in a form enriched with one enantiomer.

In some embodiments of the present invention, R₁ is selected from H, F, Cl, Br, I, OH, NH₂, CN, and CH₃; preferably H, F, Cl, and Br; and more preferably F; the other variables are defined as herein.

In some embodiments of the present invention, R₂ is selected from H and CH₃, and preferably H; the other variables are defined as herein.

In some embodiments of the present invention, R₃ is selected from H, F, Cl, Br, and I; preferably H and F; and more preferably H.

In some embodiments of the present invention, R₄ and R₅ are each independently selected from H, F, Cl, Br, I, OH, NH₂, and CH₃; preferably H and F; and more preferably H; the other variables are defined as herein.

In some embodiments of the present invention, R₆ is selected from H and CH₃; the other variables are defined as herein.

In some embodiments of the present invention, R₇ is selected from H, F, Cl, Br, I, OH, NH₂, CN, and CH₃, CH₂CH₃, CH₂CH₂CH₃, and CH(CH₃)₂ optionally substituted with 1, 2, or 3 R_{g}; preferably H, CH₃, CH₂CH₃, CH₂CH₂CH₃, and CH(CH₃)₂; more preferably H and CH₃; and most preferably H; the other variables are defined as herein.

In some embodiments of the present invention, L₁ is selected from -CH₂-, -CH₂CH₂-, -O-, and -NH-; preferably -NH- and -O-; and more preferably -O-; the other variables are defined as herein.

In some embodiments of the present invention, L₂ is selected from -CH₂-, -CH₂CH₂-, -CH₂CH(CH₃)-, 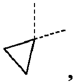 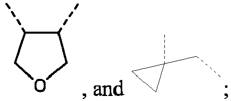 preferably -CH₂CH(CH₃)-, 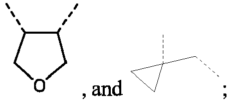 and more preferably 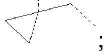 the other variables are defined as herein.

In some embodiments of the present invention, W is selected from -CH- and N, and preferably -N-; the other variables are defined as herein.

In some embodiments of the present invention, X is selected from -CH₂-, -O-, and -NH-, and preferably -O-; the other variables are defined as herein.

In some embodiments of the present invention, Z₁ is selected from -CH(CH₃) and -CH₂-, and preferably -CH₂-; the other variables are defined as herein.

In some embodiments of the present invention, Z₂ is selected from -CH₂-, -CH₂CH₂-, and -CH₂CH₂CH₂-; preferably -CH₂- and -CH₂CH₂; and more preferably -CH₂-; the other variables are defined as herein.

In some embodiments of the present invention, R is selected from H, -CH₃ and -CH₂CH₃; preferably H and - CH₃; and more preferably H; the other variables are defined as herein.

In some embodiments of the present invention, Rₐ, R_{b}, R_{c}, R_{d}, Rₑ, and R_{f} are each independently selected from H and F, and preferably H; the other variables are defined as herein.

In some embodiments of the present invention, the structural unit 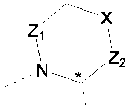 is selected from 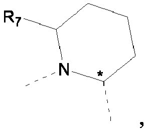 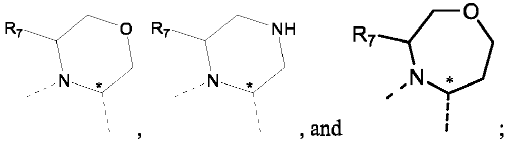 the other variables are defined as herein. In some embodiments of the present invention, the structural unit 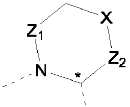 is selected from 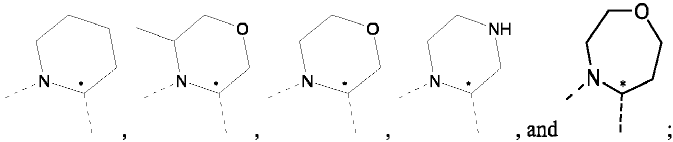 preferably 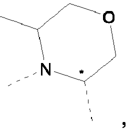 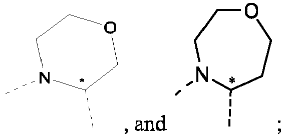 more preferably 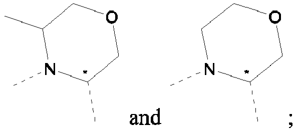 and most preferably 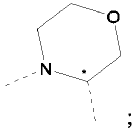 the other variables defined as herein.

In some embodiments of the present invention, the compound of formula (II), the isomer thereof or the pharmaceutically acceptable salt thereof is provided, wherein W is selected from -CH- and N; X is selected from -CH₂-, -O-, and -NH-; R₁ is F; R₂ is H; Z₁ is selected from -CH₂- and -CH(CH₃)-; Z₂ is selected from - CH₂- and -CH₂CH₂-; L₁ is -O-; and L₂ is selected from -CH₂CH(CH₃)-,

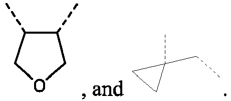

In some embodiments of the present invention, provided is the compound, the isomer thereof or the pharmaceutically acceptable salt thereof, selected from: 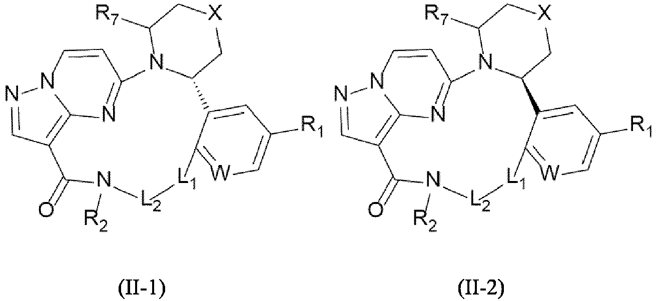 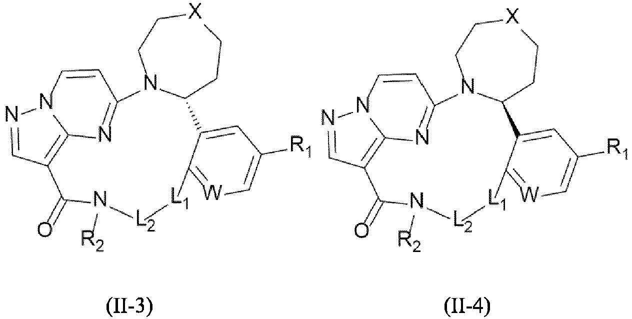 wherein W, X, R₁, R₂, R₇, L₁, and L₂ are defined as herein.

In some embodiments of the present invention, provided is the compound, the isomer thereof or the pharmaceutically acceptable salt thereof, selected from: 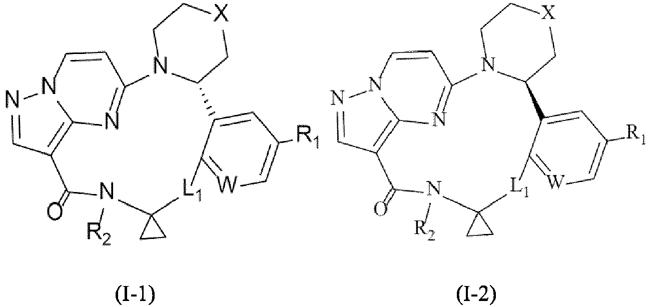 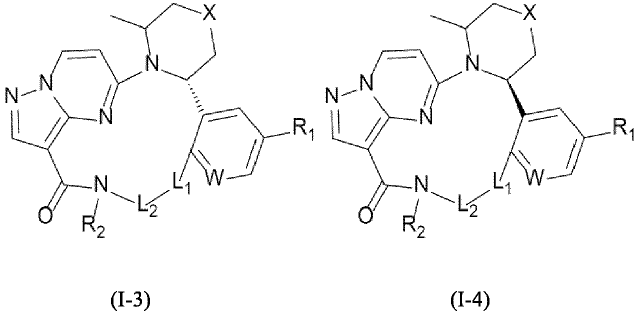 wherein W, X, R₁, R₂, L₁, and L₂ are defined as herein.

In some other embodiments of the present invention, any combination of the above variables is provided.

The present invention also provides a compound, an isomer thereof or a pharmaceutically acceptable salt thereof, selected from:

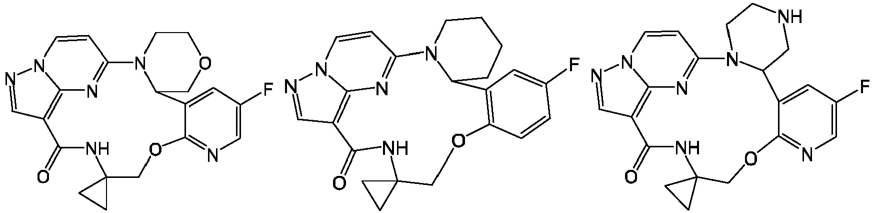

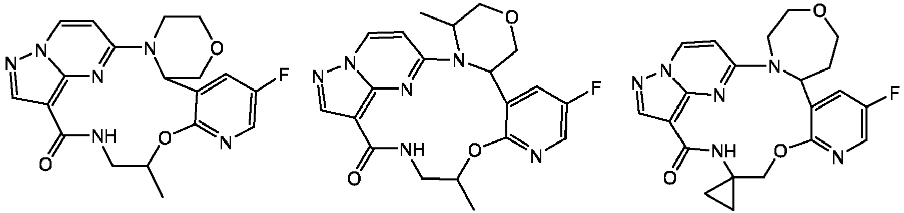

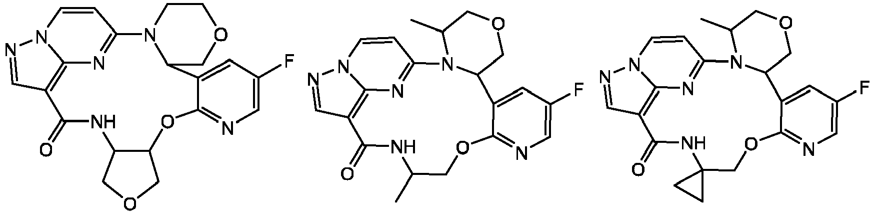

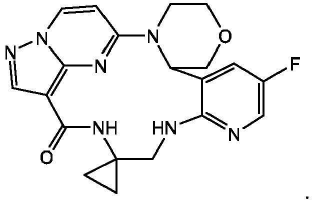

In some embodiments of the present invention, provided is the compound, the isomer thereof or the pharmaceutically acceptable salt thereof, selected from:

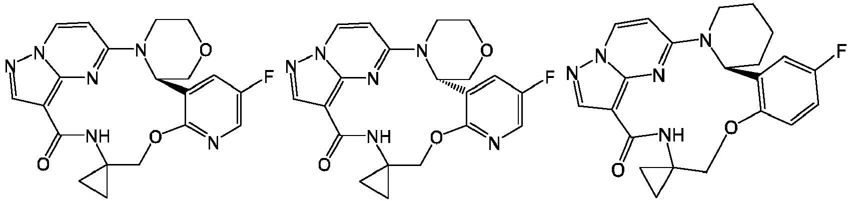

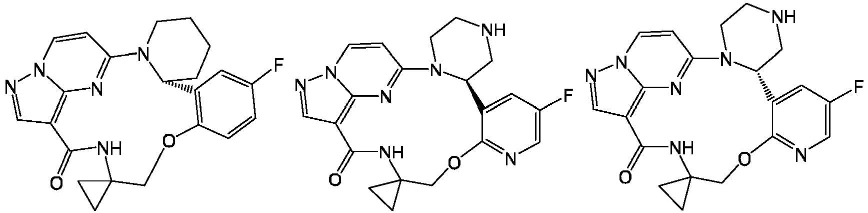

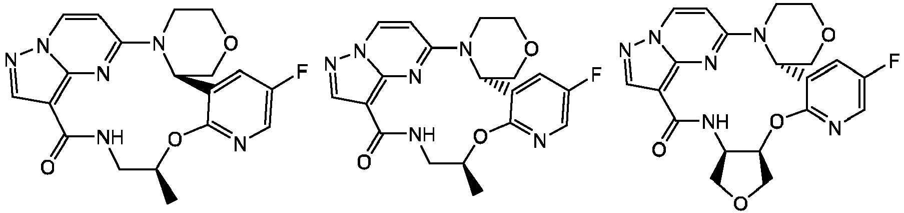

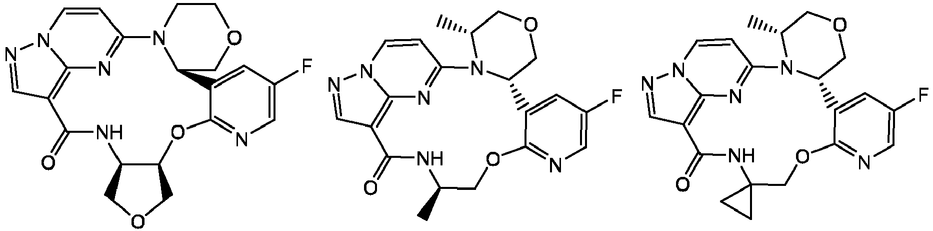

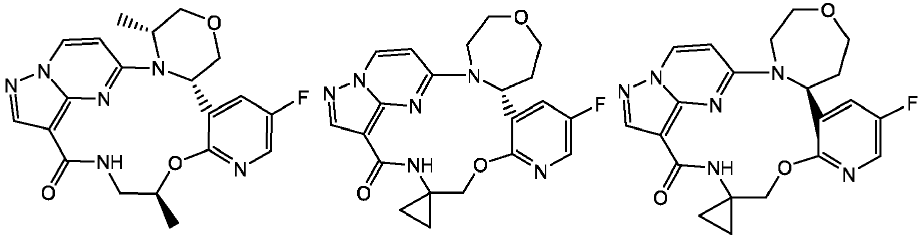

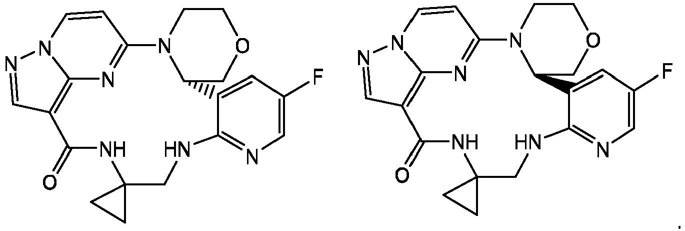

The present invention also provides a pharmaceutical composition, comprising a therapeutically effective amount of the compound, the isomer thereof or the pharmaceutically acceptable salt thereof as an active ingredient, and a pharmaceutically acceptable carrier.

The present invention also provides use of the compound, the isomer thereof or the pharmaceutically acceptable salt thereof, or the composition thereof in preparing a medicament for treating Trk kinase-related diseases.

In some embodiments of the present invention, the medicament is used for treating a solid tumor. The solid tumor includes neuroblastoma, ovarian cancer, colorectal cancer, melanoma, head and neck cancer, gastric cancer, lung cancer, breast cancer, glioblastoma, medulloblastoma, secretory breast cancer, salivary gland cancer, and papillary thyroid carcinoma.

### DEFINATION AND DESCRIPTION

Unless otherwise stated, the following terms and phrases used herein are intended to have the following meanings. A particular term or phrase, unless otherwise specifically defined, should not be considered as uncertain or unclear, but construed according to its ordinary meaning. When referring to a trade name, it is intended to refer to its corresponding commercial product or its active ingredient.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents disclosed herein and a relatively nontoxic acid or base. When the compound disclosed herein contains a relatively acidic functional group, a base addition salt can be obtained by contacting the neutral form of such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound disclosed herein contains a relatively basic functional group, an acid addition salt can be obtained by contacting the neutral form of such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, methanesulfonic acid, and the like. Also included are salts of amino acids *(e.g.,* arginine, *etc.)* and salts of organic acids such as glucuronic acid. Certain specific compounds disclosed herein contain both basic and acidic functional groups that allow the compounds to be converted into either base or acid addition salts.

The pharmaceutically acceptable salt disclosed herein can be synthesized from the parent compound containing an acid radical or a basic group by conventional chemical methods. In general, such a salt is prepared by reacting the compounds in free acid or base form with a stoichiometric amount of the appropriate base or acid in water or an organic solvent or a mixture of the two.

The compounds disclosed herein can be in the form of a geometric isomer or stereoisomer. All such compounds are contemplated herein, including cis and trans isomers, (-)- and (+)-enantiomers, (*R*)- and (*S*)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present invention. Substituents such as alkyl may have an additional asymmetric carbon atom. All these isomers and mixtures thereof are encompassed within the scope of the present invention.

Unless otherwise stated, the term "enantiomer" or "optical isomer" refers to stereoisomers that are mirror images of each other.

Unless otherwise stated, the term "cis-trans isomer" or "geometric isomer" results from the inability of a single bond of a ring carbon atom or a double bond to rotate freely.

Unless otherwise stated, the term "diastereoisomer" refers to stereoisomers in which molecules each have two or more chiral centers and are not mirror images of each other.

Unless otherwise stated, *"(D)"* or "(+)" stands for dextrorotation, "(*L*)" or "(-)" stands for levorotation, and *"(DL)"* or "(±)" stands for racemization.

Unless otherwise stated, the absolute configuration of a stereogenic center is represented by a wedged solid bond ( 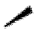 ) and a wedged dashed bond ( 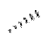 ), and the relative configuration of a stereogenic center is represented by a straight solid bond ( 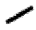 ) and a straight dashed bond ( 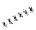 ). A wavy line ( 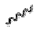 ) represents a wedged solid bond ( 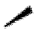 ) or a wedged dashed bond ( 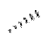 ), or a wavy line ( 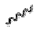 ) represents a straight solid bond ( 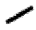 ) and a straight dashed bond ( 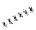 ).

The compound disclosed herein can be in the form of a specific isomer. Unless otherwise stated, the term "tautomer" or "tautomeric form" means that different functional isomers are in dynamic equilibrium at room temperature and can be rapidly converted into each other. If a tautomer is possible *(e.g.,* in solution), the chemical equilibrium of the tautomer can be reached. For example, a proton tautomer, also known as a prototropic tautomer, includes inter-conversion by proton transfer, such as keto-enol isomerization and imine-enamine isomerization. A valence isomer includes inter-conversion by recombination of some bonding electrons. A specific example of the keto-enol tautomerization is the inter-conversion between the tautomers pentane-2,4-dione and 4-hydroxypent-3-en-2-one.

Unless otherwise stated, the terms "enriched with one isomer", "isomer enriched", "enriched with one enantiomer" or "enantiomer enriched" mean that one of the isomers or enantiomers has a content less than 100%, for example, greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomeric excess" or "enantiomeric excess" refers to the difference between the relative percentages of two isomers or enantiomers. For example, if the content of one isomer or enantiomer is 90%, and the content of the other isomer or enantiomer is 10%, the isomeric or enantiomeric excess (ee value) is 80%.

Optically active (*R*)- and (*S*)-isomers as well as *D*- and *L*-isomers can be prepared by chiral synthesis or chiral reagents or other conventional techniques. An enantiomer of certain compound disclosed herein can be prepared by asymmetric synthesis or derivatization using a chiral auxiliary, wherein the resulting diastereoisomeric mixture is separated and the auxiliary group is cleaved so as to give the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to get the pure enantiomer. Furthermore, the enantiomer and the diastereoisomer are generally isolated through chromatography using a chiral stationary phase, optionally in combination with chemical derivatization *(e.g.,* carbamate formation from amines). The compound disclosed herein may contain an unnatural proportion of atomic isotope at one or more of the atoms that constitute the compound. For example, the compound may be labeled with a radioisotope such as tritium (³H), iodine-125 (¹²⁵I) or C-14 (¹⁴C). For another example, hydrogen can be substituted by deuterium to form a deuterated medicament, and the bond formed by deuterium and carbon is firmer than that formed by common hydrogen and carbon. Compared with an un-deuterated medicament, the deuterated medicament has the advantages of reduced toxic side effect, increased stability, enhanced efficacy, prolonged biological half-life and the like. All isotopic variations of the compound described herein, whether radioactive or not, are encompassed within the scope of the present invention.

"Optional" or "optionally" means that the subsequently described event or circumstance may, but not necessarily, occur, and the description includes instances where the event or circumstance occurs and instances where it does not.

The term "substituted" means that any one or more hydrogen atoms on a specific atom are substituted by substituents which may include deuterium and hydrogen variants, as long as the valence of the specific atom is normal and the compound after substitution is stable. When the substituent is oxygen *(i.e.,* =O), it means that two hydrogen atoms are substituted. Substitution by oxygen does not occur on aromatic groups. The term "optionally substituted" means that an atom can be or cannot be substituted by a substituent. Unless otherwise specified, the type and number of the substituent may be arbitrary as long as being chemically achievable.

When any variable *(e.g.,* R) occurs more than once in the composition or structure of a compound, the definition of the variable in each case is independent. Thus, for example, if a group is substituted by 0-2 R, the group can be optionally substituted with two R at most, and the definition of R in each case is independent. Furthermore, a combination of a substituent and/or a variant thereof is permissible only if the combination can result in a stable compound.

When the number of a connecting group is 0, such as -(CRR)₀-, it means that the connecting group is a single bond.

When a variable is a single bond, it means that the two groups are directly connected. For example, in A-L-Z, when L represents a single bond, it means that the structure is actually A-Z.

When a substituent is absent, it means that there is no such a substituent in a structure, *e.g.,* when X is absent in A-X, it means that the structure is actually A. When it is not specified by which atom the listed substituent is connected to the group to be substituted, the substituent can be connected via any atom of the group. For example, pyridinyl as a substituent can be connected to the group to be substituted via any carbon atom on the pyridine ring. When the listed connecting group does not indicate the direction for connecting, the direction is arbitrary. For example, when the connecting group L contained in 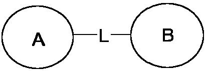 is -M-W-, -M-W-can either connect ring A and ring B to form 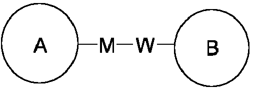 in a direction same as left-to-right reading order, or connect ring A and ring B to form 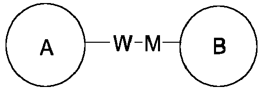 in a direction contrary to the left-to-right reading order. A combination of the connecting group, a substituent and/or a variant thereof is permissible only if the combination can result in a stable compound.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, *etc.,* and may be monovalent *(e.g.,* methyl), divalent *(e.g.,* methylene), or polyvalent *(e.g.,* methenyl). Examples of C₁₋₆ alkyl include, but are not limited to, methyl (Me), ethyl (Et), propyl (including *n-*propyl and isopropyl), butyl (including *n*-butyl, isobutyl, *s*-butyl, and *t*-butyl), pentyl (including *n*-pentyl, isopentyl, and neopentyl), hexyl, and the like.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂₋₃ alkyl, *etc.,* and may be monovalent *(e.g.,* methyl), divalent *(e.g.,* methylene), or polyvalent *(e.g.,* methenyl). Examples of C₁₋₃ alkyl include, but are not limited to, methyl (Me), ethyl (Et), propyl (including *n*-propyl and isopropyl), and the like.

Unless otherwise specified, "C₃₋₆ cycloalkyl" refers a saturated cyclic hydrocarbon group consisting of 3 to 6 carbon atoms, which includes monocyclic and bicyclic ring systems, wherein the carbon atoms may optionally be oxidized *(i.e.,* C=O). The C₃₋₆ cycloalkyl includes C₃₋₅, C₄₋₅, C₅₋₆ cycloalkyl and the like, and may be monovalent, divalent or polyvalent. Examples of C₃₋₆ cycloalkyl include, but are not limited to, cyclopropyl, cyclobutyl, cyclopentyl, cyclohexyl and the like.

Unless otherwise specified, the term "3-6 membered heterocycloalkyl", by itself or in combination with other terms, refers to a saturated cyclic group consisting of 3 to 6 ring atoms, of which 1, 2, 3, or 4 ring atoms are heteroatoms independently selected from O, S, and N, with the remaining being carbon atoms. The nitrogen atom is optionally quaternized, and the nitrogen and sulfur heteroatoms can be optionally oxidized *(i.e.,* NO and S(O)ₚ, where p is 1 or 2). This includes monocyclic and bicyclic systems, wherein the bicyclic system includes spirocyclic, fused, and bridged rings. Furthermore, with respect to the "3-6 membered heterocycloalkyl", a heteroatom may occupy the position where the heterocycloalkyl is attached to the rest of the molecule. The 3-6 membered heterocycloalkyl includes 4-6 membered, 5-6 membered, 4 membered, 5 membered and 6 membered heterocycloalkyl, and the like. Examples of 3-6 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, *etc.),* tetrahydrofuranyl (including tetrahydrofuran-2-yl, *etc.),* tetrahydropyranyl, piperidinyl (including 1-piperidinyl, 2-piperidinyl, 3-piperidinyl, *etc.),* piperazinyl (including 1-piperazinyl, 2-piperazinyl, *etc.),* morpholinyl (including 3-morpholinyl, 4-morpholinyl, *etc.),* dioxanyl, dithianyl, isoxazolidinyl, isothiazolidinyl, 1,2-oxazinyl, 1,2-thiazinyl, hexahydropyridazinyl, homopiperazinyl, homopiperidinyl, *etc.*

Unless otherwise specified, Cₙ₋ₙ₊ₘ or Cₙ₋Cₙ₊ₘ includes any one of the specific cases of n to n+m carbons; for example, C₁₋₁₂ includes C₁, C₂, C₃, C₄, C₅, C₆, C₇, C₈, C₉, C₁₀, C₁₁ and C₁₂; Cₙ₋ₙ₊ₘ or Cₙ-Cₙ₊ₘ also includes any ranges in n to n+m; for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, *etc.* Similarly, n-n+m membered represents the number of atoms on the ring is n to n+m; for example, 3-12 membered ring includes 3 membered ring, 4 membered ring, 5 membered ring, 6 membered ring, 7 membered ring, 8 membered ring, 9 membered ring, 10 membered ring, 11 membered ring and 12 membered ring; n-n+m membered also represents any range in n to n+m; for example, 3-12 membered ring includes 3-6 membered ring, 3-9 membered ring, 5-6 membered ring, 5-7 membered ring, 6-7 membered ring, 6-8 membered ring, 6-10 membered ring, *etc.*

The term "leaving group" refers to a functional group or atom that can be substituted by another functional group or atom through a substitution reaction *(e.g.,* an affinity substitution reaction). For example, representative leaving groups include triflate; chlorine; bromine; iodine; sulfonate groups such as methanesulfonate, toluenesulfonate, *p*-bromobenzenesulfonate, and *p*-toluenesulfonate; and acyloxy groups such as acetoxy and trifluoroacetyloxy.

The term "protecting group" includes, but is not limited to, "amino protecting group", "hydroxyl protecting group" or "thiol protecting group". The term "amino protecting group" refers to a protecting group suitable for use in preventing side reaction at the amino nitrogen position. Representative amino protecting groups include, but are not limited to: formyl; acyl, for example alkanoyl (such as acetyl, trichloroacetyl or trifluoroacetyl); alkoxycarbonyl such as *t*-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and *t*-butyldimethylsilyl (TBS); and the like. The term "hydroxyl protecting group" refers to a protecting group suitable for use in preventing side reaction of a hydroxyl group. Representative hydroxyl protecting groups include, but are not limited to: alkyl such as methyl, ethyl and *tert-*butyl; acyl such as alkanoyl *(e.g.,* acetyl); arylmethyl such as benzyl (Bn), *p*-methoxybenzyl (PMB), 9-fluorenylmethyl (Fm) and diphenylmethyl (DPM); silyl such as trimethylsilyl (TMS) and *t*-butyldimethylsilyl (TBS); and the like.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. Preferred embodiments include, but are not limited to, the examples disclosed herein.

The solvent used in the present invention can be commercially available. The following abbreviations are used in the present invention: aq for water; HATU for O-(7-azabenzotriazol-1-yl)-*N*,*N*,*N'*,*N'*-tetramethyluronium hexafluorophosphate; EDC for *N*-(3-dimethylaminopropyl)-*N'*-ethylcarbodiimide hydrochloride; m-CPBA for 3-chloroperoxybenzoic acid; eq for equivalent; CDI for carbonyldiimidazole; DCM for dichloromethane; PE for petroleum ether; DIAD for diisopropyl azodicarboxylate; DMF for *N*,*N*-dimethylformamide; DMSO for dimethyl sulfoxide; EtOAc for ethyl acetate; EtOH for ethanol; MeOH for methanol; CBz for benzyloxycarbonyl, an amine protecting group; BOC for *t*-butoxycarbonyl, an amine protecting group; HOAc for acetic acid; NaCNBH₃ for sodium cyanoborohydride; r.t. for room temperature; Rt for retention time; O/N for overnight; THF for tetrahydrofuran; Boc₂O for di-*tert*-butyl dicarbonate; TFA for trifluoroacetic acid; DIPEA for diisopropylethylamine; SOCl₂ for thionyl chloride; CS₂ for carbon disulfide; TsOH for *p-*toluenesulfonic acid; NFSI for *N*-fluoro-*N*-(phenylsulfonyl)benzenesulfonamide; NCS for 1-chloropyrrolidine-2,5-dione; *n*-Bu₄NF for tetrabutylammonium fluoride; iPrOH for 2-propanol; mp for melting point; LDA for lithium diisopropylamide; PPA for polyphosphoric acid; PPH₃ for triphenylphosphine; Pd(PPh₃)₄ for tetrakis(triphenylphosphine)palladium(0).

Compounds are named either manually or by ChemDraw® software, and supplier's catalog names are given for commercially available compounds.

### TECHNICAL EFFECTS

The compounds disclosed herein show relatively high kinase inhibitory activity against a plurality of kinases and mutants thereof, which is superior to that of LOXO-195 and LOXO-101. The compounds disclosed herein show obvious effect of inhibiting cell proliferation in enzyme and cell level tests and obvious effect of inhibiting tumor in the *in vivo* animal efficacy experiment.

### BRIEF DESCRIPTION OF THE DRAWINGS

FIG. 1 shows an ellipsoid plot of a three-dimensional structure of WX001B;
FIG. 2 shows the crystal packing diagram of WX001B along b-axis;
FIG. 3 shows the effect of WX001B on tumor volume in Ba/F3 ETV6-NTRK3-G623R tumor-bearing nude mice;
FIG. 4 shows the effect of WX001B on tumor volume in Ba/F3 ETV6-NTRK3 tumor-bearing nude mice;
FIG. 5 shows the effect of WX001B on tumor weight in Ba/F3 ETV6-NTRK3-G623R tumor-bearing nude mice;
FIG. 6 shows the effect of WX001B on tumor weight in Ba/F3 ETV6-NTRK3 tumor-bearing nude mice;
FIG. 7 shows the effect of WX001B on the body weight of Ba/F3 ETV6-NTRK3-G623R tumor-bearing nude mice; and
FIG. 8 shows the effect of WX001B on the body weight of Ba/F3 ETV6-NTRK3 tumor-bearing nude mice.

### DETAILED DESCRIPTION

The present invention is described in detail below by way of examples. However, this is by no means disadvantageously limiting the scope of the present invention. Although the present invention has been described in detail herein and specific examples have also been disclosed, it will be apparent to those skilled in the art that various changes and modifications can be made to the specific examples without departing from the spirit and scope of the present invention.

### Example 1: Synthesis of Compounds WX001A and WX001B

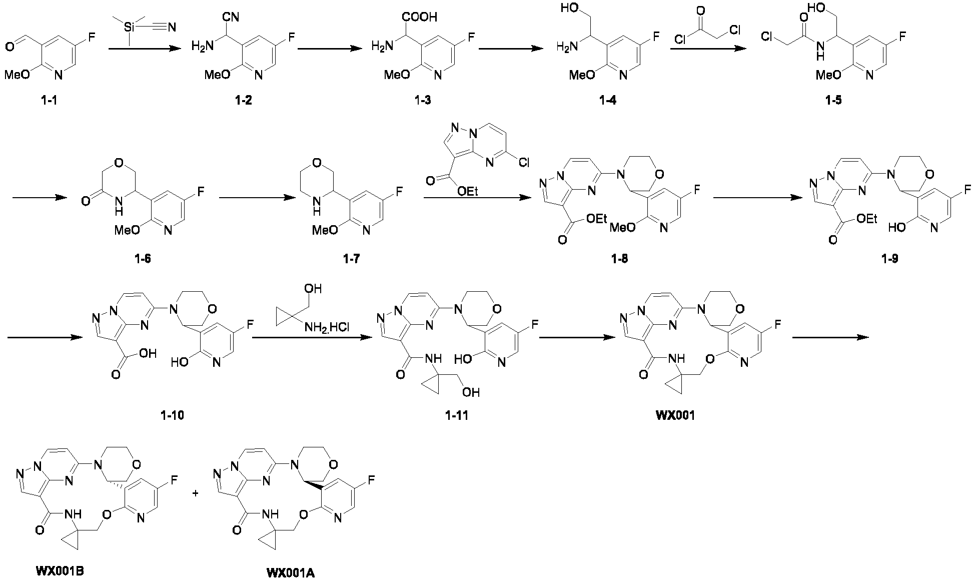

### Step 1: synthesis of compound 1-2

In a 1 L three-necked flask, compound **1-1** (20 g, 128.93 mmol, 1 *eq)* was added to a solution of ammonia gas in methanol (7 M, 199.64 mL, 10.84 *eq,* 200 mL), and trimethylsilyl cyanide (19.19 g, 193.39 mmol, 24.19 mL, 1.5 *eq)* was added dropwise at 0 °C. The reaction mixture was warmed to 20 °C, stirred for 16 hours, added with trimethylsilyl cyanide (6.40 g, 64.46 mmol, 8.06 mL, 0.5 *eq),* and then stirred for 24 hours. The reaction mixture was concentrated. The concentrated reaction mixture was added with methyl *tert*-butyl ether (100 mL), added with a 4 mol/L ethyl acetate hydrochloride solution to adjust the pH to 3-4, and filtered to give a filter cake, which was compound **1-2.** ¹H NMR (400MHz, DMSO-*d*₆) δ: 9.92 - 9.04 (m, 2H), 8.37 (d, *J*=3.0 Hz, 1H), 8.02 (dd, *J*=8.4, 2.8 Hz, 1H), 5.89 (s, 1H), 6.43 - 5.81 (m, 1H), 3.98 (s, 3H). LCMS *m*/*z* =182.0 [M+1]⁺.

### Step 2: synthesis of compound 1-3

Compound **1-2** (14 g, 64.33 mmol, 1 *eq,* hydrochloride) was added to a prepared aqueous solution of sodium hydroxide (5.15 g, 128.66 mmol, 2 *eq,* 140 mL). The reaction mixture was stirred at 90 °C under reflux for 5 hours. The reaction mixture was added with a 4 mol/L diluted hydrochloric acid to adjust the pH to 3-4, and concentrated. The concentrated solution was added with tetrahydrofuran (50 mL), and concentrated to dryness to give compound **1-3.** ¹H NMR (400MHz, DMSO-*d*₆) δ: 8.18 - 8.15 (m, 1H), 7.78 - 7.72 (m, 1H), 7.41 (s, 3H), 4.69 (s, 1H), 3.86 (s, 3H). LCMS m/z =201.0 [M+1]⁺.

### Step 3: synthesis of compound 1-4

In a 2 L three-necked flask, compound **1-3** (28.04 g, 140.08 mmol, 1 *eq)* was dissolved in tetrahydrofuran (897 mL), and the solution was added with lithium aluminum hydride (10.63 g, 280.17 mmol, 2 *eq)* at 0 °C. The reaction mixture was warmed to 20 °C, and stirred for 18 hours under nitrogen atmosphere. The reaction mixture was added with water (15 mL) to quench the reaction, added with anhydrous sodium sulfate (10 g), and filtered. The mother solution was concentrated and slowly added with a 4 mol/L ethyl acetate hydrochloride solution dropwise. A solid was precipitated, which was the product, namely compound **1-4.** ¹H NMR (400MHz, DMSO-*d*₆) δ: 8.19 (d, *J*=2.8 Hz, 1H), 7.95 (dd, *J*=9.2, 2.8 Hz, 1H), 4.43-4.41 (m, 1H), 3.92 - 3.89 (m, 3H), 3.77 - 3.70 (m, 2H). LCMS *m*/*z* =187.1 [M+1]⁺.

### Step 4: synthesis of compound 1-5

In a 100 mL three-necked flask, compound **1-4** (1.75 g, 7.86 mmol, 1 *eq,* hydrochloride) was dissolved in tetrahydrofuran (35 mL), and the solution was added with triethylamine (2.39 g, 23.58 mmol, 3.28 mL, 3 *eq),* and then added with chloroacetyl chloride (1.07 g, 9.43 mmol, 750.20 µL, 1.2 *eq)* at 0 °C. The reaction mixture was warmed to 20 °C and stirred for 10 minutes to give compound **1-5.** LCMS *m*/*z* =263.0[M+1]⁺.

### Step 5: synthesis of compound 1-6

In a 250 mL three-necked flask, sodium hydride (1.16 g, 29.02 mmol, 60% purity, 3.7 *eq)* was dissolved in tetrahydrofuran (125 mL), and the reaction mixture was added with a solution of compound **1-5** (2.06 g, 7.84 mmol, 1 *eq)* dropwise at 0 °C. The reaction mixture was warmed to 20 °C under nitrogen atmosphere, and stirred for 1 hour. The reaction mixture was added with saturated ammonium chloride (50 mL) to quench the reaction, added with water (100 mL), and extracted with ethyl acetate (100 mL × 1). The organic phase was dried over anhydrous sodium sulfate and concentrated to dryness. The crude product was purified by silica gel column chromatography (dichloromethane:methanol = 100:1 to 50:1 to 30:1 -10:1) to give compound **1-6.** LCMS *m*/*z* = 227.0 [M+1]⁺.

### Step 6: synthesis of compound 1-7

In a 100 mL single-necked flask, compound **1-6** (772 mg, 3.41 mmol, 1 *eq)* was dissolved in tetrahydrofuran (15 mL), and the solution was added with lithium aluminum hydride (518.13 mg, 13.65 mmol, 4 *eq)* at 0 °C. The reaction mixture was reacted for 1.5 hours at 50 °C. The reaction mixture was slowly added with a saturated aqueous ammonium chloride solution (15 mL) dropwise, added with water (20 mL), and extracted with dichloromethane (20 mL). The organic phase was washed with saturated brine (20 mL × 1), dried over anhydrous sodium sulfate, and concentrated to dryness to give compound **1-7.** ¹H NMR (400MHz, CDCl₃) δ: 7.86 (d, *J*=3.0 Hz, 1H), 7.64 - 7.53 (m, 1H), 4.15 (dd, *J*=9.2, 3.0 Hz, 1H), 3.97 - 3.90 (m, 1H), 3.90 (s, 3H), 3.88 - 3.80 (m, 1H), 3.59 (dt, *J*=2.8, 11.0 Hz, 1H), 3.22 (dd, *J*=108, 9.2 Hz, 1H), 3.08 (dt, *J*=11.2, 3.2 Hz, 1H), 3.00 - 2.93 (m, 1H). LCMS *m*/*z* =213.1 [M+1]⁺.

### Step 7: synthesis of compound 1-8

In a 100 mL single-necked flask, compound **1-7** (658 mg, 3.10 mmol, 1 *eq)* was dissolved in *n*-butanol (8 mL), and the solution was added with *N*,*N*-diisopropylethylamine (1.60 g, 12.40 mmol, 2.16 mL, 4 *eq)* and ethyl 5-chloropyrazolo[1,5-*a*]pyrimidine-3-carboxylate (489.71 mg, 2.17 mmol, 0.7 *eq).* The reaction mixture was stirred at 120 °C for 12 hours. The reaction mixture was added with water (10 mL) and extracted with ethyl acetate (10 mL × 3). The organic phase was washed with saturated brine (10 mL × 1), dried over anhydrous sodium sulfate, and concentrated under vacuum to give a crude product. The crude product was purified by silica gel column chromatography (petroleum ether:ethyl acetate = 10:1 to 5:1 to 1:1) to give compound **1-8.** ¹H NMR (400MHz, CDCl₃) δ: 8.39 - 8.21 (m, 2H), 7.94 (d, *J*=3.0 Hz, 1H), 7.48 (dd, *J*=8.4, 3.0 Hz, 1H), 6.39 (d, *J*=7.8 Hz, 1H), 5.25 - 5.18 (m, 1H), 4.88 - 4.81 (m, 1H), 4.46 - 4.28 (m, 3H), 4.17 (dd, *J*=11.4, 4.0 Hz, 1H), 4.02 - 3.93 (m, 1H), 3.96 (s, 3H) 3.77 (dt, *J*=3.4, 11.6 Hz, 1H), 3.65 - 3.47 (m, 1H), 1.39 (t, *J*=7.2 Hz, 3H). LCMS *m*/*z* =402.0 [M+1]⁺.

### Step 7: synthesis of compound 1-9

In a 100 mL single-necked flask, compound **1-8** (535 mg, 1.33 mmol, 1 *eq)* was dissolved in acetonitrile (10 mL), and the solution was added with sodium iodide (599.37 mg, 4.00 mmol, 3 *eq)* and trimethylchlorosilane (434.42 mg, 4.00 mmol, 507.49 µL, 3 *eq).* The reaction mixture was reacted at 75 °C for 0.5 hour. The reaction mixture was extracted with dichloromethane (10 mL × 1) and water (10 mL × 1). The organic phase was dried over anhydrous sodium sulfate and concentrated under vacuum to give compound **1-9.** ¹H NMR (400MHz, CDCl₃) δ: 8.40 - 8.27 (m, 2H), 7.61 (dd, *J*=7.6, 2.8 Hz, 1H), 6.54 (d, *J*=8.0 Hz, 1H), 5.30 - 5.20 (m, 1H), 4.86 - 4.76 (m, 1H), 4.46 - 4.28 (m, 3H), 4.20 (dd, *J*=3.8, 11.2 Hz, 1H), 3.98 (dd, *J*=12.0, 4.0 Hz, 1H), 3.77 (dt, *J*=12.0, 3.2 Hz, 1H), 3.66 - 3.52 (m, 1H), 1.39 (t, *J*=7.2 Hz, 3H). LCMS *m*/*z* =388.0 [M+1]⁺.

### Step 8: synthesis of compound 1-10

In a 100 mL single-necked flask, compound **1-9** (648 mg, 1.67 mmol, 1 *eq)* was dissolved in methanol (8 mL), and the solution was added with a prepared aqueous sodium hydroxide solution (3 M, 2.23 mL, 4 *eq,* 2.2 mL). The reaction mixture was stirred at 60 °C for 6 hours. The reaction mixture was neutralized with a 1 mol/L diluted hydrochloric acid to pH = 7, and concentrated under vacuum to give compound **1-10.** ¹H NMR (400MHz, DMSO-*d*₆) δ: 8.75 (d, *J*=7.8 Hz, 1H), 8.21 (s, 1H), 7.60 - 7.54 (m, 1H), 7.48 - 7.37 (m, 1H), 6.72 (br d, *J*=8.0 Hz, 1H), 5.37 - 5.26 (m, 1H), 4.57 - 4.47 (m, 1H), 4.41 - 4.32 (m, 1H), 4.11 - 4.02 (m, 1H), 3.88 - 3.79 (m, 1H), 3.66 - 3.47 (m, 3H). LCMS *m*/*z* =360.0 [M+1]⁺.

### Step 9: synthesis of compound 1-11

In a 100 mL three-necked flask, compound **1-10** (400 mg, 1.11 mmol, 1 *eq)* was dissolved in *N,N-*dimethylformamide (4 mL), and the solution was added with *N*,*N*-diisopropylethylamine (431.64 mg, 3.34 mmol, 581.72 µL, 3 *eq),* and then added with O-(7-azabenzotriazol-1-yl)-*N*,*N*,*N'*,*N'*-tetramethyluronium hexafluorophosphate (423.29 mg, 1.11 mmol, 1 *eq)at* 0 °C. The reaction mixture was stirred for 0.5 hour, added with (1-(hydroxymethyl)cyclopropylamino hydrochloride (137.58 mg, 1.11 mmol, 1 *eq),* warmed to 25 °C, and then stirred for 16 hours. The reaction mixture was concentrated to dryness. Slurrying and purifying: the reaction mixture was added with dichloromethane (5 mL), methanol (5 mL) and ethyl acetate (10 mL), stirred for 0.5 hour, and filtered, and the filter cake was compound 1-11. ¹H NMR (400MHz, DMSO-*d*₆) δ: 8.80 (d, *J*=8.0 Hz, 1H), 8.15 (s, 1H), 7.87 (s, 1H), 7.56 (s, 1H), 7.43 (d, *J*=6.4 Hz, 1H), 6.81 (s, 1H), 5.38 ( s, 1H), 4.69 ( s, 1H), 4.31 ( d, *J*=19.2Hz, 1H), 4.22 ( s, 1H), 4.08 - 4.02 (m, 2H), 3.89-3.85 (m, 2H), 3.66 (d, *J*=6.8 Hz, 3H), 0.75- 0.71(m, 3H), 0.60(s,1H). LCMS *m*/*z* =429.1 [M+1]⁺.

### Step 10: synthesis of compound WX001

In a 100 mL three-necked flask, compound **1-11** (120 mg, 280.10 µmol, 1 *eq)* was dissolved in dichloromethane (10 mL), and the solution was added with triphenylphosphine (220.40 mg, 840.30 µmol, 3 *eq),* and then added with diethyl azodicarboxylate (220.40 mg, 840.30 µmol, 3 *eq)* dropwise at 0 °C. The reaction mixture was warmed to 25 °C, stirred for 1 hour, supplemented with triphenylphosphine (220.40 mg, 840.30 µmol, 3 *eq)* and diethyl azodicarboxylate (243.91 mg, 1.40 mmol, 254.60 µL, 5 *eq),* and then stirred for 5 hours. The reaction mixture was extracted with water (15 mL × 1). The organic phase was dried over anhydrous sodium sulfate and concentrated under vacuum. The crude product was purified by silica gel column chromatography (dichloromethane:methanol = 100:0 to 20:1 to 15:1), added with ethyl acetate (10 mL), stirred for 0.5 hour at 25 °C, and then filtered to give a filter cake, which was the product **WX001**. LCMS *m*/*z*=411.0 [M+1]⁺.

### Step 11: synthesis of compounds WX001A and WX001B

**WX001** (68 mg, 165.69 µmol, 1 *eq)* was resolved by SFC (column: DAICEL CHIRALPAK AD-H (250mm×30mm, 5µm); mobile phase: A (CO₂) and B (methanol, with 0.1% ammonium hydroxide solution); gradient: B% = 42%, 10 min) to give **WX001A** and **WX001B**.

**WX001A:** ¹H NMR (400MHz, CDCl₃) δ: 8.98 (s, 1H), 8.34 (d, *J*=8.0 Hz, 1H), 8.27 (s, 1H), 7.90 (d, *J*=2.8 Hz, 1H), 7.76 (dd, *J*=8.4, 2.8 Hz, 1H), 6.37 (d, *J*=7.6 Hz, 1H), 6.15 (s, 1H), 5.08 (d, *J*=10.8 Hz, 1H), 4.41 - 4.39 (m, 1H), 4.13 - 3.88 (m, 4H), 3.78 - 3.75 (m, 1H), 3.60 (d, *J*=10.8 Hz, 1H), 2.28 - 2.22 (m, 1H), 1.05 - 0.97 (m, 2H), 0.84 - 0.78 (m, 1H). LCMS *m*/*z*=411.0 [M+1]⁺. SFC (column: Chiralcel AD-3, 3 µm, 0.46 cm id × 10 cm L; mobile phase: A (CO₂) and B (MeOH, with 0.05% isopropylamine); gradient: B% = 5-40%, 4 min; flow rate: 4.0 mL/min; wavelength: 220 nm; pressure: 100 bar), retention time = 2.00 min, chiral isomer excess 100.00%

**WX001B:** ¹H NMR (400MHz, CDCl₃) δ: 8.98 (s, 1H), 8.33 (d, *J*=7.6Hz, 1H), 8.26 (s, 1H), 7.89 (d, *J*=2.4 Hz, 1H), 7.77 - 7.75 (m, 1H), 6.36 (d, *J*=7.6 Hz, 1H), 6.15 (s, 1H), 5.07 (d, *J*=10.8 Hz, 1H), 4.39 (d, *J*=9.6 Hz, 1H), 4.12 - 3.88 (m, 4H), 3.76 (d, *J*=10.0 Hz, 1H), 3.60 (d, *J*=10.8 Hz, 1H), 2.27 - 2.22 (m, 1H), 1.07-0.94 (m, 2H), 0.84 - 0.79 (m, 1H). LCMS *m*/*z*=411.0 [M+1]⁺. SFC (column: Chiralcel AD-3, 3 µm, 0.46 cm id × 10 cm L; mobile phase: A (CO₂) and B (MeOH, with 0.05% isopropylamine); gradient: B% = 5-40%, 4 min; flow rate: 4.0 mL/min; wavelength: 220 nm; pressure: 100 bar), retention time = 2.43 min, chiral isomer excess 100.00%.

### Example 2: Synthesis of Compounds WX002A and WX002B

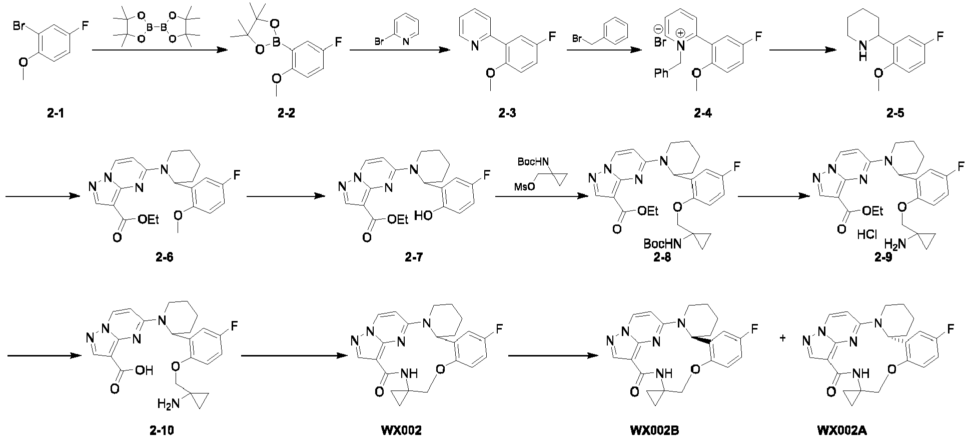

### Step 1: synthesis of compound 2-2

[1,1'-bis(diphenylphosphino)ferrocene]palladium(II)dichloride (812.63 mg, 1.11 mmol, 0.033 *eq)* was added to a suspension of compound **2-1** (6.9 g, 33.65 mmol, 1 *eq),* bis(pinacolato)diboron (9.32 g, 36.68 mmol, 1.09 *eq)* and potassium acetate (12.98 g, 132.26 mmol, 3.93 *eq)* in *N*,*N-*dimethylformamide (90 mL) at 16 °C. The resulting reaction mixture was stirred and purged three times with nitrogen at 16 °C, and then heated to 110 °C and stirred for 16 hours. The resulting reaction mixture was diluted with water (300 mL), and extracted with ethyl acetate (300 mL × 3). The organic phases were combined, washed with saturated brine (100 mL × 3), dried over anhydrous MgSO₄, filtered, and concentrated under vacuum to remove the solvent to give compound **2-2,** which was used directly in the next step. ¹H NMR (400MHz, CDCl₃) δ: 7.29 - 7.26 (m, 1 H), 6.98 - 6.96 (m, 1 H), 6.73 - 6.69 (m, 1 H), 3.73 (s, 3 H), 1.28 (s, 12 H).

### Step 2: synthesis of compound 2-3

Potassium phosphate (21.31 g, 100.39 mmol, 3.11 *eq)* was added to a suspension of compound **2-2** (8.31 g, 32.96 mmol, 1.02 *eq),* 2-bromopyridine (5.1 g, 32.28 mmol, 3.07 mL, 1 *eq)* and palladium acetate (652.23 mg, 2.91 mmol, 0.09 *eq)* in isopropanol (90 mL) at 16 °C. The resulting dark brown reaction mixture was stirred and purged three times with nitrogen at 16 °C, and then heated to 91 °C and stirred for 16 hours to give a dark brown suspension. The resulting reaction mixture was concentrated under vacuum to remove the solvent to give a crude product. The crude product was purified by column chromatography (petroleum ether:ethyl acetate = 10:1 to 3:1) to give compound **2-3.** ¹H NMR (400MHz, CDCl₃) δ: 8.69 - 8.68 (d, *J*=8.0 Hz, 1 H), 8.39 - 8.37 (d, *J*=8.0 Hz,1 H), 7.85 - 7.80 (m, 1 H), 7.33 - 7.29 (m, 1 H), 6.99 - 6.97 (m, 1 H), 6.90 - 6.89 (m, 1 H), 3.7(s, 3 H). LCMS *m*/*z :* 203.6[M+1]⁺.

### Step 3: synthesis of compound 2-4

Benzyl bromide (2.33 g, 13.65 mmol, 1.62 mL, 1.39 *eq)* was added to a solution of compound **2-3** (3.99 g, 9.82 mmol, 1 *eq)* in acetonitrile (60 mL) in portions at 16 °C. The resulting reaction mixture was heated to 91 °C and stirred for 16 hours. The resulting reaction mixture was concentrated under vacuum to remove the solvent to give a crude product. The crude product was purified by column chromatography (ethyl acetate:methanol = 100:3) to give compound **2-4.** LCMS *m*/*z* : 294.4[M+1]⁺.

### Step 4: synthesis of compound 2-5

Dry palladium hydroxide/carbon (1 g) was added to a solution of compound **2-4** (3 g, 6.41 mmol, 1 *eq)* in MeOH (90 mL) at 16 °C under nitrogen atmosphere. The resulting reaction mixture was stirred and purged three times with hydrogen at 16 °C, and then heated to 60 °C and stirred under H₂ atmosphere (50 Psi) for 16 hours. The resulting reaction mixture was cooled to room temperature (25 °C), and filtered under reduced pressure, and the filter cake was washed with methanol (30 mL × 3). The filtrates were combined and concentrated under vacuum to remove the solvent to give a crude product. The crude product was purified by column chromatography (petroleum ether:ethyl acetate = 1:1) to give compound **2-5.** LCMS *m*/*z*: 209.8[M+1]⁺.

### Step 5: synthesis of compound 2-6

Potassium fluoride (2.19 g, 37.67 mmol, 882.51 µL, 9 *eq)* was added to a solution of compound **2-5** (1.1 g, 4.19 mmol, 1 *eq)* and ethyl 5-chloropyrazolo[1,5-*a*]pyrimidine-3-carboxylate (972.72 mg, 4.31 mmol, 1.03 *eq)* in dimethyl sulfoxide (9 mL) in portions at 16 °C. The resulting reaction mixture was heated to 191 °C and stirred for 1 hour to give a dark brown suspension. The resulting reaction mixture was added with water (30 mL) to quench the reaction, and extracted with ethyl acetate (30 mL × 3). The organic phases were combined, washed with saturated brine, dried over anhydrous magnesium sulfate, filtered, and concentrated to dryness to remove the solvent. The crude product was purified by column chromatography (petroleum ether:ethyl acetate = 10:1 to 3:1) to give compound **2-6** in the form of a pale yellow solid. LCMS *m*/*z*: 399.4[M+1]⁺.

### Step 6: synthesis of compound 2-7

Boron tribromide (599.61 mg, 2.39 mmol, 230.6 µL, 3.99 *eq)* was added to a solution of compound **2-6** (0.239 g, 599.85 µmol, 1 *eq)* in dichloromethane (9 mL) in portions at -78 °C. The cold bath was then removed and the resulting reaction mixture was stirred at 26 °C for 1 hour. The resulting reaction mixture was added with water (30 mL) to quench the reaction, and then added with solid sodium carbonate to adjust the pH > 9 and allow the reaction mixture to be saturated. The suspension was extracted with dichloromethane (30 mL × 3). The organic phases were combined, dried over anhydrous magnesium sulfate, filtered, and concentrated under vacuum to remove the solvent. The resulting crude product was purified by preparative silica gel thin layer chromatography (dichloromethane:methanol = 6:1) to give compound **2-7** in the form of a pale yellow solid. LCMS *m*/*z* : 385.2[M+1]⁺.

### Step 7: synthesis of compound 2-8

Cesium carbonate (526.36 mg, 1.62 mmol, 9 *eq)* was added to a solution of compound **2-7** (69 mg, 179.50 µmol, 1 *eq)* and (1-*tert*-butoxycarbonylamino)cyclopropylmethyl methanesulfonate (300.04 mg, 1.13 mmol, 6.3 *eq)* in *N*,*N-*dimethylformamide (3 mL) at 16 °C. The resulting reaction mixture was heated to 91 °C and stirred for 16 hours. The resulting reaction mixture was added with water (10 mL) to quench the reaction, and extracted with ethyl acetate (10 mL × 3). The organic phases were combined, washed with saturated brine (10 mL × 3), dried over anhydrous MgSO₄, filtered, and concentrated under vacuum to remove the solvent to give a crude product. The crude product was purified by thin layer chromatography (petroleum ether:ethyl acetate = 1:1) to give compound **2-8.** LCMS *m*/*z*: 576.4[M+Na]⁺.

### Step 8: synthesis of compound 2-9

A solution of hydrogen chloride in ethyl acetate (4 M, 3.05 mL, 113 *eq)* was added to a solution of compound **2-8** (59.8 mg, 108.02 µmol, 1 *eq)* in methanol (3 mL) at 16 °C. The resulting reaction mixture was stirred at 16 °C for 1 hour. The resulting reaction mixture was concentrated under vacuum to give compound **2-9.** LCMS *m*/*z*: 476.3[M+ Na]⁺.

### Step 9: synthesis of compound 2-10

An aqueous sodium hydroxide solution (6 M, 2.98 mL, 139 *eq)* was added to a solution of compound **2-9** (63 mg, 128.58 µmol, 1 *eq,* hydrochloride) in methanol (3 mL) at 21 °C. The resulting reaction mixture was heated to 69 °C and stirred for 1 hour. The reaction mixture was cooled to room temperature, added with diluted hydrochloric acid (6 M) to adjust the pH to 5-6, and concentrated under vacuum to remove the solvent. The resulting pale yellow solid was added with methanol (6 mL), thoroughly triturated, and filtered to remove insoluble solids. The resulting filtrate was concentrated in vacuum to remove the solvent. Compound **2-10** was obtained and used directly in the next step. LCMS *m*/*z*: 426.2 [M+1]⁺.

### Step 10: synthesis of compound WX002

O-(7-azabenzotriazol-1-yl)-*N*,*N*,*N*',*N*'-tetramethyluronium hexafluorophosphate (143.23 mg, 376.69 µmol, 3 *eq)* was added to a solution of compound **2-10** (58 mg, 125.56 µmol, 1 *eq,* hydrochloride) and triethylamine (114.35 mg, 1.13 mmol, 157.29 µL, 9 *eq)* in *N*,*N*-dimethylformamide (3 mL) at 26 °C. The resulting reaction mixture was stirred at 18-26 °C for 16 hours. The resulting reaction mixture was added with water (10 mL) to quench the reaction, and extracted with ethyl acetate (10 mL × 3). The organic phases were combined, washed with saturated brine (10 mL × 3), dried over anhydrous magnesium sulfate, filtered, and concentrated under vacuum to remove the solvent. The resulting crude product was purified by thin layer chromatography (dichloromethane:methanol = 6:1). Compound **WX002** was obtained and used directly in the subsequent SFC resolution. LCMS *m*/*z*: 408.3 [M+1]⁺.

### Step 11: synthesis of compound WX002A

Compound **WX002** (31 mg, 76.08 µmol, 1 *eq)* was resolved by SFC (column: DAICEL CHIRALCEL OD-H (250mm×30mm, 5µm), mobile phase: A (CO₂) and B (ethanol, with 0.1% ammonium hydroxide solution); gradient: B% = 40%-40%, 20 min) to give **WX002A** and **WX002B.**

**WX002A:** ¹H NMR (400MHz, CDCl₃) δ: 9.55 (s, 1 H), 8.44 - 8.42 (d, *J*=8.0 Hz,1 H), 8.06 (s, 1 H), 7.18-7.16 (d, *J*=8.0 Hz,1 H), 6.91 - 6.88 (m, 2 H), 6.79-6.76 (d, *J*=8.0 Hz, 1 H), 6.39 - 6.36 (m, 1 H), 4.39 - 4.37 (m, 1 H), 4.19 - 4.13 (m, 1 H), 3.96 - 3.95 (m, 1 H), 3.93 - 3,89 (m, 1 H), 2.10 - 2.03 (m, 3 H), 1.93 - 1.89 (m, 2 H), 1.73 - 1.63 (m, 1 H), 1.08 - 1.05 (m, 1 H), 0.91 - 0.86 (m, 3 H). LCMS *m*/*z*: 408.1 [M+1]⁺. SFC (column: Chiralcel OD-3, 3 µm, 0.46 cm id × 10 cm L; mobile phase: A (CO₂) and B (EtOH, with 0.05% diethylamine); gradient: B% = 5-40%, 8 min; flow rate: 2.8 mL/min; wavelength: 220 nm; pressure: 100 bar), retention time = 4.585 min, chiral isomer excess 100%.

**WX002B:** ¹H NMR (400MHz, CDCl₃) δ: 9.44 (s, 1 H), 8.34 - 8.32 (d, *J*=8.0 Hz,1 H), 7.96 (s, 1 H), 7.08 - 7.06 (d, *J*=8.0 Hz,1 H), 6.80 - 6.78 (m, 2 H), 6.68 - 6.66 (d, *J*=8.0 Hz, 1 H), 6.29 - 6.26 (m, 1 H), 4.29 - 4.27 (m, 1 H), 4.06 - 4.03 (m, 1 H), 3.86 - 3.85 (m, 1 H), 3.83 - 3,78 (m, 1 H), 1.98 - 1.93 (m, 3 H), 1.93 - 1.81 (m, 2 H), 1.63 - 1.56 (m, 1 H), 0.96 - 0.93 (m, 1 H), 0.81 - 0.76 (m, 3 H). LCMS *m*/*z*: 408.1 [M+1]⁺. SFC (column: Chiralcel OD-3, 3 µm, 0.46 cm id × 10cm L; mobile phase: A (CO₂) and B (EtOH, with 0.05% diethylamine); gradient: B% = 5-40%, 8 min; flow rate: 2.8 mL/min; wavelength: 220 nm; pressure: 100 bar), retention time = 5.051 min, chiral isomer excess 100%.

### Example 3: Synthesis of Compounds WX003A and WX003B

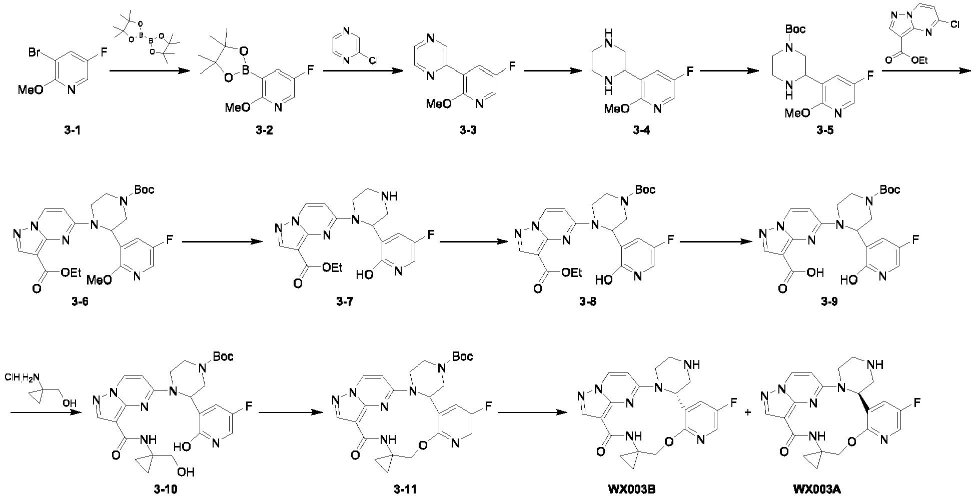

### Step 1: synthesis of compound 3-2

1,4-dioxane (150 mL), compound **3-1** (48.54 mmol, 1 *eq),* bis(pinacolato)diboron (12.33 g, 48.54 mmol, 1 *eq),* and potassium acetate (9.53 g, 97.08 mmol, 2 *eq)* were added to a prepared clean reaction flask and purged with nitrogen. The reaction mixture was added with [1,1'-bis(diphenylphosphino)ferrocene]palladium(II)dichloride dichloromethane complex (3.96 g, 4.85 mmol, 0.1 *eq),* warmed to 100 °C, and reacted for 45 minutes. After the reaction was completed, as detected by TLC (petroleum ether:ethyl acetate = 5:1), the reaction mixture was added with water (50 mL), and stirred for 3 minutes, followed by liquid separation. The organic phase was washed with saturated brine (50 mL × 1), and dried over anhydrous sodium sulfate (6 g). The resulting reaction mixture was filtered, and the filtrate was concentrated under reduced pressure at 45 °C to give compound **3-2.** ¹H NMR (400 MHz, CDCl₃) δ: 8.03 - 8.04 (m, 1H), 7.72 - 7.70 (m, 1H), 3.93 (s, 3H), 1.35 (s, 12H).

### Step 2: synthesis of compound 3-3

The reaction mixture was added with tetrahydrofuran (115 mL), water (144 mL), and compound **3-2** (14.4 g, 56.90 mmol, 1 *eq),* and stirred. Then the reaction mixture was added with 2-chloropyrazine (7.17 g, 62.59 mmol, 5.60 mL, 1.1 *eq*) and potassium carbonate (15.73 g, 113.80 mmol, 2 *eq*) sequentially, and purged with nitrogen three times. The resulting reaction mixture was added with Pd(dppf)Cl₂·CH₂Cl₂ (2.32 g, 2.84 mmol, 0.05 *eq),* warmed to 80 °C, and reacted for 1 hour. After the reaction was completed, liquid separation was performed. The aqueous phase was washed with dichloromethane (50 mL × 2). The organic phases were combined, washed with saturated brine (40 mL × 1), and dried over anhydrous sodium sulfate (10 g). The resulting reaction mixture was filtered, and the filtrate was concentrated under reduced pressure at 50 °C. The crude product was purified by silica gel column chromatography (petroleum ether:ethyl acetate = 100:0 to 50:50) to give compound **3-3.** ¹H NMR (400 MHz, CDCl₃) δ: 9.38 (d, *J=* 1.6 Hz, 1H), 8.52 (d, *J=* 2.8 Hz, 1H), 8.17 - 8.54 (m, 1H), 8.09 (d, *J=* 3.2 Hz, 1H), 4.05 (s, 3H)). LCMS *m*/*z* :206.0 [M+1]⁺.

### Step 3: synthesis of compound 3-4

Methanol (75 mL) and compound **3-3** (5.1 g, 24.86 mmol, 1 *eq)* were added to the reaction flask, and the reaction mixture was added with Pd/C (1.0 g, 50% purity). The resulting reaction mixture was warmed to 50 °C, and reacted under H₂ (50 psi) atmosphere for 15 hours. After the reaction was completed, the resulting reaction mixture was filtered. The filtrate was concentrated under reduced pressure at 55 °C to give compound **3-4.** LCMS *m*/*z* : 212.1 [M+1]⁺.

### Step 4: synthesis of compound 3-5

Dichloromethane (100 mL) and compound **3-4** (5.95 g, 28.17 mmol, 1 *eq)* were added to a reaction flask, and then stirred. The reaction mixture was added with triethylamine (5.70 g, 56.34 mmol, 7.84 mL, 2 *eq)* and Boc-anhydride (4.30 g, 19.72 mmol, 4.53 mL, 0.7 *eq)* sequentially, and reacted at 25-30 °C for 10 hours. After the reaction was completed, the reaction mixture was added with water (50 mL), and stirred for 5 minutes. Liquid separation was performed. The organic phase was washed with saturated brine (50 mL × 1), and dried over anhydrous sodium sulfate (5 g). The resulting reaction mixture was filtered, and the filtrate was concentrated under reduced pressure at 50 °C. The crude product was purified by silica gel column chromatography (dichloromethane:methanol = 100:0 to 85:15) to give compound **3-5.** LCMS *m*/*z:* 312.1 [M+1]⁺.

### Step 5: synthesis of compound 3-6

*n*-butanol (90 mL) and ethyl 5-chloropyrazolo[1,5-*a*]pyrimidine-3-carboxylate (5.20 g, 23.05 mmol, 1.2 *eq)* were added to a reaction flask, and then stirred. The reaction mixture was added with compound **3-5** (5.98 g, 19.21 mmol, 1 *eq)* and diisopropylethylamine (14.89 g, 115.24 mmol, 20.07 mL, 6 *eq)* sequentially, warmed to 120 °C, and reacted for 20 hours. After the reaction was completed, the reaction mixture was added with water (200 mL) and dichloromethane (150 mL), and stirred for 5 minutes, followed by liquid separation. The aqueous phase was washed with dichloromethane (150 mL × 1). The organic phases were combined, washed with saturated brine (150 mL × 1), and added over anhydrous sodium sulfate (5 g). The resulting reaction mixture was filtered, and the filtrate was concentrated under reduced pressure at 50 °C. The crude product was purified by silica gel column chromatography (petroleum ether:ethyl acetate = 100:1 to 30:70) to give compound **3-6.** ¹H NMR (400 MHz, CDCl₃) δ: 8.31 (s, 1H), 8.25 (d, *J=* 7.6 Hz, 1H), 7.95 (d, *J=* 2.8 Hz, 1H), 7.23 (s, 1H), 6.11 (s, 1H), 5.26 (s, 1H), 4.76 - 4.55 (m, 1H), 4.38 - 4.33 (m, 2H), 4.15 - 3.96 (m, 5H), 4.72 - 3.72 (m, 2H), 1.41 - 1.30 (m, 9H), 1.26 - 1.25 (m, 3H). LCMS m/z: 501.2 [M+1]⁺.

### Step 6: synthesis of compound 3-7

Acetonitrile (75 mL) and compound **3-6** (5.2 g, 10.39 mmol, 1 *eq)* were added to a reaction flask, and then stirred. Trimethylchlorosilane (3.39 g, 31.17 mmol, 3.96 mL, 3 *eq)* and sodium iodide (4.67 g, 31.17 mmol, 3 *eq)* were added to the reaction mixture sequentially. After being reacted at 25-30 °C for 0.5 hour, the reaction mixture was heated to 50 °C, and then reacted for 1 hour. After the reaction was completed, the reaction mixture was filtered directly. The filter cake was rinsed with acetonitrile (10 mL × 2), and concentrated under reduced pressure at 50 °C to give compound **3-7.** LCMS *m*/*z*: 387.1 [M+1]⁺.

### Step 7: synthesis of compound 3-8

Dichloromethane (90 mL) and compound **3-7** (6.3 g, 16.31 mmol, 1 *eq)* were added to a reaction flask, and then stirred. The reaction mixture was added with triethylamine (3.30 g, 32.61 mmol, 4.54 mL, 2 *eq)* and Boc-anhydride (1.96 g, 8.97 mmol, 2.06 mL, 0.55 *eq)* sequentially, and reacted at 25-30 °C for 3 hours. After the reaction was completed, the reaction mixture was added with water (50 mL), followed by liquid separation. The organic phase was washed with saturated brine (50 mL × 1), and dried over anhydrous sodium sulfate (5 g). The resulting reaction mixture was filtered, and the filtrate was concentrated under reduced pressure at 50 °C. The crude product was purified by silica gel column chromatography (dichloromethane:methanol = 100:0 to 90:10) to give compound **3-8.** ¹H NMR (400 MHz, CDCl₃) δ: 8.31 - 8.29 (m, 2H), 7.39 - 7.36 (m, 1H), 7.31 - 7.30 (m, 1H), 6.23 (s, 1H), 5.29 (s, 1H), 4.57 (s, 1H), 4.35 *(q, J=* 6.8 Hz, 2H), 4.29 - 3.93 (m, 2H), 3.86 - 3.50 (m, 2H), 3.51 - 3.48 (m, 1H), 1.55 - 1.24 (m, 12H). LCMS *m*/*z*: 487.2 [M+1]⁺.

### Step 8: synthesis of compound 3-9

Methanol (20 mL) and compound **3-8** (1 g, 2.06 mmol, 1 *eq)* were added to a reaction flask, and then stirred. The reaction mixture was added with sodium hydroxide (3 M, 2.06 mL, 3 *eq),* warmed to 70 °C, and reacted for 12 hours. After the reaction was completed, the reaction mixture was added with water (30 mL), stirred for 5 minutes, and added with a hydrochloric acid solution (0.5 M) to adjust the pH to 5-6. A white solid was precipitated, and the reaction mixture was stirred at room temperature for 30 minutes and filtered. The filter cake was dried at 50 °C to give compound **3-9.** LCMS *m*/*z*: 459.1 [M+1]⁺.

### Step 9: synthesis of compound 3-10

*N*,*N*-dimethylformamide (15 mL) and compound **3-9** (830 mg, 1.81 mmol, 1 *eq)* were added to a prepared clean reaction flask, and then stirred. After being cooled to 0-5 °C, the reaction mixture was added with diisopropylethylamine (818.95 mg, 6.34 mmol, 1.10 mL, 3.5 *eq)* and O-(7-azabenzotriazol-1-yl)-*N*,*N*,*N'*,*N'-*tetramethyluronium hexafluorophosphate (688.40 mg, 1.81 mmol, 1 *eq),* and reacted at 0-5 °C for 1 hour. The compound (1-(hydroxymethyl)cyclopropylamino hydrochloride (290.86 mg, 2.35 mmol, 1.3 *eq,* hydrochloride) was dissolved in *N*,*N*-dimethylformamide (0.3 mL), and then the solution was added dropwise to the reaction mixture. The resulting reaction mixture was warmed to 25-30 °C, and reacted for 15 hours. After the reaction was completed, the reaction mixture was added with an appropriate amount of saturated ammonium chloride solution, and stirred for 15 minutes, and then concentrated at 60 °C under reduced pressure to give a crude product. The crude product was purified by silica gel column chromatography (petroleum ether:ethyl acetate = 100:0 to 85:15), slurried with methyl *tert*-butyl ether (10 mL) and ethyl acetate (2 mL) for 1 hour, and filtered. The filter cake was slurried with methyl *tert*-butyl ether (10 mL) and ethyl acetate (2 mL) for 1 hour, and filtered. The filter cake was dried at 50 °C to give compound **3-10.** ¹H NMR (400 MHz, CDCl₃) δ: 8.71 (s, 1H), 8.14 (s, 1H), 7.53 (s, 1H), 7.38 (s, 1H), 6.83 (s, 1H), 5.33 (s, 1H), 4.45 (s, 1H), 4.00 - 4.17 (m, 2H), 3.62 - 3.53 (m, 2H), 3.30 (s, 1H), 3.09 (q, *J*= 7.6 Hz, 2H), 1.29 (s, 2H), 1.25 (s, 9H), 0.75 (s, 2H). LCMS *m*/*z*: 528.2 [M+1]⁺.

### Step 10: synthesis of compound 3-11

Dichloromethane (20 mL) and compound **3-10** (300 mg, 568.67 µmol, 1 *eq)* were added to a reaction flask, and then stirred. After being cooled to 0-5 °C, the reaction mixture was added with diethyl azodicarboxylate (990.40 mg, 5.69 mmol, 1.03 mL, 10 *eq)* and triphenylphosphine (1.49 g, 5.69 mmol, 10 *eq).* The reaction mixture was warmed to 25-30 °C, and reacted for 1 hour. After the reaction was completed, the reaction mixture was added with a saturated ammonium chloride solution (30 mL), and stirred for 2 minutes. Liquid separation was performed. The organic phase was washed with saturated brine (20 mL × 1), and dried over anhydrous sodium sulfate (1 g). The resulting reaction mixture was filtered, and the filtrate was concentrated under reduced pressure at 50 °C. The crude product was purified by silica gel column chromatography (dichloromethane:methanol = 100:0 to 90:10) to give compound **3-11.** ¹H NMR (400 MHz, CDCl₃) δ: 8.90 (s, 1H), 8.34 (d, *J* = 8.0 Hz, 1H), 8.25 (s, 1H), 7.90 (d, *J=* 2.8 Hz, 1H), 7.37 (d, *J=* 6.4 Hz, 1H), 6.42 (d, *J=* 8.0 Hz, 1H), 6.21 (s, 1H), 5.14 (d, *J=* 10.8 Hz, 1H), 3.98 - 3.93 (m, 3H), 3.77 - 3.69 (m, 2H), 3.49 (s, 1H), 2.18 - 2.22 (m, 1H), 1.64 (s, 1H), 1.39 (s, 9H), 1.12 (s, 1H), 1.05 - 1.00 (m, 1H), 0.86 - 0.81 (m, 1H). LCMS *m*/*z:* 510.3 [M+1]⁺.

### Step 11: synthesis of compounds WX003A and WX003B

Ethyl acetate (0.5 mL) and compound 3-11 (290 mg, 569.15 µmol, 1 *eq)* were added to a reaction flask, and then stirred. After being cooled to 0-5 °C, the reaction mixture was added with a solution of hydrogen chloride in ethyl acetate (4 M, 5 mL, 35.14 *eq),* and reacted at 0-5 °C for 1 hour. After the reaction was completed, the reaction mixture was filtered directly. The filter cake was rinsed with ethyl acetate (3 mL × 2), and dried at 50 °C. The solid was slurried with methanol (3 mL) for 20 minutes. The mixture was filtered. The filter cake was rinsed with methanol (0.5 mL × 1) to give a crude product, which was resolved by SFC (column: DAICEL CHIRALPAK AD-H (250mm×30mm, 5µm), mobile phase: A (CO₂) and B (ethanol, with 0.1% ammonium hydroxide solution); gradient: B% = 41%) to give **WX003A** and **WX003B.**

**WX003A:** ¹H NMR (400 MHz, CDCl₃) δ: 9.06 (s, 1H), 8.31 (d, *J=* 8.0 Hz, 1H), 8.25 (s, 1H), 7.93 (dd, *J=* 2.8, 8.8 Hz, 1H), 7.87 (d, *J=* 2.8 Hz, 1H), 6.38 (d, *J=* 8.0 Hz, 1H), 6.24 - 6.25 (m, 1H), 5.12 (d, *J=* 11.2 Hz, 1H), 3.91 - 3.84 (m, 2H), 3.61 (d, *J* = 10.4 Hz, 1H), 3.47 - 3.50 (m, 1H), 3.36 (dd, *J* = 12.4, 5.2 Hz, 1H), 3.09 - 3.17 (m, 2H), 2.21 - 2.31 (m, 1H), 1.04 - 0.98 (m, 2H), 0.85 - 0.78 (m, 1H). LCMS *m*/*z* = 410.2 [M+H]⁺. SFC (column: Chiralpak AD-3, 3 µm, 0.46 cm id × 10 cm L; mobile phase: A (CO₂) and B (EtOH, with 0.05% isopropylamine); gradient: B% = 10-40%, 5 min; flow rate: 4.0 mL/min; wavelength: 220 nm; pressure: 100 bar), retention time = 2.47 min, chiral isomer excess 100%.

**WX003B:** ¹H NMR (400 MHz, DMSO) δ: 8.88 - 8.78 (m, 2H), 8.07 - 8.05 (m, 3H), 6.90 (d, *J=* 8.0 Hz, 1H), 6.05 (t, *J* = 6.0 Hz, 1H), 4.97 *(d, J=* 10.8 Hz, 1H), 4.24 - 4.18 (m, 1H), 3.75 (d, *J* = 11.2 Hz, 1H), 3.46 - 3.38 (m, 2H), 3.16 - 3.20 (m, 1H), 2.67 (s, 1H), 1.99 - 1.97 (m, 1H), 0.98 - 0.94 (m, 2H), 0.86 - 0.78 (m, 1H). LCMS *m*/*z* = 410.2 [M+H]⁺. SFC (column: Chiralpak AD-3, 3 µm, 0.46 cm id × 10 cm L; mobile phase: A (CO₂) and B (EtOH, with 0.05% isopropylamine); gradient: B% = 10-40%, 5 min; flow rate: 4.0 mL/min; wavelength: 220 nm; pressure: 100 bar), retention time = 2.83 min, chiral isomer excess 96.5%.

### Example 4: Synthesis of Compounds WX004A and WX004B

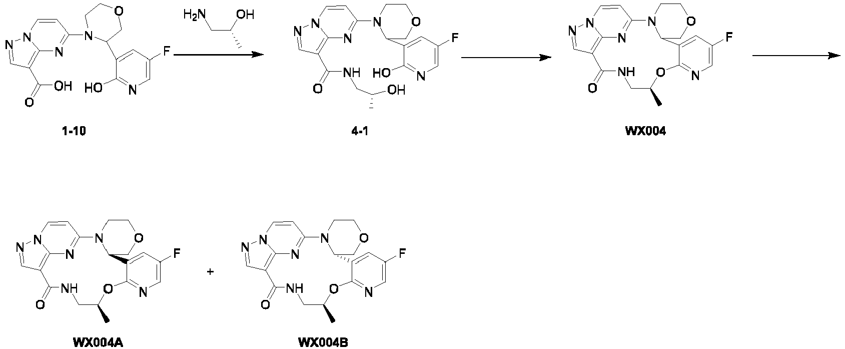

### Step 1: synthesis of compound 4-1

The compound **1-10** (500 mg, 1.39 mmol, 1 *eq)* was dissolved in *N*,*N*-dimethylformamide (4 mL), and the solution was added with *N*,*N*-diisopropylethylamine (359 mg, 2.78 mmol, 2 *eq)* and O-(7-azabenzotriazol-1-yl)-*N*,*N*,*N'*,*N'*-tetramethyluronium hexafluorophosphate (634 mg, 2.78 mmol, 1.2 *eq)* sequentially. The reaction mixture was stirred at 20 °C for 0.5 hour. The reaction mixture was then added with (*R*)-(-)-1-amino-2-propanol (136 mg, 1.81 mmol, 1.3 *eq)* and stirred at 20 °C for 4 hours. The reaction mixture was poured into water (10 mL), and extracted with dichloromethane (20 mL × 3). The combined organic phase was washed with saturated brine (5 mL), and dried over anhydrous sodium sulfate. The resulting reaction mixture was filtered to remove the desiccant, and the filtrate was concentrated under vacuum to give a crude product. The crude product was purified by silica gel column chromatography (dichloromethane:methanol = 100:1 to 10:1) to give compound 4-1. ¹H NMR (400MHz, DMSO-*d*₆) δ: 8.79 (br d, *J*=7.6 Hz, 1H), 8.16 (s, 1H), 8.00 (br s, 1H), 7.65 - 7.41 (m, 2H), 6.77 (br d, *J*=7.8 Hz, 1H), 5.41 - 5.34 (m, 1H), 4.90 - 4.72 (m, 1H), 4.50 - 4.30 (br s, 1H), 4.25 (br d, *J*=12.0 Hz, 1H), 4.09 - 3.97 (m, 1H), 3.87 (dd, *J*=4.0, 12.0 Hz, 1H), 3.81 - 3.71 (m, 1H), 3.68 - 3.54 (m, 2H), 3.18 - 3.05 (m, 1H), 1.10 - 1.00 (m, 3H). LCMS m/z: 417.2[M+1]⁺.

### Step 2: synthesis of compound WX004

Compound **4-1** (100 mg, 646 µmol, 1 *eq)* was dissolved in dichloromethane (15 mL), and the solution was added with triphenylphosphine (196 mg, 746 µmol, 3 *eq).* After being cooled to 0 °C, the reaction mixture was added with diethyl azodicarboxylate (129.85 mg, 745.57 µmol, 3 *eq),* and then warmed to 20 °C and reacted for 16 hours. The reaction mixture was added to water (10 mL), and the aqueous phase was extracted with dichloromethane (10 mL × 3). The combined organic phase was washed with saturated brine (5 mL), and dried over anhydrous sodium sulfate. The resulting reaction mixture was filtered to remove the desiccant, and the organic phase was concentrated under vacuum to give a crude product. The crude product was purified by silica gel column chromatography (dichloromethane:methanol = 100:1 to 4:1) to give compound **WX004.** LCMS *m*/*z*: 399.2[M+1]⁺.

### Step 3: synthesis of compounds WX004A and WX004B

Compound **WX004** (65 mg) was resolved by SFC (column: YMC CHIRAL Amylose-C (250mm×30mm, 10µm); mobile phase: A (CO₂) and B (isopropanol, with 0.1% ammonium hydroxide solution); gradient: B% = 40%-40%, 20 min) to give **WX004A** and **WX004B.**

**WX004A:** ¹H NMR (400MHz, DMSO-*d*₆) δ: 8.98 - 8.93 (m, 1H), 8.82 (d, *J*=7.8 Hz, 1H), 8.15 (s, 1H), 8.09 - 8.00 (m, 2H), 6.90 (d, *J*=8.0 Hz, 1H), 5.88 - 5.82 (m, 1H), 4.92 - 4.85 (m, 1H), 4.23 - 4.17 (m, 1H), 4.13 - 4.05 (m, 1H), 4.02 - 3.94 (m, 2H), 3.89 - 3.76 (m, 3H), 3.62 - 3.56 (m, 1H), 1.41 (d, *J*=6.4 Hz, 3H). LCMS *m*/*z*: 399.2 [M+1]⁺ . SFC (column: Chiralpak AD-3, 3 µm, 0.46 cm id × 10 cm L; mobile phase: A (CO₂) and B (EtOH, with 0.05% isopropylamine); gradient: B% = 5-40% (4.5 min), 40% (2.5 min), 5% (1 min); flow rate: 2.8 mL/min; wavelength: 220 nm; pressure: 100 bar), retention time = 4.595 min, chiral isomer excess 98.954%.

**WX004B:** ¹H NMR (400MHz, DMSO-*d*₆) δ: 9.38 - 9.33 (m, 1H), 8.81 (dd, *J*=1.6, 8.0 Hz, 1H), 8.14 (s, 1H), 8.05 (d, *J*=2.4 Hz, 1H), 7.91 (dd, *J*= 2.6, 8.0 Hz, 1H), 6.83 (d, *J*=7.8 Hz, 1H), 5.85 - 5.75 (m, 1H), 5.08 - 4.99 (m, 1H), 4.30 - 4.24 (m, 1H), 4.10 - 3.95 (m, 4H), 3.89 - 3.74 (m, 2H), 3.18 - 3.10 (m, 1H), 1.45 (d, *J*=6.4 Hz, 3H). LCMS m/z: 399.2 [M+1]⁺. SFC (column: Chiralpak AD-3, 3 µm, 0.46 cm id × 10 cm L; mobile phase: A (CO₂) and B (EtOH, with 0.05% isopropylamine); gradient: B% = 5-40% (4.5 min), 40% (2.5 min), 5% (1 min); flow rate: 2.8 mL/min; wavelength: 220 nm; pressure: 100 bar), retention time = 5.667 min, chiral isomer excess 100%.

Referring to the synthetic procedure of **Example 4, Example 5** was synthesized by substituting (*R*)-(-)-1-amino-2-propanol in step 1 with the fragment in the following table.

| **Example** | **Compound** | **Fragment** | **Structural formula** | **Spectrogram** |
|---|---|---|---|---|
| **5** | **WX005A** | 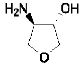 | 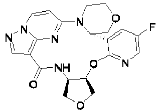 | ¹HNMR (400 MHz, DMSO-*d*₆) δ: 9.12 (d, *J*=8 Hz, 1 H), 8.82 (d, *J* =8 Hz, 1 H), 8.12 (s, 1 H), 8.02 (d, *J* =4 Hz, 1 H), 7.86 (d, *J* =8,4 Hz, 1 H), 6.87 (d, *J*=8 Hz, 1 H), 6.04 (d, *J*=6.16, 2.93 Hz, 1 H), 5.66 (s, 1 H), 4.56 - 4.49 (m, 1 H), 4.29 - 4.15 (m, 1 H), 4.13 - 3.77 (m, 8 H), 3.69 (t, *J*=8 Hz, 1 H). LCMS *m*/*z*: 427.2 [M+1]⁺. |
| | | | | SFC (column: Chiralpak IC-3, 3 µm, 0.46 cm id × 10 cm L; mobile phase: A (CO₂) and B (MeOH, with 0.05% isopropylamine); gradient: B% = 10-40%, 5 min; flow rate: 4 mL/min; wavelength: 220 nm; pressure: 100 bar), Rt =3.38 min, chiral isomer excess 100%. |
| | **WX005B** | | 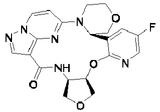 | ¹H NMR (400 MHz, DMSO-*d*₆) δ: 8.65 (d, *J*=8 Hz, 1 H), 8.06 (s, 1 H), 7.47 (s, 1 H), 7.30 (d, *J* =8 Hz, 1 H), 6.61 (d, *J* =8 Hz, 1 H), 5.24 (s, 1 H), 5.03 (d, *J* =8, 4 Hz, 1 H), 4.75 - 4.52 (m, 1 H), 4.35 (s, 1 H), 4.22 (d, *J* =12 Hz, 1 H), 4.06 - 3.83 (m, 2 H), 3.75 (d, *J* =12 Hz, 2 H), 3.61 - 3.34 (m, 4 H). LCMS *m*/*z*: 427.2 [M+1]⁺. |
| | | | | SFC (column: Chiralpak OD-3, 3 µm, 0.46 cm id × 10 cm L; mobile phase: A (CO₂) and B (MeOH, with 0.05% isopropylamine); gradient: B% = 10-40%, 5 min; flow rate: 4 mL/min; wavelength: 220 nm; pressure: 100 bar), Rt = 2.99 min, chiral isomer excess 100 %. |

### Example 6: Synthesis of Compound WX006

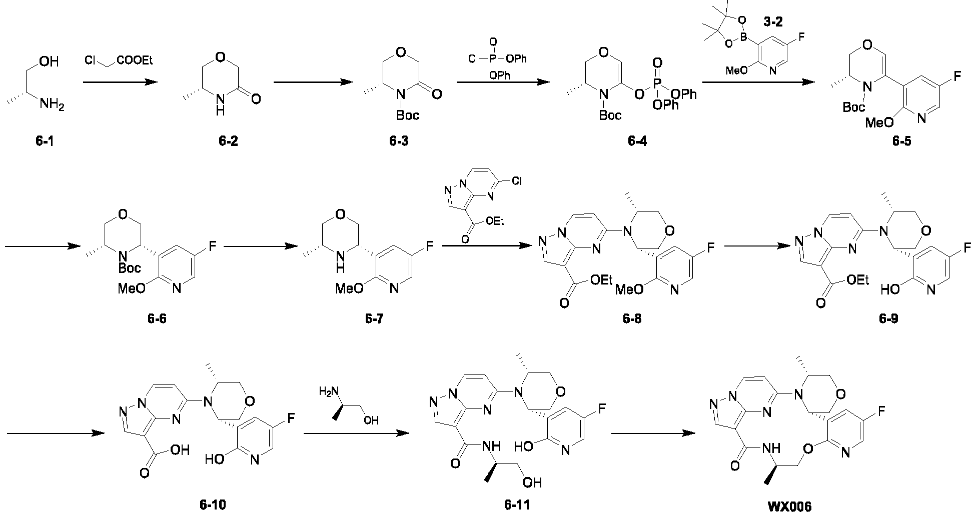

### Step 1: synthesis of compound 6-2

Sodium hydride (24 g, 2.25 eq) was added to tetrahydrofuran (1320 mL) and stirred well, and the reaction mixture was added with compound **6-1** (20 g, 266.28 mmol, 1 *eq)* at 0 °C, and stirred for 10 minutes. The reaction mixture was heated to 20 °C, added with ethyl chloroacetate (40.79 g, 332.85 mmol, 1.25 *eq)* dropwise, and stirred at 20 °C for 2 hours. The reaction mixture was slowly added with a saturated ammonium chloride solution (100 mL) to quench the reaction. The mixture was left to stand for liquid separation, and the aqueous phase was extracted with ethyl acetate (100 mL × 4). The combined organic phase was dried over anhydrous sodium sulfate. The resulting reaction mixture was filtered to remove the desiccant, and the organic phase was concentrated under vacuum to give a crude product. The crude product was purified by silica gel column chromatography (petroleum ether:ethyl acetate = 100:0 to 1:1 to 0:100) to give compound **6-2.** ¹H NMR (400MHz, CDCl₃) δ: 6.60 (s, 1H), 4.22 - 4.07 (m, 2H), 3.94 - 3.89 (m, 1H), 3.77 - 3.69 (m, 1H), 3.40 - 3.35 (m, 1H), 1.20 (d, *J*=6.4 Hz, 3H).

### Step 2: synthesis of compound 6-3

Compound **6-2** (11.6 g, 100.76 mmol, 1 *eq)* was dissolved in tetrahydrofuran (116 mL), and the solution was added with dimethylaminopyridine (1.23 g, 10.08 mmol, 0.1 *eq)* and Boc-anhydride (30.79 g, 141.06 mmol, 1.4 *eq).* The reaction mixture was stirred at 20 °C for 3 hours. The reaction mixture was added with water (100 mL), and extracted with ethyl acetate (100 mL × 2). The combined organic phase was washed with saturated brine (50 mL), and dried over anhydrous sodium sulfate. The resulting reaction mixture was filtered to remove the desiccant, and the organic phase was concentrated under vacuum to give oily compound **6-3.** The crude product was used in the next step directly without further purification.

### Step 3: synthesis of compound 6-4

Compound **6-3** (20 g, 92.92 mmol, 1 *eq)* was dissolved in tetrahydrofuran (200 mL), and the solution was added with lithium hexamethyldisilazide (1 M, 92.92 mL, 1 *eq)* dropwise at -60 °C, and stirred for 1 hour. A solution of diphenyl chlorophosphate (24.96 g, 92.92 mmol, 19.20 mL, 1 *eq)* in tetrahydrofuran (25 mL) was added dropwise at -30 °C, and the reaction mixture was stirred at -10 °C for 2 hours. The reaction mixture was added with a saturated aqueous ammonium chloride solution (50 mL) and water (100 mL) to quench the reaction, and the aqueous phase was extracted with ethyl acetate (200 mL × 2). The combined organic phase was dried over anhydrous sodium sulfate. The resulting reaction mixture was filtered to remove the desiccant, and the organic phase was concentrated under vacuum to give a crude product. The crude product was purified by silica gel column chromatography (petroleum ether:ethyl acetate = 100:0 to 10:1) to give compound **6-4.** ¹H NMR (400MHz, CDCl₃) δ: 7.37 - 7.33 (m, 4H), 7.25 - 7.18 (m, 6H), 6.34 (d, *J=* 4.0 Hz, 1H), 4.57 - 4.52 (m, 1H), 3.97 - 3.94 (m, 1H), 3.71 (dd, *J* = 11.2, 2.8 Hz, 1H), 1.46 (s, 9H), 1.22 (d, *J* = 6.8 Hz, 3H). LCMS *m*/*z*: 448.2 [M+1]⁺.

### Step 4: synthesis of compound 6-5

Compound **6-4** (21 g, 46.94 mmol, 1 *eq)* was dissolved in acetonitrile (260 mL) and water (65 mL), and the solution was added with potassium phosphate (19.93 g, 93.87 mmol, 2 *eq)* and compound **3-2** (23.76 g, 93.87 mmol, 2 *eq).* Then bis(tri-*tert*-butylphosphine)palladium(0) (520.51 mg, 1.02 mmol, 2.2% mol) was added under nitrogen atmosphere, and the resulting reaction mixture was heated to 65 °C and stirred for 17 hours. After being cooled to room temperature, the reaction mixture was added with water (100 mL) to quench the reaction. The aqueous phase was extracted with ethyl acetate (200 mL × 2). The combined organic phase was dried over anhydrous sodium sulfate. The resulting reaction mixture was filtered to remove the desiccant, and the organic phase was concentrated under vacuum to give a crude product. The crude product was purified by silica gel column chromatography (petroleum ether:ethyl acetate = 100:0 to 10:1) to give compound **6-5.** LCMS *m*/*z*: 325.1 [M+1]⁺.

### Step 5: synthesis of compound 6-6

Compound **6-5** (7.15 g, 22.04 mmol, 1 *eq)* was dissolved in ethanol (70 mL), and the solution was added with palladium hydroxide/carbon (28.60 g, 20% purity). The reaction mixture was stirred at 80 °C under hydrogen atmosphere (50 psi) for 24 hours. The reaction mixture was filtered to remove the desiccant, and the filtrate was concentrated under vacuum to give a crude product. The crude product was purified by silica gel column chromatography (petroleum ether:ethyl acetate = 100:0 to 5:1) to give compound **6-6.** LCMS *m*/*z*: 327.1 [M+1]⁺.

### Step 6: synthesis of compound 6-7

Compound **6-6** (4 g, 12.26 mmol, 1 *eq)* was dissolved in ethyl acetate (8 mL), and the solution was added with a solution of hydrogen chloride in ethyl acetate (4 M, 24.5 mL, 8 *eq).* The reaction mixture was stirred at 20 °C for 3 hours. The reaction mixture was filtered. The filter cake was rinsed with ethyl acetate (10 mL × 2), and dried under vacuum to give compound 6-7. ¹H NMR (400MHz, MeOH-*d*₄) δ: 8.15 (d, *J=* 3.2 Hz, 1H), 7.81 - 7.78 (m, 1H), 4.77 - 4.74 (m, 1H), 4.16 - 4.08 (m, 2H), 4.02 (s, 3H), 3.86 - 3.81 (m, 1H), 3.74 - 3.65 (m, 1H), 3.63 - 3.57 (m, 1H), 1.35 (d, *J*=6.8 Hz, 3H). LCMS *m*/*z*: 227.0 [M+1]⁺. The crude product was used in the next step directly without further purification.

### Step 7: synthesis of compound 6-8

Compound **6-7** (1.47 g, 6.50 mmol, 1 *eq)* was dissolved in *N*,*N*-dimethylformamide (15 mL), and the solution was added with sodium hydride (286 mg, 7.15 mmol, 60% purity, 1.1 eq) at 15 °C. The reaction mixture was stirred well. At 15 °C, the reaction mixture was added with ethyl 5-chloropyrazolo[1,5-*a*]pyrimidine-3-carboxylate (1.47 g, 6.50 mmol, 1 *eq),* and stirred for 4 hours. The reaction mixture was supplemented with sodium hydride (78 mg, 1.95 mmol, 60% purity, 0.3 *eq),* and then sirred for 2 hours. The reaction mixture was slowly added with a saturated aqueous ammonium chloride solution (3 mL) and water (20 mL) to quench the reaction, and the aqueous phase was extracted with dichloromethane (20 mL × 2). The combined organic phase was dried over anhydrous sodium sulfate. The resulting reaction mixture was filtered to remove the desiccant, and the organic phase was concentrated under vacuum to give a crude product. The crude product was purified by silica gel column chromatography (dichloromethane:methanol = 100:0 to 20:1) to give compound **6-8.** LCMS *m*/*z*: 416.2 [M+1]⁺.

### Step 8: synthesis of compound 6-9

Compound **6-8** (800 mg, 1.93 mmol, 1 *eq)* was dissolved in acetonitrile (8 mL), and the solution was added with sodium iodide (866 mg, 5.78 mmol, 3 *eq)* and trimethylchlorosilane (628 mg, 5.78 mmol, 3 *eq).* The reaction mixture was stirred under nitrogen atmosphere at 15 °C for 18 hours. The reaction mixture was added with water (10 mL) to quench the reaction, and the aqueous phase was extracted with dichloromethane (10 mL × 2). The combined organic phase was dried over anhydrous sodium sulfate. The resulting reaction mixture was filtered to remove the desiccant, and the organic phase was concentrated under vacuum to give a crude product. The crude product was purified by silica gel column chromatography (dichloromethane:methanol = 100:0 to 10:1) to give compound **6-9.** ¹H NMR (400MHz, CDCl₃) δ: 8.24 (s, 2H), 7.45 (s, 1H), 7.23 (s, 1H), 6.18 (d, *J=* 7.6 Hz, 1H), 5.18 (s, 1H), 4.77 (s, 1H), 4.45 (d, *J=* 11.6 Hz, 1H), 4.25 (t, *J=* 7.2 Hz , 2H), 3.92 - 3.77 (m, 3H), 1.51 (d, *J* = 6.4 Hz, 3H), 1.30 (m, 3H). LCMS *m*/*z:* 402.2 [M+1]⁺.

### Step 9: synthesis of compound 6-10

Compound **6-9** (330 mg, 822.14 µmol, 1 *eq)* was dissolved in methanol (4 mL), and the solution was added with an aqueous sodium hydroxide solution (3 M, 1.10 mL, 4 *eq)* and water (1.1 mL) sequentially. The reaction mixture was stirred under nitrogen atmosphere at 60 °C for 17 hours. After being cooled to room temperature, the reaction mixture was neutralized with a 3 mol/L diluted hydrochloric acid to pH 2. A solid was precipitated, and the reaction mixture was stirred at room temperature for 30 minutes and filtered. The filter cake was dried at 50 °C to give crude compound **6-10.** LCMS *m*/*z*: 374.2 [M+1]⁺. The crude product was used in the next step directly without further purification.

### Step 10: synthesis of compound 6-11

Compound **6-10** (65 mg, 174 µmol, 1 *eq)* was dissolved in *N*,*N*-dimethylformamide (3 mL), and the solution was added with 2-(7-azobenzotriazol)-*N*,*N*,*N'*,*N'*-tetramethyluronium hexafluorophosphate (72.82 mg, 192 µmol, 1.1 *eq)* and *R*-2-amine-1-propanol (14 mg, 191 µmol, 1.1 *eq)* sequentially, and then added with *N,N-*diisopropylethylamine (79 mg, 3.5 *eq)* dropwise. The reaction mixture was stirred at 15 °C for 16 hours. The reaction mixture was added with a saturated aqueous ammonium chloride solution (0.5 mL) to quench the reaction, and concentrated under vacuum to give a crude product. The crude product was purified by preparative silica gel thin layer chromatography (dichloromethane:methanol = 7:1) to give compound **6-11.** LCMS *m*/*z*: 431.2 [M+1]⁺.

### Step 11: synthesis of compound WX006

Compound **6-11** (40 mg, 92.93 µmol, 1 *eq)* was dissolved in dichloromethane (4 mL), and the solution was added with triphenylphosphine (73 mg, 279 µmol, 3 *eq).* The reaction mixture was added with diisopropyl azodicarboxylate (56 mg, 279 µmol, 3 *eq)* at 0 °C, heated to 15 °C, and stirred for 0.5 hour. The reaction mixture was concentrated to 1 mL to give a crude solution, which was purified by silica gel preparative thin layer chromatography (dichloromethane:methanol = 7:1) to give compound **WX006.** ¹H NMR (400MHz, CDCl₃) δ: 9.22 (d, *J=* 8.0 Hz, 1H), 8.37 (d, *J=* 7.6 Hz, 1H), 8.32 (s, 1H), 7.90 (d, *J=* 2.8 Hz, 1H), 7.73 - 7.70 (m, 1H), 6.36 (d, *J=* 7.6 Hz, 1H), 6.01 - 6.00 (m, 1H), 4.97 (dd, *J=* 10.4, 4.4 Hz, 1H), 4.51 - 4.50 (m, 1H), 4.14 - 3.98 (m, 6H), 1.76 (d, *J* = 6.8 Hz, 3H), 1.49 (d, *J=* 6.8 Hz, 3H). LCMS *m*/*z*: 413.2 [M+1]⁺.

Referring to the synthetic procedure of **Example 6, Example 7** and **Example 8** were synthesized by substituting *R*-2-amine-1-propanol in step 10 with the fragments in the following table, respectively.

| **Example** | **Compound** | **Fragment** | **Structural formula** | **Spectrogram** |
|---|---|---|---|---|
| **7** | **WX007** | 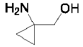 | 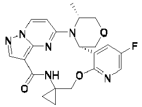 | ¹H NMR (400MHz, CDCl₃) δ: 8.87 (s, 1H), 8.35 (d, *J* = 4.0 Hz, 1H), 8.25 (s, 1H), 7.89 (d, *J* = 3.2 Hz, 1H), 7.76-7.73 (m, 1H), 6.35 (d, *J* = 8.0 Hz, 1H), 6.13 - 6.12 (m, 1H), 5.13 - 5.10 (m, 1H), 4.11 - 3.99 (m, 5H), 3.68 (d, *J* = 10.4 Hz, 1H), 2.20 - 2.15 (m, 1H), 1.74 (d, *J* = 6.8 Hz, 3H), 1.12 - 1.00 (m, 2H), 0.80 - 0.75 (m, 1H). LCMS *m*/*z*: 425.2 [M+1]⁺. |
| **8** | **WX008** | 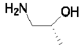 | 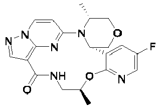 | ¹H NMR (400MHz, CDCl₃) δ: 9.23 (d, *J* = 6.0 Hz, 1H), 8.38 (d, *J* = 8.0 Hz, 1H), 8.32 (s, 1H), 7.89 (d, *J* = 2.8 Hz, 1H), 7.66 - 7.63 (m, 1H), 6.37 (d, *J* = 7.6 Hz, 1H), 5.95 (s, 1H), 5.34 - 5.29 (m, 1H), 4.19 - 4.13 (m, 1H), 4.08 - 3.96 (m, 5H), 3.29 - 3.23 (m, 1H), 1.77 (d, *J* = 6.8 Hz, 3H), 1.52 (d, *J* = 6.0 Hz, 3H). LCMS *m*/*z:* 413.2 [M+1]⁺. |

### Example 9: Synthesis of Compounds WX009A and WX009B

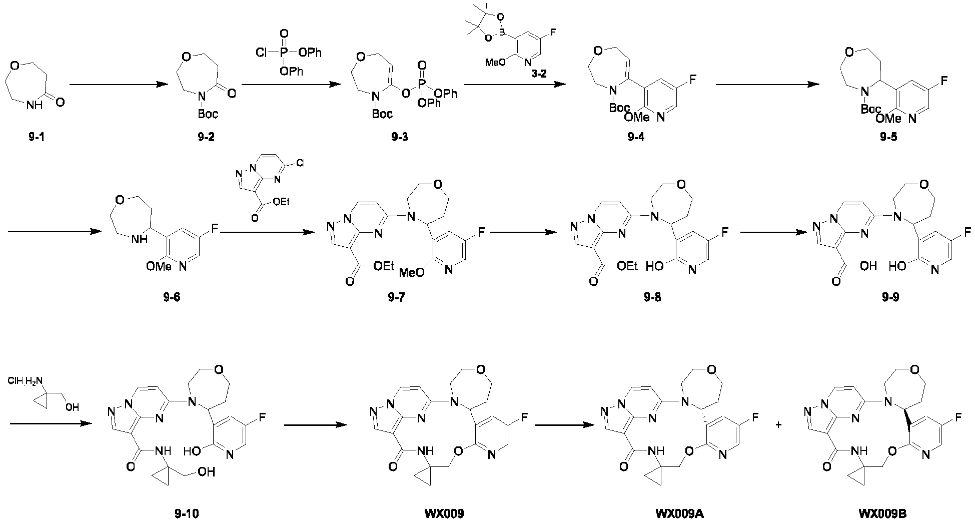

### Step 1: synthesis of compound 9-2

Compound **9-1** (10.0 g, 86.86 mmol, 1 *eq)* was dissolved in tetrahydrofuran (100 mL), and the solution was added with 4-dimethylaminopyridine (1.06 g, 8.69 mmol, 0.1 *eq)* and Boc-anhydride (24.64 g, 112.92 mmol, 1.3 *eq).* The reaction mixture was stirred at 15 °C for 16 hours. The reaction mixture was poured into a saturated sodium chloride solution (50 mL), and the aqueous phase was extracted with ethyl acetate (100 mL × 2). The organic phases were combined, and added with 1% aqueous hydrochloric acid solution to adjust the pH to 3 in an ice water bath, followed by liquid separation. The organic phase was washed with saturated sodium bicarbonate solution (50 mL) and saturated brine (50 mL) sequentially, dried over anhydrous sodium sulfate, filtered, and concentrated under vacuum to give compound **9-2.** ¹H NMR (400MHz, CDCl₃) δ: 3.98 - 3.93 (m, 2H), 3.88 - 3.78 (m, 4H), 2.90 - 2.83 (m, 2H), 1.53 (s, 9H). The crude product was used in the next step directly without further purification.

### Step 2: synthesis of compound 9-3

Under nitrogen atmosphere, compound **9-2** (4 g, 18.58 mmol, 1 *eq)* was dissolved in tetrahydrofuran (50 mL), and the reaction mixture was then cooled to -55 °C, and slowly added with lithium hexamethyldisilazide (18.58 mL, 1 M, 1 *eq)* dropwise. The reaction mixture was stirred at -60 °C to -30 °C for 45 minutes. The reaction mixture was added with diphenyl chlorophosphate (4.99 g, 18.58 mmol, 3.84 mL, 1 *eq)* dropwise at - 30 °C, and then cooled to -60 °C and stirred for 75 minutes. The reaction mixture was poured into water (30 mL), and extracted with ethyl acetate (30 mL × 3). The combined organic phase was washed with saturated brine (20 mL), dried over anhydrous sodium sulfate, and concentrated under vacuum to give a crude product. The crude product was purified by silica gel column chromatography (petroleum ether:ethyl acetate = 15:1 to 1:1) to give compound **9-3.** ¹H NMR (400MHz, CDCl₃) δ: 7.43 - 7.29 (m, 4H), 7.24 (br d, *J*=8.5 Hz, 6H), 5.51 (dt, *J*=2.8, 6.1 Hz, 1H), 4.10 - 3.98 (m, 2H), 3.78 - 3.70 (m, 2H), 3.69 - 3.56 (m, 2H), 1.43 (s, 9H). LCMSm/z: 348.1 [M-100+1]⁺.

### Step 3: synthesis of compound 9-4

Compounds **9-3** (1 g, 2.24 mmol, 1 *eq)* and **3-2** (1.13 g, 4.47 mmol, 2 *eq)* were dissolved in acetonitrile (10 mL), and the solution was added with potassium phosphate (949 mg, 4.47 mmol, 2 *eq)* and water (2.5 mL) sequentially. The reaction mixture was purged with nitrogen, added with bis(tri-tert-butylphosphine)palladium(0) (228 mg, 447 µmol, 0.2 *eq)* under nitrogen atmosphere, heated to 75 °C and then stirred for 10 hours. After being cooed to room temperature, the reaction mixture was poured into water (50 mL), and extracted with ethyl acetate (50 mL × 3). The combined organic phase was washed with saturated brine (30 mL), dried over anhydrous sodium sulfate, filtered, and concentrated under vacuum to give a crude product. The crude product was purified by silica gel column chromatography (petroleum ether:ethyl acetate = 10: 1 to 0: 1) to give a crude product. The crude product was purified by preparative HPLC (column: YMC Triart C18 (250mm×50mm, 7µm), mobile phase: [water (0.04% ammonium hydroxide solution + 10 mM ammonium bicarbonate)-acetonitrile]; acetonitrile B%: 40%-65%, 18 min) to give compound **9-4.** ¹H NMR (400MHz, DMSO-*d*₆) δ: 8.13 - 8.08 (m, 1H), 7.61 - 7.54 (m, 1H), 5.79 - 5.73 (m, 1H), 4.14 (d, *J*=5.6 Hz, 2H), 3.82 (s, 3H), 3.80 - 3.69 (m, 4H), 1.02 (s, 9H). LCMS m/z: 325.1 [M+1]⁺.

### Step 4: synthesis of compound 9-5

Compound **9-4** (2.7 g, 8.32 mmol, 1 *eq)* was dissolved in ethanol (50 mL), and the solution was added with palladium hydroxide/carbon (2 g, 20% purity). The reaction mixture was stirred at 80 °C under hydrogen atmosphere (50 psi) for 3 hours. The reaction mixture was filtered, and the filtrate was concentrated under vacuum to give compound **9-5.** ¹H NMR (400MHz, CDCl₃) δ: 7.89 - 7.84 (m, 1H), 7.20 - 7.07 (m, 1H), 5.27 - 5.22 (m, 0.5H), 5.11 - 5.04 (m, 0.5H), 4.39 - 4.32 (m, 0.5H), 4.19 - 3.94 (m, 2.5H), 3.93 (s, 3H), 3.60 - 3.34 (m, 3H), 2.69 - 2.57 (m, 0.5H), 2.42 - 2.32 (m, 0.5H), 2.02 - 1.83 (m, 1H), 1.46 (s, 4H), 1.25 (s, 5H). LCMS m/z: 326.9 [M+1]⁺.

### Step 5: synthesis of compound 9-6

Compound **9-5** (2.4 g, 7.35 mmol, 1 *eq*) was dissolved in ethyl acetate (10 mL), and the solution was added with hydrogen chloride/ethyl acetate (4 M, 14.7 mL, 8 *eq*). The reaction mixture was stirred at 18 °C for 16 hours. The reaction mixture was directly concentrated to give a hydrochloride of compound **9-6.** LCMS m/z:226.9 [M+1]⁺.

### Step 6: synthesis of compound 9-7

Compound **9-6** (2.3 g, 7.69 mmol, 1 *eq,* hydrochloride) was dissolved in *N*-methylpyrrolidone (18 mL), and the solution was added with *N,N*-diisopropylethylamine (2.48 g, 19.22 mmol, 2.5 *eq*) and ethyl 5-chloropyrazolo[1,5-*a*]pyrimidine-3-carboxylate (1.56 g, 6.92 mmol, 0.9 *eq).* The reaction mixture was stirred at 75 °C for 16 hours. After being cooled to room temperature, the reaction mixture was added with water (20 mL), then ethyl acetate (20 mL) was added, and the resulting reaction mixture was stirred for 5 minutes, followed by liquid separation. The aqueous phase was extracted with ethyl acetate (20 mL × 3). The combined organic phase was washed with saturated brine (20 mL), dried over anhydrous sodium sulfate, and filtered, and the filtrate was concentrated under vacuum to give a crude product. The crude product was purified by silica gel column chromatography (petroleum ether:ethyl acetate = 10:1 to 0:1) to give compound **9-7.** LCMS m/z: 416.0 [M+1]⁺.

### Step 7: synthesis of compound 9-8

Compound **9-7** (3.18 g, 7.65 mmol, 1 *eq*) was dissolved in acetonitrile (30 mL), and the solution was added with sodium iodide (3.44 g, 22.96 mmol, 3 *eq*) and trimethylchlorosilane (2.49 g, 22.96 mmol, 3 *eq*) sequentially. The reaction mixture was heated to 65 °C, and stirred for 16 hours. After being cooled to room temperature, the reaction mixture was added to water (40 mL), and extracted with dichloromethane (50 mL × 3). The combined organic phase was washed with brine (50 mL), dried over anhydrous sodium sulfate, and filtered, and the filtrate was concentrated under vacuum to give compound **9-8.** LCMS m/z:401.9 [M+1]⁺.

### Step 8: synthesis of compound 9-9

Compound **9-8** (4.31 g, 10.74 mmol, 1 *eq*) was dissolved in methanol (40 mL), and the solution was added with an aqueous sodium hydroxide solution (3 M, 14.32 mL, 4 *eq*) and water (11 mL) sequentially. The reaction mixture was heated to 60 °C and stirred for 16 hours. After being cooled to room temperature, the reaction mixture was added with 1 N hydrochloric acid dropwise to adjust the pH to 2-3 in an ice water bath, and extracted with dichloromethane (50 mL × 3). The combined organic phase was washed with brine (20 mL), dried over anhydrous sodium sulfate, and filtered, and the filtrate was concentrated under vacuum to give compound **9-9.** LCMS m/z: 374.0 [M+1]⁺. The crude product was used in the next step directly without further purification.

### Step 9: synthesis of compound 9-10

Compound **9-9** (1.6 g, 4.29 mmol, 1 *eq*) and 1-(hydroxymethyl)cyclopropylamino hydrochloride (583 mg, 4.71 mmol, 1.1 *eq*) were dissolved in *N,N*-dimethylformamide (12 mL), and the solution was added with 2-(7-azobenzotriazol)-*N,N,N',N'*-tetramethyluronium hexafluorophosphate (1.79 g, 4.71 mmol, 1.1 *eq*) and *N,N-*diisopropylethylamine (1.94 g, 15.00 mmol, 3.5 *eq,* hydrochloride) sequentially. The reaction mixture was stirred at 15 °C for 2 hours. The reaction mixture was poured into water (20 mL), and extracted with ethyl acetate (30 mL × 3). The combined organic phase was washed with saturated brine (20 mL), dried over anhydrous sodium sulfate, filtered, and concentrated under vacuum to give a crude product. The crude product was purified by silica gel column chromatography (dichloromethane:methanol = 1:0 to 10:1) to give compound **9-10.** LCMS m/z: 443.0 [M+1]⁺.

### Step 10: synthesis of compound WX009

Compound **9-10** (0.43 g, 0.97 mmol, 1 *eq*) was dissolved in dichloromethane (6 mL), and the solution was added with triphenylphosphine (765 mg, 2.92 mmol, 3 *eq*), and then added with diethyl azodicarboxylate (508 mg, 2.92 mmol, 3 *eq*) at 0 °C. The reaction mixture was heated to 15 °C and stirred for 16 hours. The reaction mixture was directly purified by silica gel column chromatography (dichloromethane:methanol = 1:1 to 10:1) to give compound **WX009.** LCMS m/z: 425.1[M+1]⁺.

### Step 11: synthesis of compounds WX009A and WX009B

Compound **WX009** (0.33 g, 0.78 mmol) was resolved by SFC (column: DAICEL CHIRALCEL OJ-H (250mm×30mm, 5µm), mobile phase: A (CO₂) and B (ethanol, with 0.1% ammonium hydroxide solution); gradient: B% = 35%-35%, 20 min) to give **WX009A** and **WX009B.**

**WX009A:** ¹H NMR (400MHz, DMSO-*d*₆) δ: 8.97 - 8.86 (m, 1H), 8.81 - 8.69 (m, 1H), 8.10 - 7.96 (m, 2H), 7.91 - 7.79 (m, 1H), 6.92 - 6.85 (m, 1H), 5.95 - 5.82 (m, 1H), 4.93 - 4.83 (m, 1H), 4.40 - 4.29 (m, 2H), 4.12 - 3.97 (m, 2H), 3.89 - 3.75 (m, 1H), 3.52 - 3.25 (m, 2H), 2.40 - 2.25 (m, 1H), 2.24 - 2.16 (m, 1H), 2.00 - 1.91 (m, 1H), 1.14 - 1.05 (m, 1H), 0.97 - 0.89 (m, 1H), 0.84 - 0.75 (m, 1H). LCMS m/z: 425.1[M+1]⁺. SFC (column: Chiralcel AD-3, 3 µm, 0.46 cm id × 15 cm L; mobile phase: A (CO₂) and B (EtOH, with 0.05% isopropylamine); gradient: B% = 5-40%, 5 min; gradient: B% = 40-5%, 0.5 min; gradient: B% = 5%, 1.5 min; flow rate: 2.5 mL/min; wavelength: 220 nm; pressure: 100 bar), retention time = 4.880 min, chiral isomer excess 100.00%.

**WX009B:** ¹H NMR (400MHz, DMSO-*d*₆) δ: 8.96 - 8.85 (m, 1H), 8.80 - 8.68 (m, 1H), 8.09 - 7.95 (m, 2H), 7.91 - 7.79 (m, 1H), 6.92 - 6.85 (m, 1H), 5.95 - 5.82 (m, 1H), 4.93 - 4.83 (m, 1H), 4.40 - 4.29 (m, 2H), 4.12 - 3.97 (m, 2H), 3.87 - 3.73 (m, 1H), 3.50 - 3.25 (m, 2H), 2.47 - 2.27 (m, 1H), 2.26 - 2.10 (m, 1H), 1.97 - 1.89 (m, 1H), 1.11 - 1.02 (m, 1H), 0.99 - 0.86 (m, 1H), 0.81 - 0.72 (m, 1H). LCMS m/z: 425.1[M+1]⁺. SFC (column: Chiralcel AD-3, 3 µm, 0.46 cm id × 15 cm L; mobile phase: A (CO₂) and B (EtOH, with 0.05% isopropylamine); gradient: B% = 5-40%, 5 min; gradient: B% = 40-5%, 0.5 min; gradient: B% = 5%, 1.5 min; flow rate: 2.5 mL/min; wavelength: 220 nm; pressure: 100 bar), retention time = 5.335 min, chiral isomer excess 99.12%.

### Example 10: Synthesis of Compounds WX010A and WX010B

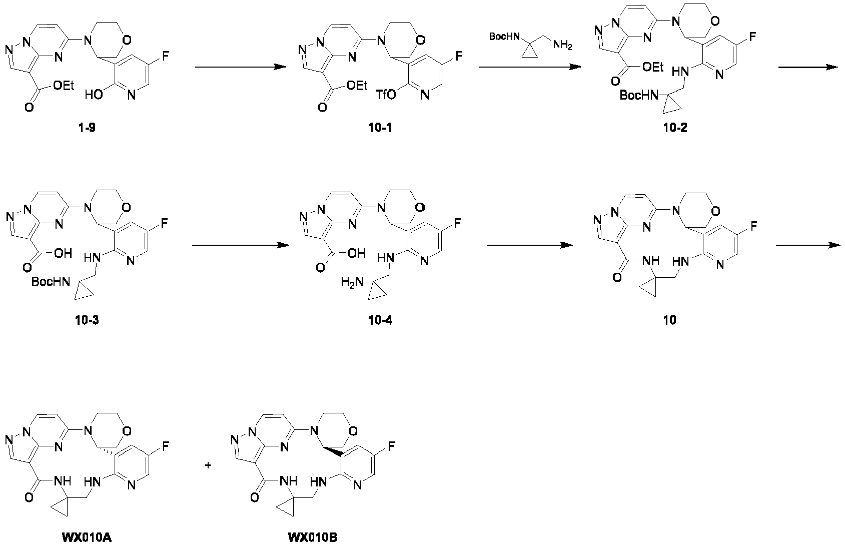

### Step 1: synthesis of compound 10-1

Under nitrogen atmosphere, compound **1-9** was dissolved in dichloromethane (30 mL), and the solution was added dropwise with *N,N*-diisopropylethylamine (1.40 g, 10.84 mmol, 1.4 *eq*) and trifluoromethanesulfonic anhydride (2.62 g, 9.29 mmol, 1.2 *eq*) sequentially at 0 °C. The reaction mixture was slowly warmed to room temperature, and stirred at 18 °C for 6 hours. The reaction mixture was poured into water (50 mL), and extracted with dichloromethane (50 mL × 3). The combined organic phase was washed with saturated brine (10 mL), and dried over anhydrous sodium sulfate. The resulting reaction mixture was filtered to remove the desiccant, and the organic phase was concentrated under vacuum to give a crude product. The crude product was purified by silica gel column chromatography (petroleum ether:ethyl acetate = 10:1 to 0:1) to give compound **10-1.** ¹H NMR (400MHz, DMSO-*d*₆) δ: 8.87 (d, *J*=7.6 Hz, 1H), 8.45 (s, 1H), 8.25 (s, 1H), 8.20 - 8.14 (m, 1H), 6.95 (d, *J*=8.0 Hz, 1H), 5.95 - 5.86 (m, 1H), 4.33 - 4.23 (m, 2H), 4.17 (q, *J*=7.0 Hz, 1H), 4.06 - 3.97 (m, 1H), 3.72 - 3.63 (m, 1H), 3.57 - 3.47 (m, 1H), 1.99 (s, 2H), 1.22 (t, *J*=7.0 Hz, 3H). LCMS m/z: 520.1[M+1]⁺.

### Step 2: synthesis of compound 10-2

Compound **10-1** (800 mg, 1.54 mmol, 1 *eq*), 1-Boc-amino-1-aminomethylcyclopropane (500 mg, 2.68 mmol, 1.74 *eq*) and *N,N*-diisopropylethylamine (399 mg, 3.08 mmol, 2 *eq*) were dissolved in acetonitrile (5 mL), and the reaction mixture was heated to reflux for 110 hours. After being cooled to room temperature, the reaction mixture was filtered, and the filtrate was added with water (10 mL), and extracted with ethyl acetate (20 mL × 3). The combined organic phase was washed with saturated brine (10 mL), dried over anhydrous sodium sulfate, and concentrated under vacuum to give a crude product. The crude product was purified by silica gel column chromatography (petroleum ether:ethyl acetate = 10:1 to 0:1) to give compound **10-2.** LCMS *m*/*z:* 556.2[M+1]⁺.

### Step 3: synthesis of compound 10-3

Compound **10-2** (200 mg, 360 µmol, 1 *eq*) was dissolved in methanol (4 mL), and the solution was added with an aqueous sodium hydroxide solution (3 M, 0.48 mL, 4 *eq*) and water (1.1 mL) sequentially. The reaction mixture was stirred at 15 °C for 24 hours. The reaction mixture was concentrated under vacuum at 15 °C to remove methanol, and then added with 1 M hydrochloric acid to adjust the pH to 3-4. A solid was precipitated. The mixture was filtered. The filter cake was dried under vacuum to give compound **10-3.** LCMS *m*/*z*: 528.1[M+1]⁺.

### Step 4: synthesis of compound 10-4

Compound **10-3** (130 mg, 246 µmol, 1 *eq*) was dissolved in ethyl acetate (4 mL), and the solution was added with a solution of hydrogen chloride in ethyl acetate (4 M, 3 mL). The reaction mixture was stirred at 15 °C for 16 hours. The reaction mixture was concentrated under vacuum to give compound **10-4.** LCMS *m*/*z*: 428.0[M+1]⁺.

### Step 5: synthesis of compound 10

Compound **10-4** (85 mg, 199 µmol, 1 *eq*) was dissolved in *N,N-*dimethylformamide (6 mL), and the solution was added with 2-(7-azobenzotriazol)-*N,N,N',N'*-tetramethyluronium hexafluorophosphate (91 mg, 239 µmol, *1.2 eq*) and *N,N*-diisopropylethylamine (90 mg, 696 µmol, 3.5 *eq*) sequentially. The reaction mixture was stirred at 15 °C for 16 hours. The reaction mixture was concentrated under vacuum to give a crude product, which was purified by silica gel column chromatography (dichloromethane:methanol = 100:1 to 10:1) to give compound **10.** LCMS *m*/*z*: 409.9[M+1]⁺.

### Step 6: synthesis of compounds WX010A and WX010B

Compound 10 (50 mg, 0.12 mmol) was resolved by SFC (column: DAICEL CHIRALCEL OJ-H (250mm×30mm, 5µm), mobile phase: A (CO₂) and B (ethanol, with 0.1% ammonium hydroxide solution); gradient: B% = 30%-30%, 20 min) to give **WX010A** and **WX010B.**

**WX010A:** ¹H NMR (400MHz, DMSO-*d*₆) δ: 8.78 (d, *J*=7.8 Hz, 1H), 8.61 (s, 1H), 8.03 (s, 1H), 7.90 - 7.87 (m, 1H), 7.57 (s, 1H), 6.95 - 6.88 (m, 1H), 6.85 - 6.80 (m, 1H), 5.60 - 5.55 (m, 1H), 4.28 - 4.20 (m, 2H), 4.05 - 3.92 (m, 3H), 3.89 - 3.81 (m, 2H), 2.80 - 2.72 (m, 1H), 1.89 - 1.79 (m, 1H), 1.19 - 1.10 (m, 1H), 0.81 - 0.71 (m, 1H), 0.66 - 0.56 (m, 1H). LCMS *m*/*z:* 409.9[M+1]⁺. SFC (column: Chiralcel OJ-3, 3 µm, 0.46 cm id × 10cm L; mobile phase: A (CO₂) and B (EtOH, with 0.05% isopropylamine); gradient: B% = 5-40%, 4 min; gradient: B% = 40%, 2.5 min; gradient: B% = 5%, 1.5 min; flow rate: 2.8 mL/min; wavelength: 220 nm; pressure: 100 bar), retention time = 3.158 min, chiral isomer excess 99.82%.

**WX010B:** ¹H NMR (400MHz, DMSO-*d*₆) δ: 8.78 (d, *J*=8.0 Hz, 1H), 8.61 (s, 1H), 8.03 (s, 1H), 7.89 (d, *J*=3.0 Hz, 1H), 7.58 (dd, *J*=2.8, 9.4 Hz, 1H), 6.89 (br d, *J*=4.4 Hz, 1H), 6.82 (d, *J*=8.0 Hz, 1H), 5.59 - 5.54 (m, 1H), 4.30 - 4.19 (m, 2H), 4.10 - 3.91 (m, 3H), 3.88 - 3.79 (m, 2H), 2.76 (dd, *J*=3.0, 13.2 Hz, 1H), 1.91 - 1.77 (m, 1H), 1.19 - 1.10 (m, 1H), 0.81 - 0.70 (m, 1H), 0.67 - 0.56 (m, 1H). LCMS *m*/*z:* 409.9[M+1]⁺. SFC (column: Chiralcel OJ-3, 3 µm, 0.46 cm id × 10 cm L; mobile phase: A (CO₂) and B (EtOH, with 0.05% isopropylamine); gradient: B% = 5-40%, 4 min; gradient: B% =40%, 2.5 min; gradient: B% = 5%, 1.5 min; flow rate: 2.8 mL/min; wavelength: 220 nm; pressure: 100 bar), retention time = 3.724 min, chiral isomer excess 99.10%.

### Example 11: Single Crystal X-Ray Diffraction Analysis of Compound WX001B

The culture procedure of single crystal: a sample WX001B (about 5 mg) was placed in a 2 mL brown sample vial, followed by the addition of 200 µL of ethyl acetate. After sufficient dissolving, the sample vial was left to stand at room temperature.

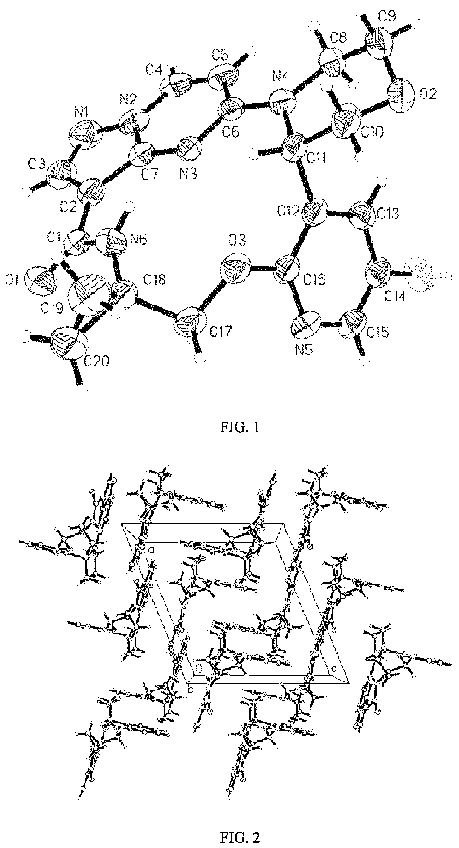

After eight days, under an ethyl acetate condition, colorless long columnar crystals were generated. The crystals were collected, and the diffraction intensity data were collected using a Bruker D8 venture diffractometer in the CuKα radiation, by ϕ/w scanning. After the data collection, the crystal structure was analyzed using the direct method (Shelxs97). The absolute configuration of compound WX001B can be determined from its single crystal data. The ellipsoid plot of the three-dimensional structure of compound WX001B is shown in FIG. 1; the crystal packing diagram of compound WX001B along the b-axis is shown in FIG. 2.

**Table 1. Crystal data and structure refinement of compound WX001B**

| | | |
|---|---|---|
| **Identification code** | WX001B | |
| **Empirical formula** | C₂₀H₁₉FN₆O₃ | |
| **Formula weight** | 410.41 | |
| **Temperature** | 296(2) K | |
| **Wavelength** | 1.54178 A | |
| **Crystal system, space group** | Monoclinic, C2 | |
| **Unit cell dimensions** | a = 12.757(5) A | alpha = 90 deg |
| | b = 16.289(6) A | beta = 112.330(16) deg. |
| | c = 11.971(7) A | gamma = 90 deg |
| **Volume** | 2301.0(19) A^3 | |
| **Z, Calculated density** | 4, 1.185 Mg/m^3 | |
| **Absorption coefficient** | 0.740 mm^-1 | |
| **F(000)** | 856 | |
| **Crystal size** | 0.24 × 0.10 × 0.08 mm | |
| **Theta range for data collection** | 3.992 to 68.739 deg | |
| **Limiting indices** | -15<=h<=15, -19<=k<=19, -14<=1<=13 | |
| **Reflections collected / unique** | 14159 / 4157 [R(int) = 0.0815] | |
| **Completeness to theta = 67.679** | 99.9 % | |
| **Absorption correction** | None | |
| **Refinement method** | Full-matrix least-squares on F^2 | |
| **Data / restraints / parameters** | 4157 / 1 / 272 | |
| **Goodness-of-fit on F^2** | 1.117 | |
| **Final R indices [I>2sigma(I)]** | R1 = 0.0623, wR2 = 0.1815 | |
| **R indices (all data)** | R1 = 0.0869, wR2 = 0.2016 | |
| **Absolute structure parameter** | -0.02(12) | |
| **Extinction coefficient** | 0.0013(4) | |
| **Largest diff. peak and hole** | 0.267 and -0.226 e.A^-3 | |

**Table 2. Atomic coordinates (x 10⁴) and equivalent isotropic displacement parameters (Å² × 10³) of WX001B crystal**

| | **x** | **y** | **z** | **U(eq)** |
|---|---|---|---|---|
| F(1) | 3825(4) | 3389(3) | 3235(4) | 110(1) |
| O(1) | 6651(4) | 6701(3) | 9172(4) | 107(2) |
| O(2) | 1510(4) | 2932(3) | 5823(4) | 93(1) |
| O(3) | 3292(3) | 5638(2) | 6281(3) | 74(1) |
| N(1) | 7794(4) | 4332(4) | 10581(5) | 98(2) |
| N(2) | 6795(3) | 3982(3) | 9862(4) | 73(1) |
| N(3) | 4953(3) | 4334(2) | 8460(3) | 56(1) |
| N(4) | 3645(4) | 3334(3) | 7554(4) | 67(1) |
| N(5) | 3611(4) | 5320(3) | 4553(4) | 74(1) |
| N(6) | 4932(4) | 6113(3) | 8444(4) | 78(1) |
| C(1) | 6060(5) | 6111(3) | 9052(4) | 70(1) |
| C(2) | 6508(4) | 5307(4) | 9547(5) | 71(1) |
| C(3) | 7584(5) | 5109(5) | 10373(5) | 94(2) |
| C(4) | 6554(6) | 3159(4) | 9688(6) | 77(2) |
| C(5) | 5541(5) | 2924(3) | 8947(5) | 74(1) |
| C(6) | 4704(5) | 3537(3) | 8312(4) | 60(1) |
| C(7) | 5994(4) | 4537(3) | 9212(4) | 59(1) |
| C(8) | 3309(7) | 2478(4) | 7281(6) | 88(2) |
| C(9) | 2040(7) | 2374(5) | 6757(7) | 105(3) |
| C(10) | 1678(5) | 3732(5) | 6327(5) | 82(2) |
| C(11) | 2926(4) | 3984(3) | 6803(4) | 61(1) |
| C(12) | 3262(4) | 4237(3) | 5747(4) | 61(1) |
| C(13) | 3395(5) | 3664(4) | 4955(5) | 72(1) |
| C(14) | 3653(5) | 3949(4) | 3999(5) | 76(1) |
| C(15) | 3748(5) | 4743(4) | 3810(5) | 79(2) |
| C(16) | 3404(4) | 5062(3) | 5509(4) | 63(1) |
| C(17) | 3854(7) | 6413(4) | 6334(5) | 86(2) |
| C(18) | 4244(6) | 6705(3) | 7617(5) | 78(2) |
| C(19) | 3507(10) | 7230(5) | 7992(9) | 121(3) |
| C(20) | 4452(9) | 7593(4) | 7820(8) | 110(2) |
| H(6A) | 4577 | 5698 | 8573 | 94 |
| H(3B) | 8121 | 5509 | 10753 | 113 |
| H(4A) | 7102 | 2772 | 10091 | 92 |
| H(5B) | 5365 | 2368 | 8831 | 89 |
| H(8A) | 3625 | 2156 | 8016 | 106 |
| H(8B) | 3619 | 2269 | 6712 | 106 |
| H(9A) | 1857 | 1819 | 6450 | 127 |
| H(9B) | 1750 | 2451 | 7390 | 127 |
| H(10A) | 1409 | 3749 | 6983 | 99 |
| H(10B) | 1236 | 4121 | 5717 | 99 |
| H(11A) | 3011 | 4467 | 7317 | 73 |
| H(13A) | 3314 | 3105 | 5063 | 86 |
| H(15A) | 3912 | 4907 | 3150 | 95 |
| H(17A) | 3336 | 6807 | 5795 | 103 |
| H(17B) | 4496 | 6345 | 6096 | 103 |
| H(19A) | 2757 | 7350 | 7399 | 145 |
| H(19B) | 3547 | 7173 | 8814 | 145 |
| H(20A) | 5090 | 7764 | 8533 | 133 |
| H(20B) | 4299 | 7940 | 7118 | 133 |

**Table 3. Bond lengths (Å) and bond angles [deg] of compound WX001B**

| | | | |
|---|---|---|---|
| F(1)-C(14) | 1.368(6) | C(2)-C(3)-H(3B) | 122.0 |
| O(1)-C(1) | 1.196(7) | C(5)-C(4)-N(2) | 119.5(5) |
| O(2)-C(9) | 1.400(10) | C(5)-C(4)-H(4A) | 120.2 |
| O(2)-C(10) | 1.417(8) | N(2)-C(4)-H(4A) | 120.2 |
| O(3)-C(16) | 1.363(6) | C(4)-C(5)-C(6) | 119.4(5) |
| O(3)-C(17) | 1.440(8) | C(4)-C(5)-H(5B) | 120.3 |
| N(1)-C(3) | 1.297(10) | C(6)-C(5)-H(5B) | 120.3 |
| N(1)-N(2) | 1.362(7) | N(3)-C(6)-N(4) | 117.1(4) |
| N(2)-C(7) | 1.365(6) | N(3)-C(6)-C(5) | 120.7(5) |
| N(2)-C(4) | 1.373(8) | N(4)-C(6)-C(5) | 122.2(5) |
| N(3)-C(6) | 1.331(6) | N(3)-C(7)-N(2) | 124.0(5) |
| N(3)-C(7) | 1.332(6) | N(3)-C(7)-C(2) | 130.8(5) |
| N(4)-C(6) | 1.352(7) | N(2)-C(7)-C(2) | 105.2(4) |
| N(4)-C(8) | 1.460(7) | N(4)-C(8)-C(9) | 112.2(6) |
| N(4)-C(11) | 1.463(7) | N(4)-C(8)-H(8A) | 109.2 |
| N(5)-C(16) | 1.336(6) | C(9)-C(8)-H(8A) | 109.2 |
| N(5)-C(15) | 1.350(8) | N(4)-C(8)-H(8B) | 109.2 |
| N(6)-C(1) | 1.343(7) | C(9)-C(8)-H(8B) | 109.2 |
| N(6)-C(18) | 1.421(7) | H(8A)-C(8)-H(8B) | 107.9 |
| N(6)-H(6A) | 0.8600 | O(2)-C(9)-C(8) | 112.1(5) |
| C(1)-C(2) | 1.460(8) | O(2)-C(9)-H(9A) | 109.2 |
| C(2)-C(3) | 1.391(8) | C(8)-C(9)-H(9A) | 109.2 |
| C(2)-C(7) | 1.402(8) | O(2)-C(9)-H(9B) | 109.2 |
| C(3)-H(3B) | 0.9300 | C(8)-C(9)-H(9B) | 109.2 |
| C(4)-C(5) | 1.316(9) | H(9A)-C(9)-H(9B) | 107.9 |
| C(4)-H(4A) | 0.9300 | O(2)-C(10)-C(11) | 111.9(5) |
| C(5)-C(6) | 1.449(7) | O(2)-C(10)-H(10A) | 109.2 |
| C(5)-H(5B) | 0.9300 | C(11)-C(10)-H(10A) | 109.2 |
| C(8)-C(9) | 1.506(11) | O(2)-C(10)-H(10B) | 109.2 |
| C(8)-H(8A) | 0.9700 | C(11)-C(10)-H(10B) | 109.2 |
| C(8)-H(8B) | 0.9700 | H(10A)-C(10)-H(10B) | 107.9 |
| C(9)-H(9A) | 0.9700 | N(4)-C(11)-C(10) | 110.8(5) |
| C(9)-H(9B) | 0.9700 | N(4)-C(11)-C(12) | 113.2(4) |
| C(10)-C(11) | 1.529(7) | C(10)-C(11)-C(12) | 110.0(4) |
| C(10)-H(10A) | 0.9700 | N(4)-C(11)-H(11A) | 107.5 |
| C(10)-H(10B) | 0.9700 | C(10)-C(11)-H(11A) | 107.5 |
| C(11)-C(12) | 1.538(7) | C(12)-C(11)-H(11A) | 107.5 |
| C(11)-H(11A) | 0.9800 | C(13)-C(12)-C(16) | 116.7(4) |
| C(12)-C(13) | 1.386(7) | C(13)-C(12)-C(11) | 121.7(5) |
| C(12)-C(16) | 1.399(7) | C(16)-C(12)-C(11) | 121.6(4) |
| C(13)-C(14) | 1.386(7) | C(14)-C(13)-C(12) | 117.9(5) |
| C(13)-H(13A) | 0.9300 | C(14)-C(13)-H(13A) | 121.1 |
| C(14)-C(15) | 1.327(9) | C(12)-C(13)-H(13A) | 121.1 |
| C(15)-H(15A) | 0.9300 | C(15)-C(14)-F(1) | 119.2(5) |
| C(17)-C(18) | 1.501(8) | C(15)-C(14)-C(13) | 122.2(5) |
| C(17)-H(17A) | 0.9700 | F(1)-C(14)-C(13) | 118.5(5) |
| C(17)-H(17B) | 0.9700 | C(14)-C(15)-N(5) | 121.6(5) |
| C(18)-C(19) | 1.462(10) | C(14)-C(15)-H(15A) | 119.2 |
| C(18)-C(20) | 1.473(9) | N(5)-C(15)-H(15A) | 119.2 |
| C(19)-C(20) | 1.426(14) | N(5)-C(16)-O(3) | 118.0(5) |
| C(19)-H(19A) | 0.9700 | N(5)-C(16)-C(12) | 124.0(5) |
| C(19)-H(19B) | 0.9700 | O(3)-C(16)-C(12) | 117.9(4) |
| C(20)-H(20A) | 0.9700 | O(3)-C(17)-C(18) | 106.8(5) |
| C(20)-H(20B) | 0.9700 | O(3)-C(17)-H(17A) | 110.4 |
| C(9)-O(2)-C(10) | 107.9(5) | C(18)-C(17)-H(17A) | 110.4 |
| C(16)-O(3)-C(17) | 117.1(4) | O(3)-C(17)-H(17B) | 110.4 |
| C(3)-N(1)-N(2) | 102.2(5) | C(18)-C(17)-H(17B) | 110.4 |
| N(1)-N(2)-C(7) | 113.7(5) | H(17A)-C(17)-H(17B) | 108.6 |
| N(1)-N(2)-C(4) | 127.3(5) | N(6)-C(18)-C(19) | 118.8(6) |
| C(7)-N(2)-C(4) | 118.9(5) | N(6)-C(18)-C(20) | 121.8(6) |
| C(6)-N(3)-C(7) | 117.3(4) | C(19)-C(18)-C(20) | 58.1(6) |
| C(6)-N(4)-C(8) | 121.2(5) | N(6)-C(18)-C(17) | 112.0(5) |
| C(6)-N(4)-C(11) | 118.0(4) | C(19)-C(18)-C(17) | 119.8(6) |
| C(8)-N(4)-C(11) | 119.5(4) | C(20)-C(18)-C(17) | 116.7(5) |
| C(16)-N(5)-C(15) | 117.5(5) | C(20)-C(19)-C(18) | 61.3(6) |
| C(1)-N(6)-C(18) | 129.1(5) | C(20)-C(19)-H(19A) | 117.6 |
| C(1)-N(6)-H(6A) | 115.5 | C(18)-C(19)-H(19A) | 117.6 |
| C(18)-N(6)-H(6A) | 115.5 | C(20)-C(19)-H(19B) | 117.6 |
| O(1)-C(1)-N(6) | 124.0(6) | C(18)-C(19)-H(19B) | 117.6 |
| O(1)-C(1)-C(2) | 122.6(5) | H(19A)-C(19)-H(19B) | 114.7 |
| N(6)-C(1)-C(2) | 113.4(4) | C(19)-C(20)-C(18) | 60.5(5) |
| C(3)-C(2)-C(7) | 102.9(5) | C(19)-C(20)-H(20A) | 117.7 |
| C(3)-C(2)-C(1) | 128.7(5) | C(18)-C(20)-H(20A) | 117.7 |
| C(7)-C(2)-C(1) | 128.1(4) | C(19)-C(20)-H(20B) | 117.7 |
| N(1)-C(3)-C(2) | 116.0(6) | C(18)-C(20)-H(20B) | 117.7 |
| N(1)-C(3)-H(3B) | 122.0 | H(20A)-C(20)-H(20B) | 114.8 |

**Table 4. Twist angles [deg] of compound WX001B**

| | | | |
|---|---|---|---|
| C(3)-N(1)-N(2)-C(7) | 0.9(7) | C(6)-N(4)-C(11)-C(10) | -161.4(4) |
| C(3)-N(1)-N(2)-C(4) | 177.0(6) | C(8)-N(4)-C(11)-C(10) | 32.0(6) |
| C(18)-N(6)-C(1)-O(1) | 15.6(10) | C(6)-N(4)-C(11)-C(12) | 74.4(5) |
| C(18)-N(6)-C(1)-C(2) | -163.6(5) | C(8)-N(4)-C(11)-C(12) | -92.1(6) |
| O(1)-C(1)-C(2)-C(3) | 12.1(10) | O(2)-C(10)-C(11)-N(4) | -48.3(6) |
| N(6)-C(1)-C(2)-C(3) | -168.7(6) | O(2)-C(10)-C(11)-C(12) | 77.7(6) |
| O(1)-C(1)-C(2)-C(7) | -160.9(6) | N(4)-C(11)-C(12)-C(13) | 51.6(6) |
| N(6)-C(1)-C(2)-C(7) | 18.3(8) | C(10)-C(11)-C(12)-C(13) | -72.9(6) |
| N(2)-N(1)-C(3)-C(2) | -0.7(8) | N(4)-C(11)-C(12)-C(16) | -131.0(5) |
| C(7)-C(2)-C(3)-N(1) | 0.3(8) | C(10)-C(11)-C(12)-C(16) | 104.5(5) |
| C(1)-C(2)-C(3)-N(1) | -174.0(6) | C(16)-C(12)-C(13)-C(14) | -0.1(7) |
| N(1)-N(2)-C(4)-C(5) | -179.3(5) | C(11)-C(12)-C(13)-C(14) | 177.4(5) |
| C(7)-N(2)-C(4)-C(5) | -3.3(8) | C(12)-C(13)-C(14)-C(15) | -1.6(8) |
| N(2)-C(4)-C(5)-C(6) | 1.0(8) | C(12)-C(13)-C(14)-F(1) | 177.8(5) |
| C(7)-N(3)-C(6)-N(4) | 179.6(4) | F(1)-C(14)-C(15)-N(5) | -178.5(5) |
| C(7)-N(3)-C(6)-C(5) | -0.2(6) | C(13)-C(14)-C(15)-N(5) | 0.9(9) |
| C(8)-N(4)-C(6)-N(3) | 176.7(5) | C(16)-N(5)-C(15)-C(14) | 1.7(8) |
| C(11)-N(4)-C(6)-N(3) | 10.4(6) | C(15)-N(5)-C(16)-O(3) | 178.4(5) |
| C(8)-N(4)-C(6)-C(5) | -3.5(7) | C(15)-N(5)-C(16)-C(12) | -3.6(7) |
| C(11)-N(4)-C(6)-C(5) | -169.8(4) | C(17)-O(3)-C(16)-N(5) | -25.7(7) |
| C(4)-C(5)-C(6)-N(3) | 0.8(7) | C(17)-O(3)-C(16)-C(12) | 156.2(5) |
| C(4)-C(5)-C(6)-N(4) | -179.0(5) | C(13)-C(12)-C(16)-N(5) | 2.8(7) |
| C(6)-N(3)-C(7)-N(2) | -2.3(7) | C(11)-C(12)-C(16)-N(5) | -174.7(4) |
| C(6)-N(3)-C(7)-C(2) | 179.4(5) | C(13)-C(12)-C(16)-O(3) | -179.2(4) |
| N(1)-N(2)-C(7)-N(3) | -179.4(5) | C(11)-C(12)-C(16)-O(3) | 3.3(6) |
| C(4)-N(2)-C(7)-N(3) | 4.1(7) | C(16)-O(3)-C(17)-C(18) | -145.4(5) |
| N(1)-N(2)-C(7)-C(2) | -0.8(6) | C(1)-N(6)-C(18)-C(19) | -108.3(9) |
| C(4)-N(2)-C(7)-C(2) | -177.3(5) | C(1)-N(6)-C(18)-C(20) | -39.9(10) |
| C(3)-C(2)-C(7)-N(3) | 178.8(5) | C(1)-N(6)-C(18)-C(17) | 105.2(7) |
| C(1)-C(2)-C(7)-N(3) | -6.9(9) | O(3)-C(17)-C(18)-N(6) | 55.2(7) |
| C(3)-C(2)-C(7)-N(2) | 0.3(6) | O(3)-C(17)-C(18)-C(19) | -91.0(7) |
| C(1)-C(2)-C(7)-N(2) | 174.6(5) | O(3)-C(17)-C(18)-C(20) | -157.8(7) |
| C(6)-N(4)-C(8)-C(9) | 161.9(5) | N(6)-C(18)-C(19)-C(20) | 111.4(7) |
| C(11)-N(4)-C(8)-C(9) | -32.0(7) | C(17)-C(18)-C(19)-C(20) | -104.6(8) |
| C(10)-O(2)-C(9)-C(8) | -65.8(7) | N(6)-C(18)-C(20)-C(19) | -106.4(8) |
| N(4)-C(8)-C(9)-O(2) | 48.1(8) | C(17)-C(18)-C(20)-C(19) | 110.1(8) |
| C(9)-O(2)-C(10)-C(11) | 66.2(7) | | |

**Table 5. Hydrogen bonding of compound WX001B [A and deg]**

| **D-H...A** | **d(D-H)** | **d(H...A)** | **d(D...A)** | **<(DHA)** |
|---|---|---|---|---|
| N(6)-H(6A)...N(3) | 0.86 | 2.29 | 2.899(6) | 128.0 |

### Experimental Example 1: Kinase Inhibitory Activity of Compounds Against TrkA, TrkB, TrkC, ALK, and Ros1

The kinase inhibitory activity of compounds against TrkA, TrkB, TrkC, ALK, and Ros1 was detected by Reaction Biology Corp. A substrate, a coenzyme factor, a kinase, and a test compound (10 doses, 3-fold serial dilutions, 2% DMSO final concentration) at a certain concentration were sequentially added to a reaction buffer (20 mM Hepes (pH 7.5), 10 mM MgCl₂, 1 mM EGTA, 0.02% Brij35, 0.02 mg/mL BSA, 0.1 mM Na₃ VO₄, 2 mM DTT, 1% DMSO) and then mixed well. The mixture was incubated at room temperature for 20 minutes, added with ³³P-ATP at a certain concentration to start the reaction, and then incubated at room temperature for 120 minutes. Finally, the radioactivity of the reactant was detected by the filtration-binding method. The final kinase activity was expressed as the ratio of the remaining kinase activity in the test sample to the kinase activity of the DMSO control. The dose-response curves were fitted and IC₅₀ values were calculated by GraphPad software. The results are shown in Table 6.

**Table 6. Half maximal inhibitory concentration IC₅₀ for kinases (nM)**

| **Compound** | **TrkA** | **TrkA-G595R** | **TrkA-G667C** | **TrkC** | **ALK** | **Ros1** |
|---|---|---|---|---|---|---|
| **WX001A** | >1000 | / | / | / | / | / |
| **WX001B** | 2.4 | 3.5 | 2.3 | 0.2 | 182.0 | 1.0 |
| **WX002A** | 3.2 | 2.1 | 0.7 | 0.2 | 18.5 | 0.2 |
| **WX002B** | 526 | / | / | / | / | / |
| **WX003A** | 656 | / | / | / | / | / |
| **WX003B** | 4.9 | 9.4 | 1.7 | 0.2 | 88.9 | 1.2 |
| **WX004A** | 441 | / | / | / | / | / |
| **WX004B** | 2.7 | 2.7 | 18.2 | 0.3 | 133.5 | 8.1 |
| **WX005A** | 4.7 | 0.3 | 0.5 | 0.1 | 103.0 | 3.1 |
| **WX005B** | >1000 | / | / | / | / | / |
| **WX006** | 0.7 | 0.3 | 0.1 | 0.1 | >1000 | 4.8 |
| **WX007** | 0.6 | 0.2 | 0.1 | 0.1 | 901.00 | 2.2 |
| **WX008** | 0.7 | 0.2 | 0.1 | 0.1 | >1000 | 12.8 |
| **WX009A** | 1.8 | 0.9 | 2.9 | 0.1 | 51.3 | 0.1 |
| **WX009B** | >1000 | / | / | / | / | / |
| **WX010A** | 4.1 | 8.9 | 189.0 | 0.3 | >1000 | 28.7 |
| **WX010B** | >1000 | / | / | / | / | / |
| **LOXO-101** | 19.70 | >1000 | 512 | 1.12 | >1000 | 95.50 |
| **LOXO-195** | 6.67 | 6.19 | 110.00 | 0.50 | 274.00 | 1.15 |

| | | | | | | |
|---|---|---|---|---|---|---|
| "/": not detected. | | | | | | |

The results show that: the compounds disclosed herein show relatively high kinase inhibition activity against a plurality of kinases and mutants thereof, which is superior to that of LOXO-195 and LOXO-101.

### Experimental Example 2: Inhibitory Activity of Compounds Against Cell Proliferation

Adenosine Triphosphate (ATP) is an energy carrier commonly used in various life activities in nature, and is the minimum unit for energy storage and transfer. The CellTiter-Glo ™ cell viability assay kit uses luciferase as an assay agent, which requires the involvement of ATP in luminescence. The CellTiter-Glo™ reagent is added into cell culture medium to measure the luminescence value. The luminescence signal is directly proportional to the amount of ATP in the system, and the ATP is positively correlated with the number of viable cells. Therefore, the proliferation of cells can be detected by detecting the ATP content using the CellTiter-Glo kit. In this test, the cell lines used are Ba/F3 LMNA-NTRK1-WT, Ba/F3 LMNA-NTRK1-F589L, BaF3 LMNA-NTRK1-G595R, BaF3 LMNA-NTRK1-G667A, BaF3 ETV6-NTRK3-WT, BaF3 ETV6-NTRK3-G623R, and Ba/F3 SLC34A2-ROS1-WT stable cell strains, 5000 cells/well.

### Determination of IC₅₀:

### 1 Cell culture and inoculation

a) Cells in the logarithmic growth phase were collected and counted using a platelet counter. The cell viability was detected by the trypan blue exclusion method, and the cell viability was kept over 90%.
b) The cell concentration was adjusted; 90 µL of the cell suspension was added to a 96-well plate.
c) Cells in the 96-well plate were incubated overnight at 37 °C with 5% CO₂ and 95% humidity.

### 2 Compound dilution and addition

a) A 10-fold compound solution was prepared, with the highest concentration being 10 µM (a total of 9 concentrations, 3-fold dilution). 10 µL of compound solution was added to each well of a 96-well plate inoculated with cells, and three duplicate wells were set for each compound concentration.
b) The cells in the 96-well plate added with compound were incubated for 72 hours at 37 °C with 5% CO₂ and 95% humidity, followed by the CTG (CellTiter-Glo) analysis.

### 3 Plate reading

a) The CTG reagent was thawed and the cell plate was equilibrated to room temperature for 30 minutes.
b) An equal volume of CTG solution was added to each well.
c) The plate was shaken on an orbital shaker for 5 minutes to lyse the cells.
d) The cell plate was placed at room temperature for 20 minutes to stabilize the luminescence signal.
e) The luminescence value was read.

### 4 Data processing

The data were analyzed using GraphPad Prism 5.0 software, fitted with nonlinear S-curve regression to give dose-response curves, and IC₅₀ values were calculated therefrom. The results are shown in Table 7.

**Table 7. Half maximal inhibitory concentration IC₅₀ (nM) of cells**

| **Compound** | **Ba/F3 LMNA-NTRK1-WT** | **Ba/F3 LMNA-NTRK1-F589L** | **BaF3 LMNA-NTRK1-G595R** | **BaF3 LMNA-NTRK1-G667A** | **BaF3 ETV6-NTRK3-WT** | **BaF3 ETV6-NTRK3-G623R** | **Ba/F3 SLC34A2-ROS1-WT** |
|---|---|---|---|---|---|---|---|
| **WX001B** | 0.6 | 7.7 | 2.7 | 0.8 | 2.6 | 2.3 | 64.2 |
| **WX002A** | 0.2 | / | 0.4 | / | / | 5.8 | 12.6 |
| **WX003B** | 0.6 | / | / | 1.2 | / | / | 51.4 |
| **WX004B** | 0.8 | 37.4 | 1.2 | 3.9 | 1.5 | 3.2 | / |
| **WX005A** | 0.7 | 56.6 | 5.2 | 3.8 | 2.9 | 11.0 | 246.0 |
| **WX006** | 1.5 | 189.3 | 8.4 | 2.0 | 3.2 | 14.2 | 900.0 |
| **WX007** | 0.9 | 100.3 | 6.4 | 1.2 | 4.1 | 13.1 | 770.0 |
| **WX008** | 1.6 | 204.4 | 5.0 | 2.0 | 1.8 | 8.6 | >1000.0 |
| **WX009A** | 1.0 | 15.8 | 7.4 | 7.3 | 2.2 | 12.9 | 50.0 |
| **LOXO-101** | 17.5 | 333.0 | >1000 | 98.0 | 11.0 | 394.1 | >1000 |
| **LOXO-195** | 4.9 | 44.0 | 6.8 | 24.4 | 2.6 | 2.7 | 36.7 |

| | | | | | | | |
|---|---|---|---|---|---|---|---|
| "/": not detected. | | | | | | | |

The results show that: the compounds disclosed herein show relatively high cell proliferation inhibition activity against the Ba/F3 LMNA-NTRK1-WT, Ba/F3 LMNA-NTRK1-F589L, BaF3 LMNA-NTRK1-G595R, BaF3 LMNA-NTRK1-G667A, BaF3 ETV6-NTRK3-WT, and BaF3 ETV6-NTRK3-G623R stable cell strains, which is superior to that of LOXO-195 and LOXO-101. Meanwhile, the compounds disclosed herein show relatively weak inhibitory activity against Ba/F3 SLC34A2-ROS1-WT, and show inhibitory selectivity on NTRK fusion cell lines.

### Experimental Example 3: Pharmacokinetic Test of Compounds in Mice

**Experimental purpose:** male CD-1 mice aged 7-9 weeks were selected as test animals, and the drug concentrations of the compounds in plasma at different time points after single intravenous injection (IV) and intragastric administration (PO) of the compounds were measured by an LC/MS/MS method, so that the pharmacokinetic behavior of the compounds disclosed herein in mice was studied, and the pharmacokinetic characteristic of the compounds was evaluated.

**Drug preparation:** the compounds were all prepared into clear solution by taking 5% DMSO + 10% solutol + 85% water as vehicle, and were used for administration in IV and PO groups. The dose of each compound administered was: IV, 3 mg/kg; PO, 10 mg/kg. The pharmacokinetic parameters are shown in Table 8.

**Table 8. Results of pharmacokinetic test in mice**

| | | **LOXO-101** | **LOXO-195** | **WX001B** | **WX004B** | **WX005A** |
|---|---|---|---|---|---|---|
| **IV** | **Half-life T_{1/2} (h)** | 0.39 | 1.19 | 1.58 | 1.05 | 1.02 |
| | **Apparent volume of distribution Vd (L/kg)** | 0.97 | 0.50 | 0.77 | 0.60 | 0.78 |
| | **Apparent clearance Cl (mL/Kg/min)** | 37.4 | 11.8 | 7.33 | 8.14 | 9.78 |
| | **Area under the curve AUC₀₋ₗₐₛₜ (nM.hr)** | 3122 | 11146 | 16591 | 15393 | 11956 |
| **PO** | **Peak concentration Cₘₐₓ (nM)** | 1548 | 12700 | 12150 | 11050 | 7120 |
| | **Time to peak Tₘₐₓ (h)** | 0.38 | 0.38 | 0.75 | 0.50 | 0.38 |
| | **Area under the curve AUC₀₋ₗₐₛₜ (nM.hr)** | 2890 | 27344 | 49073 | 37294 | 21397 |
| | **Bioavailability F%** | 28% | 74% | 89% | 73% | 54% |

**The results show that:** the total systematic exposure, peak concentration, and bioavailability of the compounds disclosed herein orally administrated are obviously higher than those of LOXO-195 and larotrectinib (LOXO-101) at the same dose, demonstrating superior pharmacokinetic properties.

### Experimental Example 4: Pharmacokinetic Test of Compounds in Rats

**Experimental purpose:** male SD rats aged 7-9 weeks were selected as test animals, and the drug concentrations of the compounds in plasma at different time points after single intravenous injection (IV) and intragastric administration (PO) of the compounds were measured by an LC/MS/MS method, so that the pharmacokinetic behavior of the compounds disclosed herein in rats was studied, and the pharmacokinetic characteristic of the compounds was evaluated.

**Drug preparation:** the compounds were all prepared into clear solution by taking 5% DMSO + 10% solutol + 85% water as vehicle, and were used for administration in IV and PO groups. The dose of each compound administered was: IV, 3 mg/kg; PO, 10 mg/kg. The pharmacokinetic parameters are shown in Table 9.

**Table 9. Results of pharmacokinetic test in rats**

| | | **LOXO-101** | **WX001B** | **WX004B** | **WX006** | **WX008** | **WX009A** |
|---|---|---|---|---|---|---|---|
| **IV** | **Half-life T_{1/2} (h)** | 0.6 | 1.2 | 0.6 | 0.6 | 0.5 | 0.9 |
| | **Apparent volume of distribution Vd (L/kg)** | 1.7 | 1.9 | 1.4 | 1.3 | 1.4 | 1.3 |
| | **Apparent clearance Cl (mL/Kg/min)** | 45.6 | 19.7 | 30.9 | 32.9 | 44.0 | 19.0 |
| | **Area under the curve AUC₀₋ₗₐₛₜ (nM.hr)** | 1704 | 6241 | 4076 | 3818 | 2748 | 6190 |
| **PO** | **Peak concentration Cₘₐₓ (nM)** | 944 | 1805 | 1770 | 3750 | 1134 | 2630 |
| | **Time to peak Tₘₐₓ (h)** | 0.8 | 0.4 | 0.8 | 0.5 | 0.5 | 1.3 |
| | **Area under the curve AUC₀₋ₗₐₛₜ (nM.hr)** | 1927 | 8068 | 4715 | 6637 | 2282 | 9696 |
| | **Bioavailability F%** | 23% | 39% | 35% | 53% | 25% | 49% |

**The results show that:** the total systematic exposure, peak concentration, and bioavailability of the compounds disclosed herein orally administration are all obviously higher than those of larotrectinib (LOXO-101) at the same dose, demonstrating superior pharmacokinetic properties.

### Experimental Example 5: Efficacy Test of Compounds in Animals

### 1. Test method

### 1.1 Model preparation:

Cells in the logarithmic growth phase were collected and resuspended in serum-free medium to a cell concentration of 2-4 × 10⁷ cells/mL. An equal volume of matrigel was added to the cell suspension to achieve a final cell concentration of 1-2 × 10⁷ cells/mL. 0.2 mL of tumor cell suspension was subcutaneously inoculated at the scapula of each nude mouse with the inoculation amount of 2-4 × 10⁶ cells/mouse to prepare the animal models.

### 1.2 Experimental grouping:

The maximum and the minimum tumor diameters of the xenografts in nude mice were measured using a vernier caliper, and the tumor volume (TV) was calculated according to the formula: V = 1/2 × a × b², where a and b denote the maximum and minimum diameters of the tumor mass, respectively. 56 nude mice with appropriate tumor volume were selected and randomly divided into 7 groups: solvent control group, four dose groups (2.5 mg/kg, 5 mg/kg, 10 mg/kg, 20 mg/kg, bid) of WX001B, Loxo-195 group (100 mg/kg, bid), and Loxo-101 group (60 mg/kg, qd).

### 1.3 Observation of indexes:

Tumor diameter was measured, tumor volume was calculated, and the anti-tumor effect of WX001B was dynamically observed. The mice in groups were intragastrically administrated on the next day, and the animal body weight and the long and short diameters of the tumor were measured once every other day. The following indexes were observed on the next day after the last administration:
a) 1.3.1 Relative tumor proliferation rate T/C (%): T/C (%) = TRTV/CRTV × 100%. (TRTV: administration group RTV, CRTV: control group RTV), tumor growth inhibition rate (TGI) = 1 - T/C (%).
b) 1.3.2 Tumor inhibition rate: at the end of the test, the animals were executed by removing the cervical vertebrae. The tumor masses were peeled off and weighed, the tumor inhibition was calculated according to the formula: tumor inhibition rate = (mean tumor weight of control group - mean tumor weight of administration group) / mean tumor weight of control group × 100%, and the tumor masses were recorded by photographing.
c) 1.3.3 Animal weights: animals were weighed at each tumor diameter measurement, and a weight loss of greater than 20% was defined as a toxic drug response.

### 2. Test results

### 2.1 Effect of WX001B on tumor volume and relative tumor proliferation rate

### 2.1.1 Ba/F3 ETV6-NTRK3-G623R model

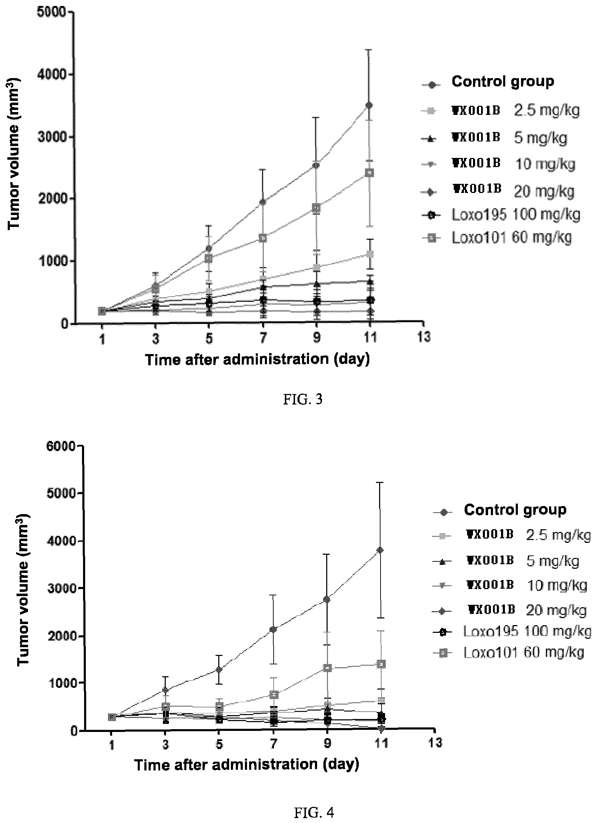

The results and statistical analysis are detailed in Table 10 and FIG. 3.

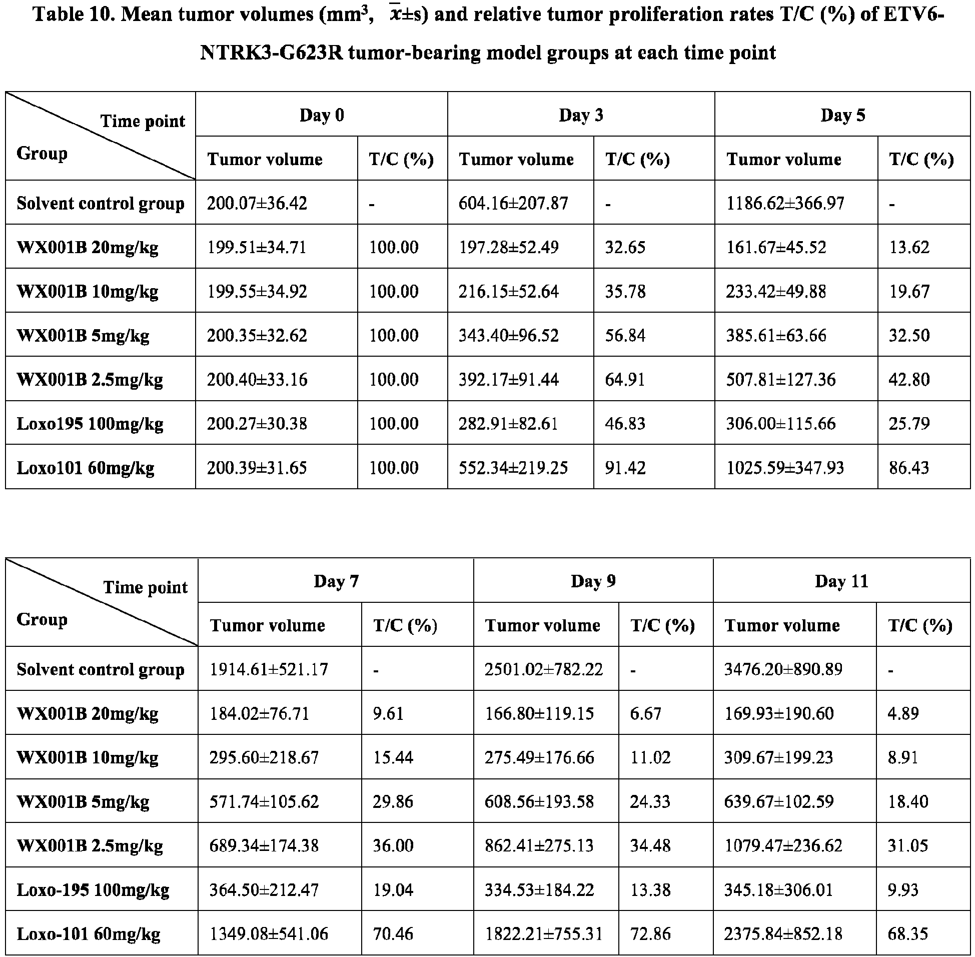

### The data show that:

During the administration, continuous tumor growth was observed in the solvent control group, and the relative tumor proliferation rates of all dose groups of WX001B and the Loxo-195 group continuously decrease.

The 2.5 mg/kg, 5 mg/kg, 10 mg/kg, and 20 mg/kg groups of WX001B have respective TGI of 68.95%, 81.60%, 91.09%, and 95.11% on the day of dissection.

The 100 mg/kg group of Loxo-195 has TGI of 90.07% on the day of dissection.

The 60 mg/kg group of Loxo-101 has TGI of 31.65% on the day of dissection, which is consistent with the first generation NTRK inhibitor being insensitive to drug resistant mutant cells.

### 2.1.2 Ba/F3 ETV6-NTRK3 model

The results and statistical analysis are detailed in Table 11 and FIG. 4.

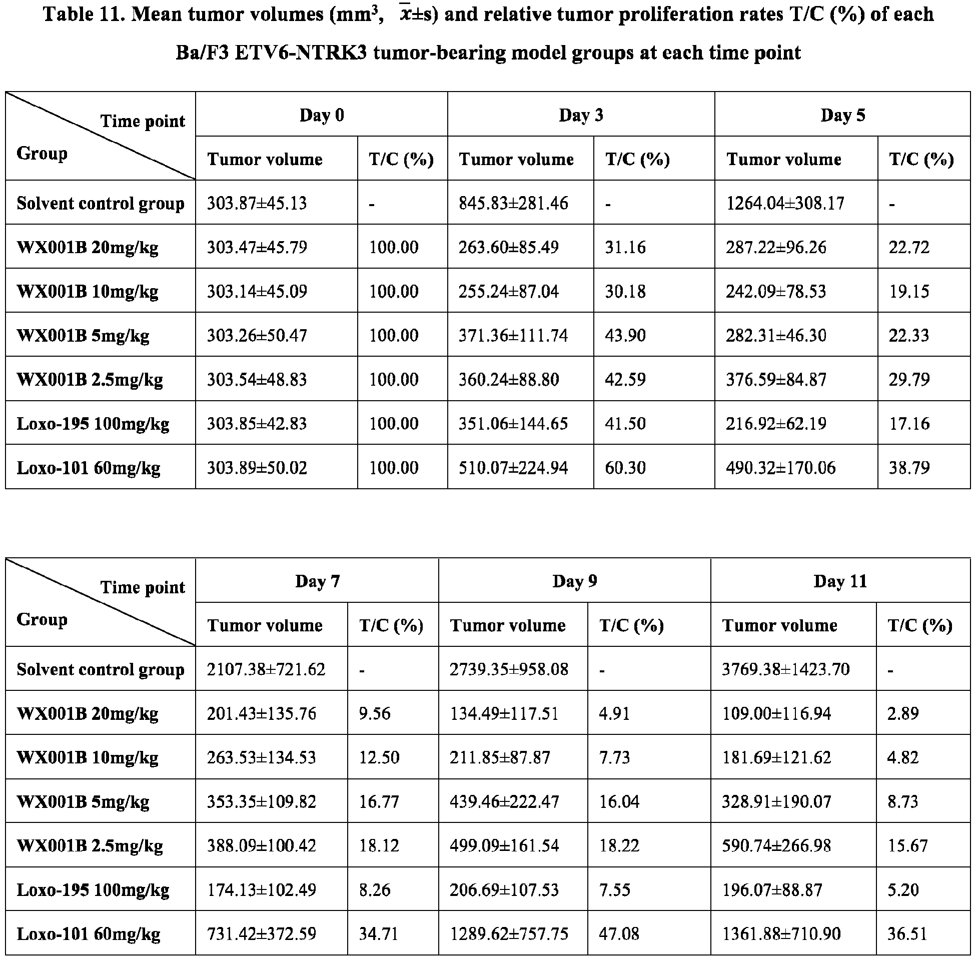

### The data show that:

During the administration, continuous tumor growth was observed in the solvent control group, and the relative tumor proliferation rates of all dose groups of WX001B, the Loxo-195 group, and the Loxo-101 group decrease.

The 2.5 mg/kg, 5 mg/kg, 10 mg/kg, and 20 mg/kg groups of WX001B have respective TGI of 84.33%, 91.27%, 95.18%, and 97.11% on the day of dissection.

The 100 mg/kg group of Loxo-195 has TGI of 94.80% on the day of dissection; the 60 mg/kg group of Loxo-101 has TGI of 63.87% on the day of dissection.

### 2.2 Effect of WX001B on tumor weight

### 2.2.1 Ba/F3 ETV6-NTRK3-G623R model

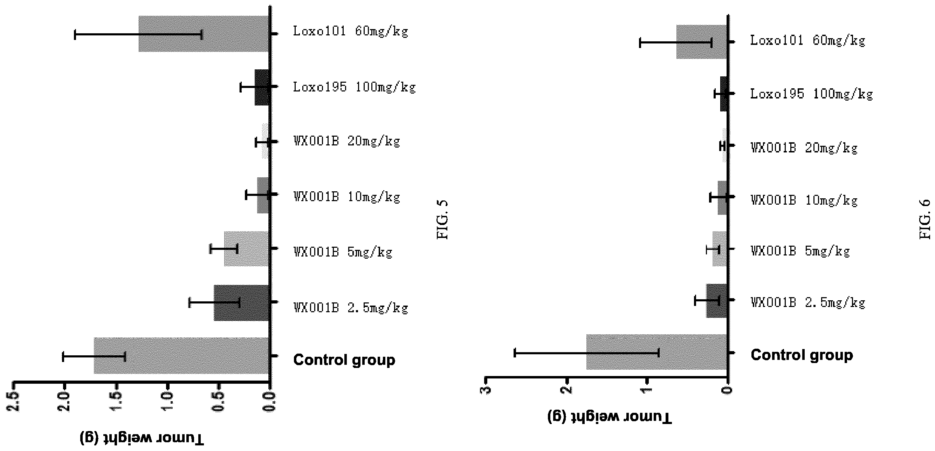

The results and statistical analysis are detailed in Table 12 and FIG. 5.

**Table 12. Effect of WX001B on the weight (x̅±s) of xenograft in Ba/F3 ETV6-NTRK3-G623R tumor-bearing nude mice**

| **Group** | **Tumor weight (g)** | **Tumor inhibition rate (%)** |
|---|---|---|
| **Solvent control** | 1.72±0.30 | - |
| **WX001B 20mg/kg** | 0.07±0.06 | 95.75 |
| **WX001B 10mg/kg** | 0.12±0.11 | 92.90 |
| **WX001B 5mg/kg** | 0.44±0.13 | 74.12 |
| **WX001B 2.5mg/kg** | 0.54±0.24 | 68.72 |
| **Loxo-195 100mg/kg** | 0.15±0.14 | 91.50 |
| **Loxo-101 60mg/kg** | 1.28±0.62 | 25.27 |

### The data show that:

At the end of the test, the tumor inhibition rates of the 20 mg/kg, 10 mg/kg, 5 mg/kg, and 2.5 mg/kg dose groups of WX001B are 95.75%, 92.90%, 74.12%, and 68.72%, respectively. The 100 mg/kg group of Loxo-195 has the tumor inhibition rate of 91.50%, and has the similar efficacy as the 10 mg/kg group of WX001B. The 60 mg/kg group of Loxo-101 has the tumor inhibition rate of 25.27% on the day of dissection, which is consistent with the first generation NTRK inhibitor being insensitive to drug resistant mutant cells.

### 2.2.2 Ba/F3 ETV6-NTRK3 model

The results and statistical analysis are detailed in Table 13 and FIG. 6.

**Table 13. Effect of WX001B on the weight (x̅±s) of xenograft in Ba/F3 ETV6-NTRK3 tumor-bearing nude mice**

| **Group** | **Tumor weight (g)** | **Tumor inhibition rate (%)** |
|---|---|---|
| **Solvent control** | 1.75±0.89 | - |
| **WX001B 20mg/kg** | 0.06±0.03 | 96.47 |
| **WX001B 10mg/kg** | 0.12±0.10 | 93.42 |
| **WX001B 5mg/kg** | 0.18±0.08 | 89.51 |
| **WX001B 2.5mg/kg** | 0.25±0.15 | 85.47 |
| **Loxo-195 100mg/kg** | 0.09±0.07 | 94.86 |
| **Loxo-101 60mg/kg** | 0.64±0.44 | 63.49 |

### The data show that:

At the end of the test, the tumor inhibition rates of the 20 mg/kg, 10 mg/kg, 5 mg/kg, and 2.5 mg/kg dose groups of WX001B are 96.47%, 93.42%, 89.51%, and 85.47%, respectively. The 100 mg/kg group of Loxo-195 has the tumor inhibition rate of 94.86%, and has the similar efficacy as the 10 mg/kg group of WX001B. The 60 mg/kg group of Loxo-101 has the tumor inhibition rate of 63.49%.

### 2.3 Effect of WX001B on animal body weight

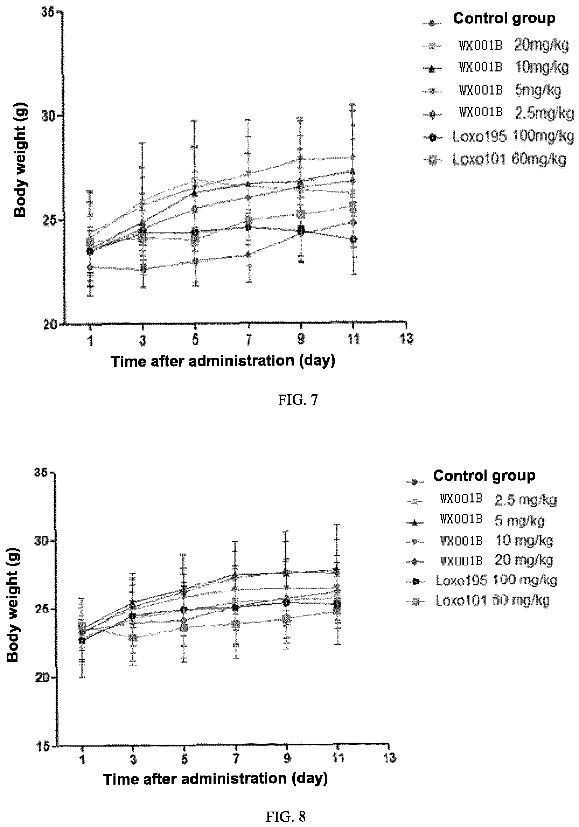

The results and statistical analysis are detailed in Tables 14-15, and FIGs. 7-8.

**Table 14. Effect of WX001B on the body weight (g, x̅±s) of Ba/F3 ETV6-NTRK3-G623R tumor-bearing nude mice**

| **Group** | **Day 0** | **Day 3** | **Day 5** | **Day 7** | **Day 9** | **Day 11** | **Rate of change in body weight (%) of day 11 to day 0** |
|---|---|---|---|---|---|---|---|
| **Solvent control** | 22.8±0.7 | 22.7±0.9 | 23.0±1.2 | 23.3±1.3 | 24.3±1.4 | 24.8±1.2 | 8.8 |
| **WX001B 20mg/kg** | 24.1±2.3 | 25.9±2.8 | 26.9±2.9 | 26.6±3.2 | 26.4±3.5 | 26.3±3.9 | 8.8 |
| **WX001B 10mg/kg** | 23.6±2.2 | 24.9±2.1 | 26.3±2.2 | 26.7±2.2 | 26.8±2.2 | 27.3±2.2 | 15.6 |
| **WX001B 5mg/kg** | 24.4±1.9 | 25.7±1.9 | 26.5±1.9 | 27.1±1.8 | 27.9±1.9 | 27.9±2.6 | 14.3 |
| **WX001B 2.5mg/kg** | 23.6±1.7 | 24.6±1.7 | 25.5±1.8 | 26.1±2.1 | 26.5±1.8 | 26.8±2.0 | 13.8 |
| **Loxo195 100mg/kg** | 23.5±1.2 | 24.4±1.1 | 24.4±0.9 | 24.6±0.9 | 24.4±1.2 | 24.0±1.7 | 2.1 |
| **Loxo101 60mg/kg** | 23.9±1.4 | 24.2±1.8 | 24.1±2.0 | 25.0±2.2 | 25.3±2.2 | 25.6±2.4 | 6.8 |

**Table 15. Effect of WX001B on the body weight (g, x̅±s) of Ba/F3 ETV6-NTRK3 tumor-bearing nude mice**

| **Group** | **Day 0** | **Day 3** | **Day 5** | **Day 7** | **Day 9** | **Day 11** | **Rate of change in body weight (%) of day 11 to day 0** |
|---|---|---|---|---|---|---|---|
| **Solvent control** | 23.4±2.4 | 24.0±2.7 | 24.1±2.9 | 25.1±2.9 | 25.7±1.4 | 26.1±2.6 | 4.8 |
| **WX001B 20mg/kg** | 23.2±1.9 | 25.1±2.5 | 26.2±2.8 | 27.2±2.7 | 27.7±2.9 | 27.6±3.5 | 5.1 |
| **WX001B 10mg/kg** | 23.3±0.9 | 24.9±0.7 | 25.8±0.7 | 26.3±1.1 | 26.4±1.1 | 26.4±1.4 | 4.8 |
| **WX001B 5mg/kg** | 23.5±1.6 | 25.5±1.8 | 26.4±1.6 | 27.5±1.7 | 27.5±2.3 | 27.8±2.2 | 5.0 |
| **WX001B 2.5mg/kg** | 22.9±1.6 | 24.2±2.0 | 24.8±1.8 | 25.4±2.0 | 25.5±1.8 | 25.7±1.9 | 4.9 |
| **Loxo195 100mg/kg** | 22.7±2.7 | 24.4±2.7 | 24.9±2.6 | 25.1±2.8 | 25.4±3.0 | 25.2±3.0 | 4.9 |
| **Loxo101 60mg/kg** | 23.8±1.6 | 22.9±2.0 | 23.6±2.2 | 23.9±2.5 | 24.2±2.2 | 24.7±2.7 | 4.4 |

### The data show that:

Continuous weight increase is observed in animals in all dose groups of WX001B, with no significant difference compared with the solvent control group, and the animals have good tolerance.

### 2.4 General observations

### 2.4.1 Ba/F3 ETV6-NTRK3-G623R model

The 1# animal in the 100 mg/kg dose group of Loxo-195 developed thorax congestion at D8 and died at D10, possibly associated with the administration operation. Other animals were found to be in good condition during the administration. The animals in the high dose groups developed abdominal distension, possibly associated with hyperphagia.

### 2.4.2 Ba/F3 ETV6-NTRK3 model

All animals in all dose groups of WX001B survived until the end of administration, and were in good condition during administration. The animals in the high dose groups developed abdominal distension, possibly associated with hyperphagia.

### 3. Conclusion

Under the conditions of this test, WX001B inhibits the growth of subcutaneous xenografts of Ba/F3 ETV6-NTRK3-G623R cells and Ba/F3 ETV6-NTRK3 nude mice in a dose-dependent manner at 2.5-20 mg/kg, and the animals have good tolerance. In these two models, the tumor inhibition effect of WX001B is better than that of the first generation inhibitor Loxo-101; the tumor inhibition effect of WX001B at 10 mg/kg is similar to that of the Loxo-195 at 100 mg/kg.

## Claims

1. A compound of formula (II), an isomer thereof or a pharmaceutically acceptable salt thereof, 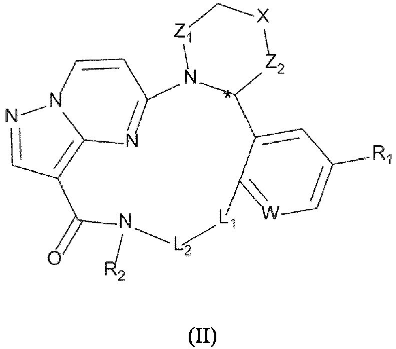 wherein,
W is selected from -C(R₃)- and N;
X is selected from -C(R₄)(R₅)-, -O-, and -N(R₆)-;
Z₁ is selected from -CH(R₇)-;
Z₂ is selected from -CH₂-, -CH₂CH₂-, and -CH₂CH₂CH₂-;
R₁ is selected from H, F, Cl, Br, I, OH, NH₂, CN, and C₁₋₆ alkyl optionally substituted with 1, 2, or 3 Rₐ;
R₂ is selected from H and C₁₋₆ alkyl optionally substituted with 1, 2, or 3 R_{b};
R₃ is selected from H, F, Cl, Br, I, OH, and NH₂;
R₄ and R₅ are each independently selected from H, F, Cl, Br, I, OH, NH₂, and C₁₋₆ alkyl optionally substituted with 1, 2, or 3 R_{c};
R₆ is selected from H and C₁₋₆ alkyl optionally substituted with 1, 2, or 3 R_{d};
R₇ is selected from H, F, Cl, Br, I, OH, NH₂, CN and C₁₋₆ alkyl optionally substituted with 1, 2, or 3 R_{g};
L₁ is selected from -O-, -N(R)-, and -C₁₋₃ alkyl- optionally substituted with 1, 2, or 3 Rₑ;
L₂ is selected from -C₁₋₃ alkyl-, -C₃₋₆ cycloalkyl-, -3-6 membered heterocycloalkyl-, and -C₃₋₆ cycloalkyl-C₁₋₃ alkyl-, the -C₁₋₃ alkyl-, -C₃₋₆ cycloalkyl-, -3-6 membered heterocycloalkyl-, and -C₃₋₆ cycloalkyl-C₁₋₃ alkyl-being optionally substituted with 1, 2, or 3 R_{f};
Rₐ, R_{b}, R_{c}, R_{d}, Rₑ, R_{f}, and R_{g} are each independently selected from H, F, Cl, Br, I, OH, and NH₂; and
R is selected from H and C₁₋₃ alkyl; and
the carbon atom with "*" is a chiral carbon atom present in a form of a single (*R*)- or (*S*)- enantiomer or in a form enriched with one enantiomer;
wherein the 3-6 membered heterocycloalkyl contains 1, 2, 3, or 4 heteroatoms or heteroatom groups independently selected from O, NH, S, and N.

2. The compound, the isomer thereof or the pharmaceutically acceptable salt thereof according to claim 1, wherein R₁ is selected from H, F, Cl, Br, I, OH, NH₂, CN and CH₃; preferably H, F, Cl and Br; and more preferably F.

3. The compound, the isomer thereof or the pharmaceutically acceptable salt thereof according to claim 1 or 2, wherein R₂ is selected from H and CH₃, and preferably H.

4. The compound, the isomer thereof or the pharmaceutically acceptable salt thereof according to any one of claims 1 to 3, wherein R₃ is selected from H, F, Cl, Br and I; preferably H and F; and more preferably H.

5. The compound, the isomer thereof or the pharmaceutically acceptable salt thereof according to any one of claims 1 to 4, wherein R₄ and R₅ are each independently selected from H, F, Cl, Br, I, OH, NH₂ and CH₃; preferably H and F; and more preferably H.

6. The compound, the isomer thereof or the pharmaceutically acceptable salt thereof according to any one of claims 1 to 5, wherein R₆ is selected from H and CH₃, and preferably H.

7. The compound, the isomer thereof or the pharmaceutically acceptable salt thereof according to any one of claims 1 to 6, wherein R₇ is selected from H, F, Cl, Br, I, OH, NH₂, CN, and CH₃, CH₂CH₃, CH₂CH₂CH₃ and CH(CH₃)₂ optionally substituted with 1, 2 or 3 R_{g}; preferably H, CH₃, CH₂CH₃, CH₂CH₂CH₃ and CH(CH₃)₂; more preferably H and CH₃; and most preferably H.

8. The compound, the isomer thereof or the pharmaceutically acceptable salt thereof according to any one of claims 1 to 7, wherein L₁ is selected from -CH₂-, -CH₂CH₂-, -O- and -NH-; preferably -O- and -NH-; and more preferably -O-.

9. The compound, the isomer thereof or the pharmaceutically acceptable salt thereof according to any one of claims 1 to 8, wherein L₂ is selected from -CH₂-, -CH₂CH₂-, -CH₂CH(CH₃)-, 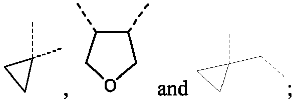 preferably -CH₂CH(CH₃)-, 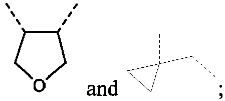 and more preferably -

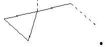

10. The compound, the isomer thereof or the pharmaceutically acceptable salt thereof according to any one of claims 1 to 9, wherein W is selected from -CH- and N; and preferably N.

11. The compound, the isomer thereof or the pharmaceutically acceptable salt thereof according to any one of claims 1 to 10, wherein X is selected from -CH₂-, -O- and -NH-; and preferably -O-.

12. The compound, the isomer thereof or the pharmaceutically acceptable salt thereof according to any one of claims 1 to 11, wherein Z₁ is selected from -CH(CH₃) and -CH₂-, and preferably -CH₂-.

13. The compound, the isomer thereof or the pharmaceutically acceptable salt thereof according to any one of claims 1 to 12, wherein Z₂ is selected from -CH₂-, -CH₂CH₂- and -CH₂CH₂CH₂-; preferably -CH₂- and-CH₂CH₂-; and more preferably -CH₂-.

14. The compound, the isomer thereof or the pharmaceutically acceptable salt thereof according to any one of claims 1 to 13, wherein the structural unit 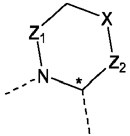 is selected from

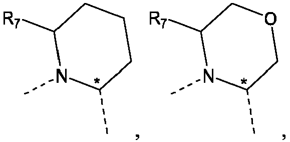

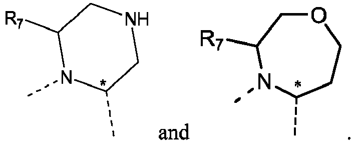

15. The compound, the isomer thereof or the pharmaceutically acceptable salt thereof according to any one of claims 1 to 14, wherein the structural unit 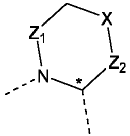 is selected from 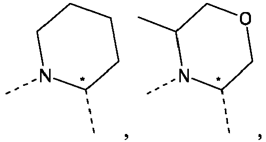 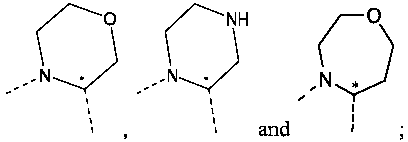 preferably 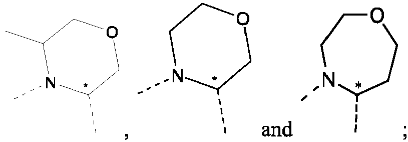 and more preferably

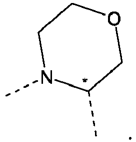

16. The compound, the isomer thereof or the pharmaceutically acceptable salt thereof according to any one of claims 1 to 15, wherein W is selected from -CH- and N; X is selected from -CH₂-, -O- and -NH-; R₁ is F; R₂ is H; Z₁ is selected from -CH₂- and -CH(CH₃)-; Z₂ is selected from -CH₂- and -CH₂CH₂-; L₁ is -O-; and L₂ is selected from -CH₂CH(CH₃)-,

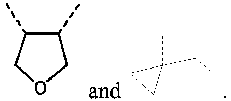

17. The compound, the isomer thereof or the pharmaceutically acceptable salt thereof according to any one of claims 1 to 16, selected from: 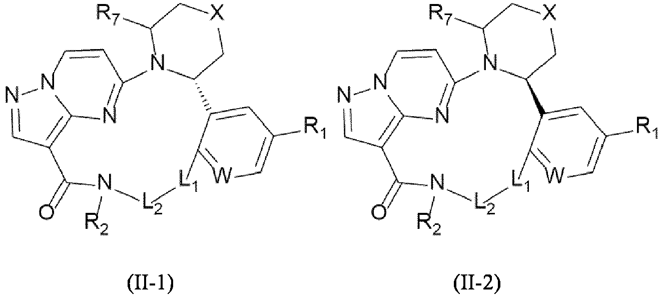 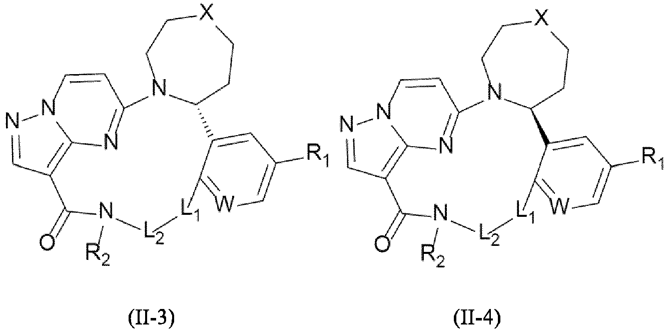 wherein,
W, X, R₁, R₂, R₇, L₁, and L₂ are defined as in any one of claims 1 to 11.

18. The compound, the isomer thereof or the pharmaceutically acceptable salt thereof according to any one of claims 1 to 17, selected from: 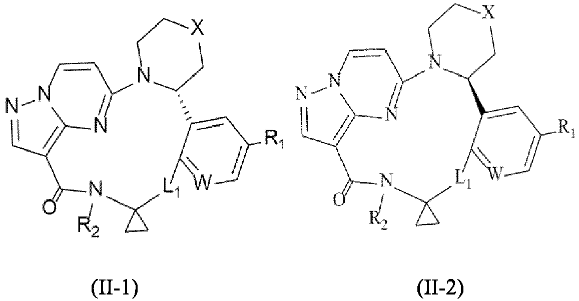 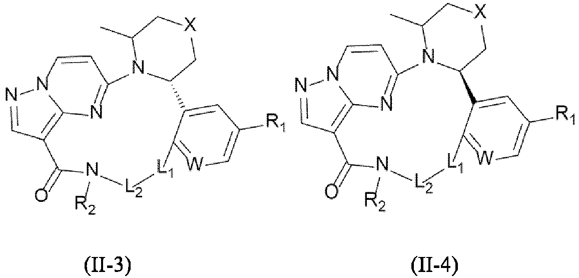 wherein,
W, X, R₁, R₂, L₁, and L₂ are defined as in any one of claims 1 to 11.

19. The compound, the isomer thereof or the pharmaceutically acceptable salt thereof according to any one of claims 1 to 18, selected from:

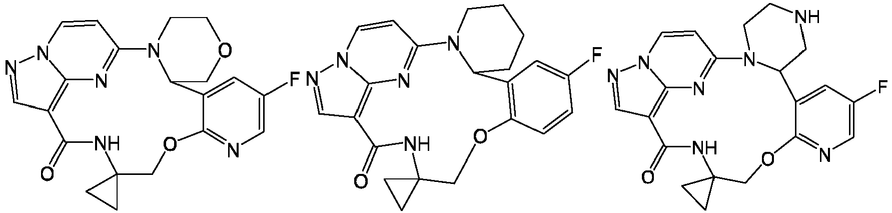

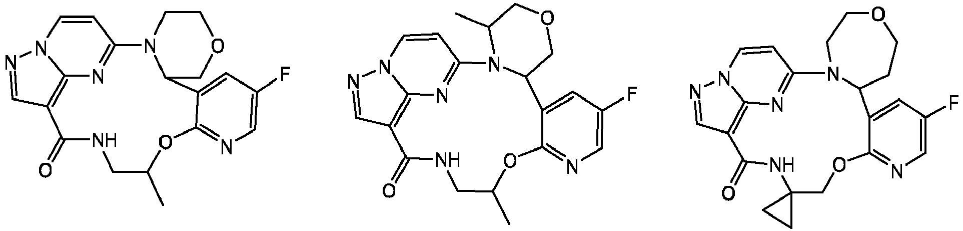

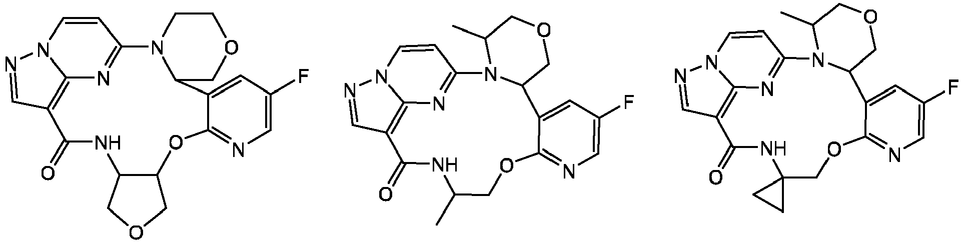

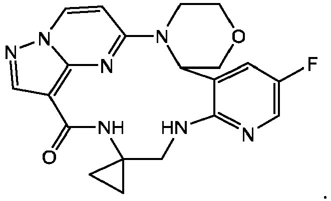

20. The compound, the isomer thereof or the pharmaceutically acceptable salt thereof according to claim 19, selected from:

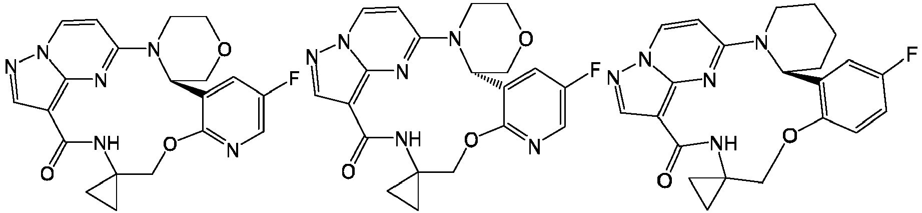

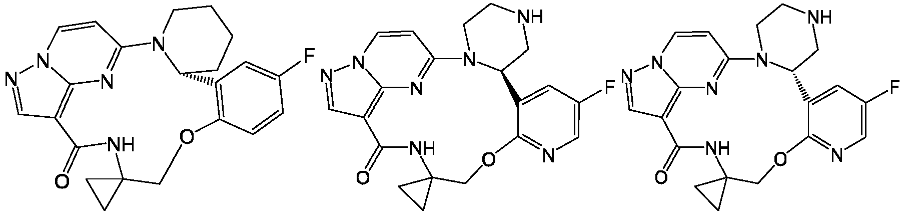

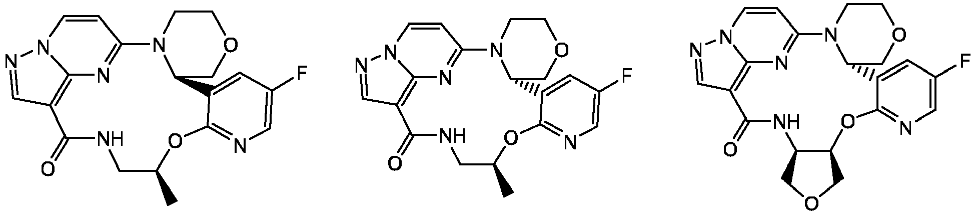

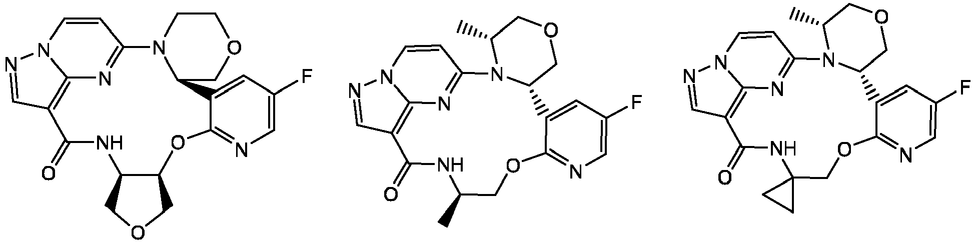

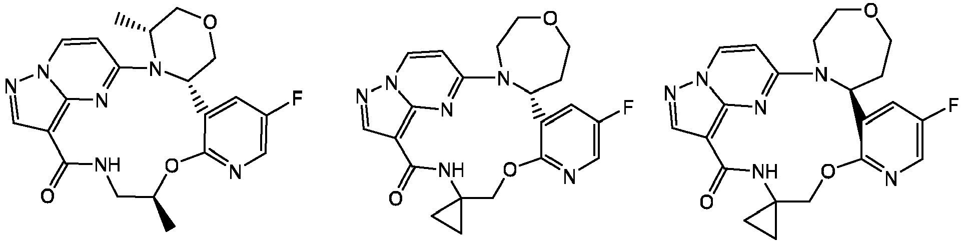

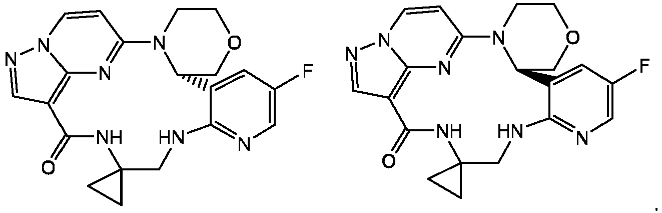

21. A pharmaceutical composition comprising a therapeutically effective amount of the compound, the isomer thereof or the pharmaceutically acceptable salt thereof according to any one of claims 1 to 20 as an active ingredient, and a pharmaceutically acceptable carrier.

22. Use of the compound, the isomer thereof or the pharmaceutically acceptable salt thereof according to any one of claims 1 to 20, or the composition according to claim 21 in preparing a medicament for treating Trk kinase-related diseases.

23. The use according to claim 22, wherein the medicament is used for treating a solid tumor.
